(19) Europäisches Patentamt
European Patent Office
Office européen des brevets

(11) Veröffentlichungsnummer: **0 056 992**
**A1**

(12) # EUROPÄISCHE PATENTANMELDUNG

(21) Anmeldenummer: 82100445.4

(22) Anmeldetag: 22.01.82

(51) Int. Cl.³: C 07 H 13/04
C 07 H 15/04, C 07 H 15/18
A 61 K 31/70
//C07C103/52

(30) Priorität: 23.01.81 CH 439/81

(43) Veröffentlichungstag der Anmeldung: 04.08.82 Patentblatt 82/31

(84) Benannte Vertragsstaaten: AT BE CH DE FR IT LI LU NL SE

(71) Anmelder: CIBA-GEIGY AG
Patentabteilung Postfach
CH-4002 Basel(CH)

(72) Erfinder: Baschang, Gerhard, Dr.
Bückenweg 7
CH-4126 Bettingen(CH)

(72) Erfinder: Hartmann, Albert, Dr.
Steingasse 21A
D-7889 Grenzach(DE)

(72) Erfinder: Wacker, Oskar, Dr.
Löwenbergstrasse 60
CH-4059 Basel(CH)

(72) Erfinder: Tarcsay, Lajos, Dr.
Muttenzerstrasse 25
D-7889 Grenzach-Wyhlen(DE)

(74) Vertreter: Zumstein sen., Fritz, Dr. et al.
Bräuhausstrasse 4
D-8000 München 2(DE)

(54) **Phosphorylverbindungen, pharmazeutische Präparate enthaltend solche Verbindungen, sowie ihre Verwendung.**

(57) Beschrieben sind Hexapyranoseverbindungen der Formel I und ihre Salze mit immunomodulatorischer Wirkung, die z.B. in Form pharmazeutischer Präparate, auch zusammen mit Antibiotika, verwendet werden können, und Verfahren zu ihrer Herstellung.

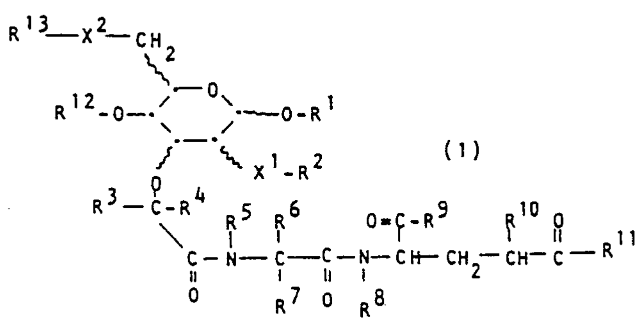

(1)

In Formel I bedeuten $X^1$ und $X^2$ unabhängig voneinander eine Gruppe der Formel -O- oder $-N(R^{14})-$, wobei $R^{14}$ für Wasserstoff oder Niederalkyl steht, $R^1$, $R^2$, $R^{12}$ und $R^{13}$ unabhängig voneinander einen Rest der Formel Ia,

$$-(Z^1-Y^1-X^3)_n-A^1 \qquad (Ia)$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der Formel -O- oder $-N(R^{14})-$, worin $R^{14}$ die oben gegebene Bedeutung hat, und $A^1$ für einen Rest der Formel Ib,

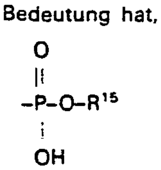

(Ib)

./...

EP 0 056 992 A1

worin $R^{15}$ einen mindestens 7 Kohlenstoffatome aufweisenden aliphatischen oder cycloaliphatischen Rest darstellt, oder für eine Gruppe der Formel Ic,

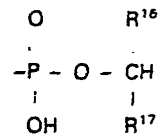

(Ic)

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder einen unter physiologischen Bedingungen abspaltbaren Rest,

$R^3$, $R^4$, $R^5$, $R^7$ und $R^8$ unabhängig voneinander Wasserstoff oder Niederalkyl,

$R^6$ Wasserstoff oder Niederalkyl, das unsubstituiert oder durch eine Gruppe der Formel Id,

$$-E-(Z^2-Y^2-X^4)_m-A^2 \qquad \text{(Id)}$$

worin m für 0 oder 1, E für eine Gruppe der Formel –O–, –S– oder $-N(R^{14})-$, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel –O– oder $-N(R^{14})-$, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder

$R^5$ und $R^6$ zusammen unsubstituiertes oder substituiertes 1,3- oder 1,4-Niederalkylen,

$R^9$ und $R^{11}$ unabhängig voneinander einen Rest der Formel Ie,

$$-X^5-Y^3-X^6-A^3 \qquad \text{(Ie)}$$

worin $X^5$ für eine Gruppe der Formel –O–, –S– oder $-N(R^{14})-$ und $X^6$ für eine Gruppe der Formel –O– oder $-N(R^{14})-$, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und $R^{10}$ Wasserstoff oder freies, verestertes oder amidiertes Carboxy bedeuten, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ von Acyl, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies oder veräthertes Hydroxy oder Mercapto, durch von der Gruppe der Formel Id verschiedenes verestertes Hydroxy oder Me durch freies oder von der Gruppe der Formel Id vers nes substituiertes Amino, oder durch Cycloalkyl, carc sches Aryl oder stickstoffhaltiges Heteroaryl mit 5 Ringgliedern im heterocyclischen Ring substituiert i. $R^7$ von Wasserstoff verschieden ist.

0056992



4-13262/+

# Phosphorylverbindungen, pharmazeutische Präparate enthaltend solche Verbindungen, sowie ihre Verwendung.

Die vorliegende Erfindung betrifft Phosphorylverbindungen, in erster Linie Hexapyranoseverbindungen der Formel I, worin

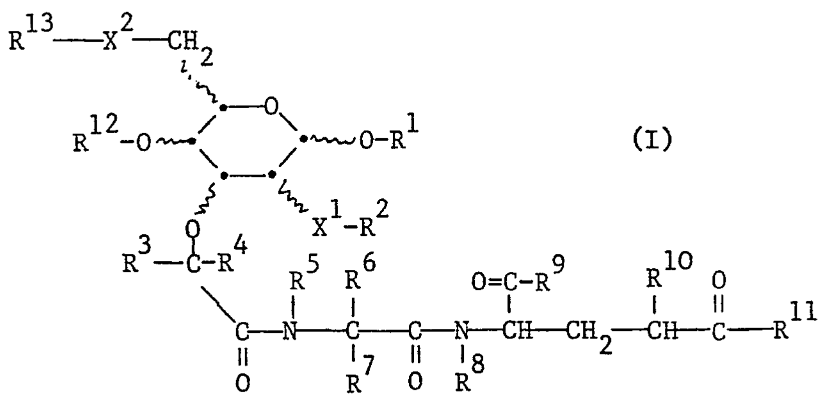

(I)

$X^1$ und $X^2$ unabhängig voneinander eine Gruppe der Formel -O- oder $-N(R^{14})-$, wobei $R^{14}$ für Wasserstoff oder Niederalkyl steht, $R^1$, $R^2$, $R^{12}$ und $R^{13}$ unabhängig voneinander einen Rest der Formel Ia,

$$-(Z^1-Y^1-X^3)_n-A^1 \qquad (Ia)$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der Formel -O- oder $-N(R^{14})-$, worin $R^{14}$ die oben gegebene Bedeutung hat, und $A^1$ für einen Rest der Formel Ib,

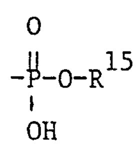

(Ib)

worin $R^{15}$ einen mindestens 7 Kohlenstoffatome aufweisenden aliphatischen oder cycloaliphatischen Rest darstellt, oder für eine Gruppe der Formel Ic,

$$\begin{array}{ccc} O & & R^{16} \\ \| & & | \\ -P & - O - & CH \\ | & & | \\ OH & & R^{17} \end{array} \qquad (Ic)$$

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder einen unter physiologischen Bedingungen abspaltbaren Rest,

$R^3$, $R^4$, $R^5$, $R^7$ und $R^8$ unabhängig voneinander Wasserstoff oder Niederalkyl,

$R^6$ Wasserstoff oder Niederalkyl, das unsubstituiert oder durch eine Gruppe der Formel Id,

$$-E-(Z^2-Y^2-X^4)_m-A^2 \qquad (Id)$$

worin m für 0 oder 1, E für eine Gruppe der Formel -O-, -S- oder $-N(R^{14})-$, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel -O- oder $-N(R^{14})-$, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder

$R^5$ und $R^6$ zusammen unsubstituiertes oder substituiertes 1,3- oder

1,4-Niederalkylen,
$R^9$ und $R^{11}$ unabhängig voneinander einen Rest der Formel Ie,

$$-X^5-Y^3-X^6-A^3 \quad \text{(Ie)}$$

worin $X^5$ für eine Gruppe der Formel -O-, -S- oder $-N(R^{14})-$ und $X^6$ für eine Gruppe der Formel -O- oder $-N(R^{14})-$, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und
$R^{10}$ Wasserstoff oder freies, verestertes oder amidiertes Carboxy bedeuten, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ von Acyl, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies oder veräthertes Hydroxy oder Mercapto, durch von der Gruppe der Formel Id verschiedenes verestertes Hydroxy oder Mercapto, durch freies oder von der Gruppe der Formel Id verschiedenes substituiertes Amino, oder durch Cycloalkyl, carbocyclisches Aryl oder stickstoffhaltiges Heteroaryl mit 5 bis 6 Ringgliedern im heterocyclischen Ring substituiert ist, oder $R^7$ von Wasserstoff verschieden ist, und Salze von solchen Verbindungen.

Die Erfindung umfasst ebenfalls die obengenannten Verbindungen als Immunomodulatoren, insbesondere als Immunstimulantien, ferner ihre Verwendung als pharmakologisch wirksame Substanzen, in erster Linie ihre Verwendung als Immunomodulatoren, insbesondere als Immunstimulantien, und ihre Verwendung zur Herstellung von pharmazeutischen Präparaten, sowie pharmazeutische Präparate, die diese Verbindungen enthalten.

Acyl, z.B. als $R^1$, $R^2$, $R^{12}$ und $R^{13}$,bedeutet in erster Linie den Acylrest einer organischen Carbonsäure, insbesondere einer aliphatischen, ferner einer cycloaliphatischen, cycloaliphatisch-aliphatischen, aromatischen oder araliphatischen Carbonsäure, die z.B. bis zu 90 Kohlenstoffatome aufweisen kann.

Aliphatische Carbonsäuren sind u.a. unsubstituierte oder, z.B. durch Hydroxy oder veräthertes oder verestertes Hydroxy, wie Niederalkoxy oder Niederalkanoyloxy, ferner unsubstituiertes oder substituiertes Amino, wie Niederalkylamino, Diniederalkylamino oder Acylamino, z.B. Alkanoylamino, substituierte Alkancarbonsäuren, sowie entsprechende Alken- oder Alkincarbonsäuren, die eine oder mehrere Doppel- bzw. Dreifachbindungen aufweisen können. Diese Säuren können z.B. bis zu 90 Kohlenstoffatome enthalten, wobei $R^2$ als Rest einer aliphatischen Carbonsäure, falls $X^1$ für eine Gruppe der Formel $-N(R^{14})-$ steht, vorzugsweise den Acylrest einer unsubstituierten oder Hydroxy-substituierten Niederalkancarbonsäure bedeutet.

Cycloaliphatische Carbonsäuren können mono- oder polycyclisch sein und als cycloaliphatischen Rest unsubstituiertes oder,z.B. durch Hydroxy,substituiertes mono- oder polycyclisches Cycloalkyl, sowie entsprechendes Cycloalkenyl enthalten.

In cycloaliphatisch-aliphatischen Resten haben der cycloaliphatische und der aliphatische Teil die oben gegebenen Bedeutungen; solche Reste sind insbesondere mono- oder polycyclisches Cycloalkyl-niederalkyl.

Aromatische und araliphatische Carbonsäuren sind u.a. unsubstituierte oder, z.B. durch Niederalkyl, Hydroxy, Niederalkoxy oder Halogen,substituierte Benzoe- oder Phenylniederalkancarbonsäuren.

Unter physiologischen Bedingungen abspaltbare Gruppen sind in erster Linie organische Silylgruppen, insbesondere aliphatisch substituierte Silylgruppen, wie Triniederalkylsilyl.

Substituenten von Alkylen, das durch die Reste $Y^1$, $Y^2$ bzw. $Y^3$ repräsentiert wird, sind u.a. Hydroxy, verestertes oder veräthertes Hydroxy, wie Acyloxy, z.B. Niederalkanoyloxy, oder Niederalkoxy, Amino oder substituiertes Amino, wie Niederalkylamino, Diniederalkylamino oder Acylamino, z.B. Niederalkanoylamino. In dem von Iminocarbonyl oder Oxycarbonyl unterbrochenen Alkylen können eine oder mehrere, z.B. zwei, solche Gruppierungen vorkommen, wobei sie als solche der Formel $-N(R^{14})-C(=O)-$ bzw. $-O-C(=O)-$, sowie als solche der Formel $-C(=O)-N(R^{14})-$ bzw. $-C(=O)-O-$ vorliegen können, und $R^{14}$ die oben gegebene Bedeutung hat und vorzugsweise für Wasserstoff steht. Ein durch die Gruppen $R^5$ und $R^6$ gebildetes Alkylen mit 3 bis 4 Kohlenstoffatomen in der Kette kann z.B. durch Hydroxy, das, z.B. durch eine Gruppe der Formel Ia, acyliert sein kann, substituiert sein.

Ein die Gruppe $R^{15}$ darstellender oder ein, eine Hydroxygruppe in einem Rest $R^{16}$ und $R^{17}$ veräthernder aliphatischer Rest mit mindestens 7 Kohlenstoffatomen ist in erster Linie ein entsprechender unsubstituierter oder substituierter Alkylrest, kann aber auch für einen entsprechenden ungesättigten Rest, wie einen unsubstituierten oder substituierten, eine oder mehrere Doppelbindungen aufweisenden Alkenylrest stehen, wobei solche Reste z.B. von 7 bis und mit 90, vorzugsweise von 7 bis und mit 30 Kohlenstoffatome aufweisen. Substituenten von solchen aliphatischen Resten sind z.B. Hydroxy, veräthertes oder verestertes Hydroxy, wie Niederalkoxy oder Niederalkanoyloxy und/oder unsubstituiertes oder substituiertes Amino, wie Niederalkylamino, Diniederalkylamino oder Alkanoylamino.

Ein entsprechender cycloaliphatischer Rest, der die Gruppe $R^{15}$ oder den eine Hydroxygruppe in den Resten $R^{16}$ bzw. $R^{17}$ veräthernden Rest darstellt, ist insbesondere mono- oder polycyclisches Cycloalkyl, ferner entsprechendes Cycloalkenyl, das eine oder mehrere Doppelbindungen aufweisen kann. Dabei enthalten solche Reste mindestens 7, vorzugsweise 7 bis 30 Kohlenstoffatome, und können zudem, z.B. durch Hydroxy, veräthertes oder verestertes Hydroxy, wie Niederalkoxy oder

Niederalkanoyloxy, oder unsubstituiertes oder substituiertes Amino, wie Niederalkylamino, Diniederalkylamino oder Alkanoylamino substituiert sein.

Veräthertes Hydroxy oder substituiertes Amino als Rest $R^9$ oder $R^{11}$ ist z.B. Niederalkoxy bzw. z.B. Niederalkylamino, worin Niederalkyl substituiert sein kann.

Die eine Hydroxygruppe in den Resten $R^{16}$ bzw. $R^{17}$ veresternden Reste sind in erster Linie Acylreste von organischen Carbonsäuren, insbesondere der obengenannten aliphatischen, sowie cycloaliphatischen, cycloaliphatisch-aliphatischen, aromatischen oder araliphatischen Carbonsäuren, vorzugsweise mit 7 bis 90 Kohlenstoffatomen.

Veräthertes bzw. vom Rest der Formel Id verschiedenes verestertes Hydroxy oder Mercapto als Substituent von Niederalkyl $R^6$ ist z.B. Niederalkoxy, Acyloxy, wie Alkanoyloxy, worin Alkanoyl bis zu 90, z.B von 7 bis 30, Kohlenstoffatome enthalten und gegebenenfalls, z.B. durch Hydroxy, substituiert sein kann, oder Halogen, ferner Niederalkylthio oder Acylthio, wie Alkanoylthio, worin Alkanoyl bis zu 90, z.B. von 7 bis 30, Kohlenstoffatome enthält. Vom Rest der Formel Id verschiedenes substituiertes Amino als Substituent einer Niederalkylgruppe $R^6$ ist z.B. Niederalkylamino, Guanylamino oder Acylamino, wie Alkanoylamino, worin Alkanoyl bis zu 90, z.B. bis zu 30, Kohlenstoffatome enthalten kann. Verestertes Carboxy als Substituent eines Niederalkylrestes $R^6$ ist vorzugsweise ein mit einem aliphatischen Rest, wie Alkyl mit bis zu 30 Kohlenstoffatomen, verestertes Carboxy, d.h. z.B. entsprechendes Alkoxycarbonyl, während entsprechendes amidiertes Carboxy z.B. Aminocarbonyl oder Niederalkylaminocarbonyl ist, worin Niederalkyl z.B. durch Carboxy, Alkoxycarbonyl oder Aminocarbonyl substituiert sein kann. Verestertes bzw. amidiertes Carboxy im Rest $R^6$ kann auch einen Rest der Formel

$$-\overset{O}{\overset{\|}{C}}-E-(Y^2-X^4)_m-A^2 \qquad \text{(Ida)}$$

darstellen, worin m, E, $Y^2$, $X^4$ und $A^2$ die oben gegebenen Bedeutungen haben.

Aryl als Substituent einer Niederalkylgruppe $R^6$ ist in erster Linie unsubstituiertes oder, z.B. durch Niederalkyl, Hydroxy oder veräthertes oder verestertes Hydroxy, wie Niederalkoxy oder Halogen, substituiertes Phenyl, während stickstoffhaltiges Heteroaryl mit 5 bis 6 Ringgliedern im heterocyclischen Ring als entsprechender Substituent von $R^6$ mono- oder bicyclisches, ein oder zwei Stickstoffatome als Ringglieder enthaltendes Heteroaryl darstellt.

Veräthertes Mercapto als Rest $R^9$ oder $R^{11}$ ist insbesondere Niederalkylthio, während in einem Niederalkylaminorest $R^9$ oder $R^{11}$ die Niederalkylgruppe z.B. durch Carboxy, Niederalkoxycarbonyl oder Aminocarbonyl substituiert sein kann.

Verestertes Carboxy $R^{10}$ ist insbesondere Niederalkoxycarbonyl, während amidiertes Carboxyl $R^{10}$ Carbamoyl oder N-Niederalkyl-carbamoyl sein kann, worin Niederalkyl z.B. durch Carboxy, Niederalkoxycarbonyl oder Aminocarbonyl substituiert sein kann.

In Verbindungen der Formel I mit freien funktionellen Gruppen, wie Hydroxy, Mercapto, Amino und Carboxyl, können solche Gruppen in geschützer Form vorliegen. Dabei sind Hydroxy und Mercapto vorzugsweise in acylierter oder verätherter Form geschützt, während Amino vorzugsweise in acylierter und Carboxy in veresterter Form geschützt sind.

Die oben verwendeten Allgemeinbegriffe haben im Zusammenhang mit der vorliegenden Beschreibung die folgenden Bedeutungen, wobei mit "nieder" bezeichnete Reste und Verbindungen bis und mit 7, vorzugsweise bis und mit 4 Kohlenstoffatome enthalten:

Alkyl ist z.B. Niederalkyl, wie Methyl, Aethyl, n-Propyl, Isopropyl, n-Butyl, Tributyl, sek.-Butyl oder tert.-Butyl, sowie n-Pentyl, Neopentyl, n-Hexyl oder n-Heptyl, oder Höheralkyl, wie geradkettiges oder verzweigtes Octyl, Nonyl, Decyl, Undecyl, Dodecyl, Tridecyl, Tetradecyl, Pentadecyl, Hexadecyl, Heptadecyl, Nonadecyl oder Heneicosyl, sowie Höheralkyl der Triacontyl-, Tetracontyl-, Pentacontyl-, Hexacontyl-, Heptacontyl-, Octacontyl- oder Nonacontylserien.

Niederalkoxy ist z.B. Methoxy, Aethoxy, n-Propyloxy, Isopropyloxy, n-Butyloxy, Isobutyloxy oder tert.-Butyloxy.

Alkanoyloxy ist Nieder- oder Höheralkanoyloxy, wobei Niederalkanoyloxy z.B. Formyloxy, Acetoxy, Propionyloxy oder Butyryloxy ist, während Höheralkanoyloxy z.B. Lauryloxy, Myristinoyloxy, Palmitoyloxy, Stearoyloxy oder Behenoyloxy ist. Durch Hydroxy substituiertes Alkanoyloxy, z.B. Höheralkanoyloxy, ist u.a. Mycoloyloxy.

Niederalkylamino ist z.B. Methylamino, Aethylamino, n-Propylamino oder Isopropylamino. Di-niederalkyl-amino ist z.B. Dimethylamino, Diäthylamino oder Di-isopropylamino. Alkanoylamino ist Niederalkanoylamino, z.B. Formylamino, Acetylamino oder Propionylamino, oder Höheralkanoylamino, z.B. Laurylamino, Palmitoylamino, Stearoylamino oder Behenoylamino.

Eine Alkancarbonsäure ist z.B. eine Niederalkancarbonsäure, wie Essigsäure, Propionsäure, Buttersäure oder Capronsäure, oder eine Höheralkancarbonsäure, wie Laurinsäure, Myristinsäure, Palmitinsäure, Stearinsäure oder Behensäure, während z.B. eine durch Hydroxy substituierte Alkansäure u.a. Mycolsäure sein kann.

Alken- und Alkincarbonsäuren sind u.a. Niederalken- bzw. Niederalkincarbonsäuren, wie Acrylsäure, Crotonsäure oder Tetrolsäure, oder Höheralken- bzw. Höheralkincarbonsäuren, wie Undecylensäure, Oelsäure oder Elaidinsäure. Der Acylrest einer Niederalkancarbonsäure, welcher die Gruppe $R^2$ darstellt, falls $X^1$ für den Rest der Formel $-N(R^{14})-$ steht, ist in erster Linie Acetyl oder Hydroxyacetyl, sowie Propionyl.

Cycloalkyl ist z.B. Cyclopentyl, Cyclohexyl oder Adamantyl, während Cycloalkenyl z.B. 1-Cyclohexenyl, und Cycloalkylniederalkyl z.B. 3-Cholanylmethyl oder der Acylrest der Cholansäure sein kann.

Phenylniederalkancarbonsäuren sind z.B. Phenylessigsäure oder Phenylpropionsäure, die, z.B. wie angegeben, substituiert sein können.

Halogen ist vorzugsweise Halogen mit einer Ordnungszahl bis zu 35 und steht besonders für Chlor, ferner auch für Fluor oder Brom.

Triniederalkylsilyl ist in erster Linie Trimethylsilyl.

Alkylen ist geradkettig oder verzweigt und ist insbesondere Niederalkylen, z.B. Methylen, Aethylen, 1,2-Propylen, 1,3-Propylen oder 1,6-Hexylen, ferner auch Höheralkylen, wie 1,11-Undecylen.

Alkenyl ist Niederalkenyl, z.B. Allyl oder Methallyl, oder Höheralkenyl, z.B. Decenyl.

Niederalkylthio ist z.B. Methylthio oder Aethylthio.

In einem Alkanoylthiorest bedeutet der Alkanoylrest Niederalkanoyl, z.B. Acetyl, Propionyl, Butyryl oder Hexanoyl, kann aber auch Höheralkanoyl, z.B. Lauryl, Myristinoyl, Palmitoyl, Stearoyl oder Behenoyl, sein.

Alkoxycarbonyl ist Niederalkoxycarbonyl, z.B. Methoxycarbonyl, Aethoxycarbonyl oder tert.-Butyloxycarbonyl, oder Höheralkoxycarbonyl, z.B. Dodecyloxycarbonyl, Tetradecyloxycarbonyl, Hexadecyloxycarbonyl oder Heneicosyloxycarbonyl.

Niederalkylaminocarbonyl ist z.B. Methylaminocarbonyl oder Aethylaminocarbonyl, ferner Carboxy-, Niederalkoxycarbonyl- oder Carbamoylniederalkylaminocarbonyl, wie Carboxymethylaminocarbonyl, 1-Carboxyäthylaminocarbonyl, Methoxycarbonylmethylaminocarbonyl oder Carbamoylmethylaminocarbonyl.

Stickstoffhaltiges Heteroaryl mit 5 bis 6 Ringgliedern im heterocyclischen Ring ist z.B. Imidazolyl, wie 4-Imidazolyl, oder Indolyl, wie 3-Indolyl.

In den Verbindungen der Formel I mit freien funktionellen Gruppen, wie Carboxyl, Amino, Hydroxy oder Mercapto, können solche Gruppen, wie obenerwähnt, in geschützer Form vorliegen, wobei als Schutzgruppen die in der Peptid-, Penicillin-, Cephalosporin- und Zuckerchemie üblicherweise verwendeten Schutzgruppen zur Anwendung kommen.

Solche Schutzgruppen sind leicht, das heisst, ohne dass unerwünschte Nebenreaktionen stattfinden, beispielsweise solvolytisch, reduktiv oder photolytisch, abspaltbar.

Schutzgruppen dieser Art, sowie ihre Abspaltung sind beispielsweise in "Protective Groups in Organic Chemistry" (Plenum Press, London, New York, 1973), ferner in Schröder and Lübke "The Peptides", Bd. I (Academic Press, London, New York 1965), sowie in Houben-Weyl, "Methoden der organischen Chemie", Bd. 15/1 (4. Auflage, Georg Thieme Verlag, Stuttgart 1974) beschrieben.

So sind Carboxylgruppen üblicherweise in veresterter Form geschützt, die als veresternde Gruppen in erster Linie in 1-Stellung verzweigte oder in 1- oder 2-Stellung geeignet substituierte Niederalkylgruppen enthalten. Bevorzugte, in veresterter Form geschützte Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls, z.B. durch Niederalkyl, Niederalkoxy, Hydroxy, Halogen und/oder Nitro, substituierte Phenylreste darstellen, wie unsubstituiertes oder,z.B. wie oben erwähnt, substituiertes Benzyloxycarbonyl, z.B. Benzyloxycarbonyl, 4-Nitro-benzyloxycarbonyl oder 4-Methoxy-benzyloxycarbonyl, oder unsubstituiertes oder,z.B. wie oben erwähnt, substituiertes Diphenylmethoxycarbonyl, z.B. Diphenylmethoxycarbonyl oder Di-(4-methoxyphenyl)-methoxycarbonyl, Aroylmethoxycarbonyl, worin die Aroylgruppe unsubstituiertes oder,z.B. durch Halogen,substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen unsubstituierten oder, z.B. durch Niederalkyl, Phenyl, Niederalkoxy, Halogen und/oder Nitro,substituierten aliphatischen, araliphatischen, cycloaliphatischen oder aromatischen Kohlenwasserstoffrest mit z.B. bis zu 15 Kohlenstoffatomen, wie entsprechendes unsubstituiertes oder substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen. In diesen enthält das Siliciumatom vorzugsweise Niederalkyl, insbesondere Methyl, ferner Niederalkoxy, z.B. Methoxy, und/oder Halogen, z.B. Chlor, als Substituenten. Geeignete Silylschutzgruppen sind in erster Linie Triniederalkylsilyl, insbesondere Trimethylsilyl und Dimethyl-tert.-butylsilyl.

Bevorzugte Carboxylgruppen in geschützter Form sind tert.-Niederalkoxycarbonyl, wie tert.-Butyloxycarbonyl, und in erster Linie unsubstituiertes oder z.B. wie oben erwähnt, substituiertes Benzyloxycarbonyl oder Diphenylmethoxycarbonyl.

Eine geschützte Aminogruppe kann z.B. in Form einer leicht spaltbaren Acylamino-, ferner als Arylmethylamino- oder Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer vorzugsweise, z.B. durch Halogen oder Aryl, substituierten Alkancarbonsäure, oder eines Kohlensäurehalbesters. Solche Acylgruppen sind beispielsweise Niederalkanoyl, wie Formyl oder Acetyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die unsubstituiert sind oder, z.B. durch Niederalkyl, insbesondere tert.-Niederalkyl, Niederalkoxy, Hydroxy, Halogen und/oder Nitro, substituiertes Phenyl darstellen, wie unsubstituiertes oder substituiertes Benzyloxycarbonyl, z.B. Benzyloxycarbonyl oder 4-Nitrobenzyloxycarbonyl, oder Diphenylmethoxycarbonyl, z.B. Benzhydroxycarbonyl oder Di-(4-methoxyphenyl)-methoxycarbonyl, Aroylmethoxycarbonyl, worin die Aroylgruppe unsubstituiertes oder, z.B. durch Halogen, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, wie z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Eine Arylmethylaminogruppe ist eine Mono-, Di- oder insbesondere Triarylmethylaminogruppe, worin die Arylreste insbesondere unsubstituierte oder substituierte Phenylreste sind. Solche Gruppen sind beispielsweise Benzyl-, Diphenylmethyl- und insbesondere Tritylamino.

Aminogruppen können auch organische Silylgruppen als Schutzgruppen enthalten. Geeignete Silylschutzgruppen sind in erster Linie Triniederalkylsilyl, insbesondere Trimethylsilyl und Dimethyl-tert.-butylsilyl.

Bevorzugte Aminoschutzgruppen sind Acylreste von Kohlensäurehalbestern, insbesondere tert.-Butyloxycarbonyl, unsubstituiertes oder, z.B. wie angegeben, substituiertes Benzyloxycarbonyl, z.B. Benzyloxycarbonyl oder 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Hydroxy-, sowie Mercaptoschutzgruppen sind z.B. Acylreste, wie unsubstituiertes oder,z.B. durch Halogen, substituiertes Niederalkanoyl, wie 2,2-Dichloracetyl, oder insbesondere die im Zusammenhang mit den Aminoschutzgruppen genannten Acylreste von Kohlensäurehalbestern, insbesondere 2,2,2-Trichloräthoxycarbonyl, ferner leicht abspaltbare veräthernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyäthyl, 1-Aethoxyäthyl, 1-Methylthiomethyl, 1-Methylthio-äthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl,oder entsprechende Thiaanaloge, sowie unsubstituiertes oder substituiertes 1-Phenylniederalkyl, wie unsubstituiertes oder substituiertes Benzyl, Diphenylmethyl oder Trityl, wobei als Substituenten der Phenylreste z.B. Halogen, Niederalkoxy und/oder Nitro in Frage kommen.

Hydroxy-, sowie Mercaptogruppen können auch in Form von entsprechenden organischen Silyloxy- oder Silylthiogruppen geschützt sein. Geeignete Silylschutzgruppen sind in erster Linie Triniederalkylsilyl, insbesondere Trimethylsilyl oder Dimethyl-tert.-butylsilyl.

Dabei können zwei freie funktionelle Gruppen auch mittels einer gemeinsamen, leicht abspaltbaren Schutzgruppe geschützt sein. So können z.B. durch die Reste $R^{12}$-O- und $R^{13}$-$X^2$- dargestellte Hydroxygruppen durch einen unsubstituierten oder vorzugsweise, z.B. durch Niederalkyl, wie Methyl, oder Aryl, wie Phenyl, substituierten Methylenrest, wie Methylen, Isopropyliden, Propyliden oder Benzyliden, geschützt sein.

Die Hexapyranose-Verbindungen der Formel I können in Form von Isomerengemischen oder reinen Isomeren vorliegen.

So können sie im Zuckerteil die L- oder die DL-Konfiguration haben, weisen jedoch vorzugsweise die D-Konfiguration auf. Ferner kann der Hexapyranoseteil derjenige irgendeiner Hexose sein, ist jedoch vorzugsweise derjenige einer Allose, Galactose oder Mannose, in erster Linie aber einer Glucose. D.h. die Verbindungen der vorliegenden Erfindung sind insbesondere entsprechende Allo-, Galacto- oder Mannopyranose-Verbindungen, in erster Linie aber entsprechende Glucopyranose-Verbindungen, die vorzugsweise die D-Konfiguration aufweisen.

Ferner liegt der mit dem Sauerstoffatom verknüpfte Rest der Formel $-C(R^3)(R^4)-C(=O)-$, falls eine der Gruppen $R^3$ und $R^4$ von Wasserstoff verschieden ist, vorzugsweise in optisch aktiver Form vor und hat insbesondere die D-Form, während der Rest der Aminosäure der Formel $-N(R^5)-C(R^6)(R^7)-C(=O)-$, falls einer der Reste $R^6$ und $R^7$ von Wasserstoff verschieden ist, ebenfalls vorzugsweise in optisch aktiver, in erster Linie in der L-Form vorliegt, und der terminale α-Aminoglutarsäurerest vorzugsweise in optisch aktiver, insbesondere in der D-Form vorliegt. Ferner kann die gegebenenfalls substituierte 1-Hydroxygruppe der Formel $-O-R^1$ die α- oder die β-Konfiguration haben;

die neuen Verbindungen der Formel I können jedoch auch als Gemisch der 1-α- und 1-β-Isomeren vorliegen.

In den Verbindungen der Formel I kann das über ein Sauerstoffatom an den Phosphor gebundene Proton leicht durch ein Kation ersetzt werden, d.h. die Verbindungen bilden Salze.Dabei können die Verbindungen der Formel I in Form eines Gemisches der freien Verbindungen und ihrer Salze vorliegen;letztere gehören ebenfalls zum Gegenstand der Erfindung.Insbesondere betrifft die Erfindung pharmazeutisch verwendbare, nicht toxische Salze der Verbindungen der Formel I. Es sind dies in erster Linie Metall- oder Ammoniumsalze, wie Alkalimetall- und Erdalkalimetall-, z.B. Natrium-, Kalium-, Magnesium- oder Calciumsalze, sowie Ammoniumsalze oder Salze mit geeigneten organischen Aminen, wie Niederalkylaminen, z.B. Triäthylamin. Verbindungen der Formel I mit basischen Gruppen, z.B. Aminogruppen, liegen als innere Salze vor, können aber, wenn mehr basische als saure Gruppen in einem Molekül der Formel I vorhanden sind, zusätzlich auch Säureadditionssalze mit externen Säuren bilden, wie Salze mit anorganischen Säuren, wie Mineralsäuren, z.B. Salz-, Schwefel- oder Phosphorsäure, oder organischen Carbon- oder Sulfonsäuren, z.B. Essig-, Malein-, Fumar-, Wein-, Zitronen-, Methansulfon- oder 4-Toluolsulfonsäure. Zur Isolierung oder Reinigung können auch pharmazeutisch ungeeignete Salze Verwendung finden. Zur therapeutischen Anwendung gelangen jedoch nur die pharmazeutisch verwendbaren, nicht toxischen Salze, die deshalb bevorzugt werden.

Die neuen Verbindungen der vorliegenden Erfindung weisen eine Reihe wertvoller pharmakologischer Eigenschaften, insbesondere ausgeprägte immunomodulatorische, z.B. immunpotenzierende, Wirkungen auf.

So wird durch diese Verbindung in vivo die Antikörperbildungsfähigkeit von Mäusen erheblich gesteigert, was anhand der folgenden Versuchsanordnung nachgewiesen werden kann:

NMRI Mäusen werden durch intraperitoneale Injektion von 10 µg präzipitatfreiem Bovin-Serum-Albumin (BSA) am Tag 0 immunisiert. 9, 15 und 29 Tage später werden Serumproben entnommen und auf ihren Gehalt an anti-BSA-Antikörpern mit einer passiven Haemagglutinationstechnik

untersucht. In der verwendeten Dosis ist lösliches BSA für die Empfängertiere subimmunogen, d.h. es vermag keine oder nur eine ganz geringfügige Produktion von Antikörpern auszulösen. Zusätzliche Behandlung der Mäuse mit immunpotenzierenden Stoffen vor oder nach der Antigengabe führt zu einem Anstieg der Antikörpertiter im Serum. Der Effekt der Behandlung wird durch den erreichten Scorewert, d.h. durch die Summe der $log_2$ Titerdifferenzen an den drei Blutungstagen ausgedrückt.

In diesem Test sind die Verbindungen der Formel I in der Lage, bei intraperitonealer oder subkutaner Applikation von 0,5 - 5 mg/kg an fünf aufeinander folgenden Tagen nach Immunisierung mit BSA die Antikörperproduktion gegen BSA signifikant zu steigern.

Auch Manifestationen der zellvermittelten Immunität können durch die genannten Verbindung in vivo potenziert werden, was anhand des folgenden Experimentes nachgewiesen werden kann:

Während die Sensibilisierung von Meerschweinchen mit BSA in inkomplettem Freund'schem Adjuvans nur zu humoraler Antikörperbildung führt, induziert die Beimischung der erfindungsgemässen Phosphorylmuramylpeptide in einem Dosisbereich von 5 bis 50 µg zur Antigen-Oelemulsion Spättyp-Ueberempfindlichkeit gegenüber BSA; 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund'schem Adjuvans (d.h. mit Zusatz von Mykobakterien) erhalten werden. Die $ED_{50}$-Werte (benötigte µg/Tier zur Induktion einer Differenz des Reaktionsvolumens [Erythemfläche x Hautdickenzunahme] bei behandelten und unbehandelten Tieren von 200 µl, 24 Stunden nach Auslösung) betragen für die Verbindungen der vorliegenden Erfindung etwa 10 bis etwa 20 µg.

Besonders hervorzuheben ist auch die Fähigkeit der Verbindungen der vorliegenden Erfindung durch Applikation zusammen mit BSA in Liposomen (Eilecithin : Cholesterin 4:1; 4 mg/Tier) und ohne die toxische

Mineralöl-Komponente in Meerschweinchen eine Spättypüberempfindlichkeit gegen BSA zu induzieren. Quantitativ und qualitativ sind diese Spättyp-Reaktionen ebenfalls identisch mit denjenigen, die durch Immunisierung mit BSA in komplettem Freund'schen Adjuvans erhalten werden. Die $ED_{50}$-Werte betragen 100-300 µg pro Tier.

Die neuen Verbindungen der vorliegenden Erfindung sind auch in der folgenden Versuchsanordnung wirksam:

Balb/c Mäuse werden durch intraperitoneale Injektion von 2 x $10^4$ P815 Mastocytomzellen am Tag 0 immunisiert. Am Tag 15 werden die Milzzellen der so immunisierten Tiere in vitro auf das Vorhandensein zytotoxischer, gegen P815 Mastocytom-Zellen gerichteter T-Lymphocyten untersucht. Dazu werden die P815 Zielzellen mit $^{51}Cr$ markiert und das Ausmass der zytotoxischen Reaktion durch Messen der Radioaktivität im Kulturüberstand ermittelt. In der verwendeten Dosis sind die P815 Mastozytomzellen für die Empfänger-Mäuse subimmunogen, d.h. sie induzieren keine oder nur ganz geringe Bildung zytotoxischer T-Zellen. Gleichzeitige intraperitoneale Applikation von 1 bis 50 µg der Verbindungen der vorliegenden Erfindung führen zu einer signifikanten Steigerung der Bildung zytotoxischer T-Zellen (Faktor 10 bis 30 gegenüber unbehandelten Mäusen).

Die immunpotenzierenden Eigenschaften der neuen Verbindungen der vorliegenden Erfindung lassen sich auch im Falle der Induktion spezifischer Immuntoleranz gegen Transplantationsantigene durch Immunisierung mit adjuvierten Autoblasten bei der Maus nachweisen:

In einer gemischten Lymphocytenkultur werden Milzlymphocyten des zukünftigen Transplantat-Empfängers (C57Bl/6J Mäuse) mit bestrahlten Milzzellen des zukünftigen Transplantat-Spenders (CBA/J Mäuse) inkubiert. T-Lymphocyten mit spezifischen Rezeptoren für die Histokompatibilitätsantigene des Spenders proliferieren und werden zu Blasten; diese können durch Sedimentation von den andern Zellen abgetrennt werden. Die spezifischen Blasten exprimieren die relevanten idiotypischen Spezifitäten der Membranrezeptoren und werden adjuviert mit komplettem Freund'schen Adjuvans (CFA) als Autoimmunogene zur Induktion spezifischer Toleranz gegen die betreffenden Transplantationsantigene in die prospektiven Transplantat-Empfänger (C 57 Bl/6J) injiziert. Die Immunisierung erfolgt viermal in vierwöchigen Abständen mit autologen anti-CBA/J T Lymphoblasten. Adsorbate von T-Autoblasten mit den neuen Verbindungen der Formel (I) ($10^9$ Blasten werden in einer Lösung von 20 mg Substanz in 20 ml PBS suspendiert. Nach zweistündiger Inkubation werden die Zellen zentrifugiert und zweimal mit PBS gewaschen.) sind in der Lage, spezifische Immuntoleranz in Abwesenheit von CFA zu induzieren, wobei die Adsorbate gleich wirksam sind wie Lymphoblasten in CFA.

Die Verbindungen der vorliegenden Erfindung sind ausserdem in der Lage, in Konzentrationen von etwa 0,5 bis etwa 100 ug/ml in Milzzellkulturen von normalen Mäusen die Bildung antikörperproduzierender Zellen zu induzieren (Vermehrung der 19S-plaquebildenden Zellen um einen Faktor 10 bis 30 über den Kontrollwert [in Abwesenheit der stimulierenden Substanzen]). So werden in Anwesenheit der neuen Verbindungen z.B. spezifische Antikörper gegen Schaferythrocyten gebildet, ohne dass den Kulturen Schaferythrocyten zur Immunisierung zugesetzt werden. Andererseits vermögen die genannten Substanzen im selben Konzentrationsbereich auch die immunologische Reaktivität von T-zellverarmten Milzzellkulturen (von kongenital athymischen nu/nu Mäusen) gegenüber einem normalerweise thymusabhängigen Antigen (Schaferythrocyten) zu steigern (Faktor 10 bis 30 gegenüber unbehandelten Kontrollkulturen). Durch die genannten Verbindungen werden aber in vitro direkt oder indirekt nicht nur Proliferations- und Syntheseleistungen von B-Lymphocyten (d.h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören) vermittelt. So vermögen z.B. die neuen Verbindungen in einem Konzentrationsbereich von etwa 1 bis etwa 20 µg/ml die Reaktivität von cortisonresistenten Thymuszellen gegenüber allogenen bestrahlten Stimulatorlymphocyten erheblich (bis zu 10-fach) zu potenzieren.

Die oben erwähnten Wirkungen kommen wahrscheilich indirekt dadurch zustande, dass die Verbindungen der vorliegenden Erfindung Makrophagen aktivieren, die ihrerseits die Reaktivität von T- und B-Lymphocyten fördern. Tatsächlich kann man zeigen, dass die genannten Verbindungen bereits in geringen Konzentrationen (0,5 - 10 µg/ml) grosse Mengen "colony stimulating activity" (CSA) aus Maus-Makrophagen freisetzen (Induktion von bis zu 150 bis 200 Kolonien innert 7 Tagen aus $10^5$ Mäuse-Knochenmarkzellen, nach Zugabe von 20 % Ueberstand aus während 24 Stunden mit Substanz inkubierten Makrophagenkulturen, im Vergleich zu 0 bis 5 Kolonien bei Zugabe von Ueberständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die neuen Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirkung der neuen Verbindungen kann auch in vivo nachgewiesen werden: So führt die Injektion einer Verbindung der vorliegenden Erfindung innert 3 bis 9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5 % Endkonzentration] im Vergleich zu 0 bis 5 Kolonien bei unbehandelten Tieren). Dementsprechend wird durch Verabreichung derselben Verbindungen in vivo die Antikörperbildungsfähigkeit von Mäusen erheblich potenziert.

Die immunpotenzierenden Eigenschaften der neuen Verbindungen der vorliegenden Erfindung lassen sich auch in Tumormodellen demonstrieren, so z.B. beim Ehrlich Ascites Tumor in der Maus:

Eine intraperitoneale Injektion von $10^6$ syngenen Ehrlich Ascites Tumorzellen führt bei Balb/c Mäusen durchschnittlich in 18 Tagen zum Absterben der Tiere. Injiziert man den Mäusen intraperitoneal $10^7$ (Gruppe 1), $10^6$ (Gruppe 2) und $10^5$ (Gruppe 3) Ascites Tumorzellen, die man in vitro mit den neuen Verbindungen der Formel I beladen hat ($10^9$ Ascites Tumorzellen werden in einer Lösung von 40 mg der Prüfsubstanz in 20 ml phosphat-gepufferter physiologischer Kochsalzlösung (PBS) suspendiert und nach zweistündiger Inkubation bei 37°C werden die Zellen zentrifugiert und zweimal mit PBS gewaschen; die Zellen inkorporieren bei dieser Behandlung die Prüfverbindung in ihre Membran), so kommt es in 18 Tagen zu keinem Tumorwachstum. Am 19. Tag werden die Tiere jeweils mit $10^6$ nativen Ehrlich Ascites Tumorzellen intraperitoneal belastet. Folgende Effekte werden beobachtet:

Gruppe 1 : 8 von 10 Tieren überleben den 80. Tag
Gruppe 2 : 6 von 10 Tieren überleben den 80. Tag
Gruppe 3 : die Tiere sterben, wie die Kontrolltiere, nach 18 Tagen.

Die Verbindungen gemäss der vorliegenden Erfindung sind zudem wenig toxisch: Auch 5malige intraperitoneale Applikation in einer Dosis von 100 mg/kg/Tag an fünf aufeinanderfolgenden Tagen wurde von Mäusen anscheinend symptomlos vertragen. Da die für die Immunstimulation benötigten Dosen sehr gering sind, ist die therapeutische Breite der neuen Verbindungen sehr gross.

Die neuen Verbindungen gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Verbindungen gemäss der vorliegenden Erfindung können somit als Adjuvantien in Mischung mit Impfstoffen dazu benützt werden, den Impferfolg zu verbessern und den durch humorale Antikörper und/

oder zelluläre Immunität vermittelten Infektionsschutz gegenüber bakteriellen, viralen oder parasitären Erregern zu verbessern.

Schliesslich eignen sich die neuen Verbindung in Mischung mit verschiedenen Antigenen als Adjuvantien bei der experimentellen und industriellen Herstellung von Antiseren für Therapie und Diagnostik und bei der Induktion von immunologisch aktivierten Lymphocytenpopulationen für Zelltransferverfahren.

Darüberhinaus können die neuen Verbindungen auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Hormonen auftreten. Die genannten Verbindungen können somit vorzugsweise auch in Kombination mit Antibiotika, Chemotherapeutika oder anderen Heilmitteln verabreicht werden. Schliesslich sind die beschriebenen neuen Verbindungen auch zur allgemeinen Prophylaxe von Infektionskrankheiten bei Mensch und Tier geeignet.

Die Kombination der erfindungsgemässen neuen Verbindungen mit Antibiotika gehöhrt zum Gegenstand der Erfindung und bewirkt eine Steigerung der antibiotischen Aktivität. Zu diesem Zwecke verwendet man eine wirksame oder unter-wirksame Dosis des Antibiotikums, je nach Art des letzteren z.B. von etwa 20 bis etwa 750 mg pro Einzeldosis.

Die Verbindungen der vorliegenden Erfindung werden in Einzeldosen von etwa 0.1 mg bis ungefähr zur halben Antibiotikamenge, vorzugsweise von 0.5 bis 10 mg, verwendet. Dabei kann die neue Verbindung bis zu 24 Stunden vor oder nach, in vielen Fällen bis zu 7 Tagen vor, vorzugsweise jedoch etwa gleichzeitig mit dem Antibiotikum verabreicht werden.

Die Verabreichung der Antibiotika erfolgt auf dem üblichen Wege, wie subkutan, intravenös oder oral, während man die neuen Verbindungen, insbesondere wenn sie getrennt von den Antibiotika verabreicht werden, meist subkutan verabfolgt.

Bei diesem Verfahren kann man einzelne Antibiotika, wie auch Antibiotikagemische verwenden. Antibiotikapräparate, welche dadurch gekennzeichnet sind, dass sie eines oder mehrere der vorgenannten Antibiotika und mindestens eine Verbindung der vorliegenden Erfindung enthalten, weisen die üblichen Mengen an Antibiotika, z.B. zwischen 20 und 1000 mg, bevorzugt zwischen etwa 200 und 500 mg auf, sowie 0.1 mg bis zur Hälfte der Antibiotikamenge, vorzugsweise 0.5 bis 10 mg, an Muramylpeptid der Formel I. Diese Präparate können, insbesondere wenn sie für orale Gabe zu verwenden sind, ausserdem noch die üblichen Mengen an pharmakologischen Trägerstoffen, Streck- und/oder Verdünnungsmitteln enthalten.

Die hohe antibiotische Wirkung einer Kombinationsbehandlung kann durch "in vivo"-Versuche nachgewiesen werden, die an verschiedenen Tierarten, insbesondere Säugetieren, wie Mäusen, durchgeführt werden. Dazu infiziert man sie mit einer letalen oder sub-letalen Dosis eines pathogenen Microorganismus und gibt dann das genannte neue Kombinationspräparat bzw. die einzelnen Dosen an neuer Verbindung und Antibiotikum. Die Wirkung wird als $ED_{50}$ bestimmt, das ist diejenige Dosis bei denen 50 % der Tiere überleben.

Es wurde nun überraschenderweise gefunden, dass die Infektion mit pathogenen Keimen, insbesondere der weniger beeinflussbaren gramnegativen Bakterien, wie z.B. Stämmen von Aerobakter, Brucella, Escherichia, Klebsiella, Malleomyces, Neisseria, Pasteurella, Proteus, Pseudomonas, Shigella und Vibrio, aber auch von gram-positiven Bakterien, wie von Aktinomycetes, Clostridia, Corynebakterien, Diplokokken, Mycobakterien oder Staphylococcen, oder von Pilzen, wie Candida Albicans, Cryptokokkus neoformans, Plastomyces dermatitides oder Hystoplasma capsulatum, in erhöhtem Masse gehemmt wird.

Von den zur Kombination mit den erfindungsgemässen Verbindungen in Frage kommenden Antibiotika sind besonders solche aus den Gruppen der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine zu nennen.

Als bevorzugte Antibiotika seien unter den β-Lactamen die Penicilline, Cephalosporine, Peneme, Nocardicine, Thienamycine und Clavulansäuren genannt.

Penicillin-Antibiotika sind in erster Linie Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo[5.2.2.$0^{2,6}$]undec-8-enyl)-methylenamino-penicillansäure.

Aus der Gruppe der Cephalosporine können z.B. Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7β-[D-2-amino-2-(1,4-cyclohexadienyl)-acetamido]-3-methoxy-3-cephem-4-carbonsäure), Cefsulodin, Cefotaxim, Cefotiam, Ceftezol oder Cefazedon genannt werden.

Unter den Nocardicinen ist z.B. Nocardicin A und unter den Thienamycinen und Clavulansäuren z.B. Thienamycin bzw. Clavulansäure zu erwähnen.

Unter den Aminoglycosiden sind insbesondere Streptomycine, z.B. Streptomycin und Streptomycin A, Neomycine, z.B. Neomycin B, Tobramycine, z.B. Tobramycin oder Dibekacin, Kanamycine (z.B. Gemische von Kanamycin A, B und C), sowie Amicacine, Gentamycine (z.B. Gemische von Gentamycin A, $C_1$, $C_2$ oder $C_{1a}$), oder Sisomicine, wie Sisomicin

oder Netilmicin, ferner Lividomycin, Ribocamycin und Paromomycin zu erwähnen.

Als Tetracycline sind insbesondere Tetracyclin, Doxycyclin, Chlortetracyclin, Oxytetracyclin oder Methacyclin zu nennen.

Als Macrolide sind z.B. Maridomycin, Spiramycine, wie Spiramycin I, II und III, Erythromycine, z.B. Erythromycin, Oleandomycine,z.B. Oleandomycin und Tetraacetyloleandomycin, und als Lincomycine z.B. Lincomycin und Clindamycin zu erwähnen.

Als Polyen-Antibiotika sind besonders Amphothericin B und dessen Methylester oder Nystalin zu nennen.

Als Polypeptid-Antibiotika können z.B. Colistin, Gramicidin S, Polymyxin B, Virginamycin, Tyrothricin, Viomycin oder Vancomycin besonders genannt werden.

Als Rifamycine kommen in erster Linie das Rifamycin S, Rifamycin SV oder Rifamycin B oder deren halbsynthetische Derivate,insbesondere das Rifampicin, in Frage.

Die vorliegende Erfindung betrifft insbesondere (a) Verbindungen der Formel I, worin $X^1$ und $X^2$ die oben gegebenen Bedeutungen haben, $R^1$ für den Rest der Formel Ia steht, worin n, $Z^1$, $Y^1$, $X^3$ und $A^1$ die oben gegebenen Bedeutungen haben, $R^2$, $R^{12}$ und $R^{13}$ unabhängig voneinander Wasserstoff, ein vom Rest der Formel Ia, worin n für 1 steht, verschiedenes Acyl oder eine unter physiologischen Bedingungen abspaltbare Gruppe darstellen, $R^3$, $R^4$,$R^5$,$R^7$,$R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes, substituiertes Amino, freies, verestertes oder amidiertes Carboxy,

Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie verschiedenes, veräthertes Hydroxy oder Mercapto oder freies oder von einem Rest der Formel Ie verschiedenes, substituiertes Amino bedeuten, wobei freie funktionelle Gruppen in geschützter Form vorliegen können, oder Salze, insbesondere pharmazeutisch verwendbare Salze von solchen Verbindungen.

Die vorliegende Erfindung betrifft ebenfalls insbesondere (b) Verbindungen der Formel I, worin $X^1$ und $X^2$ die oben gegebenen Bedeutungen haben, $R^2$ für den Rest der Formel Ia steht, worin n, $Z^1$, $Y^1$, $X^3$ und $A^1$ die oben gegebenen Bedeutungen haben, $R^1$, $R^{12}$ und $R^{13}$ unabhängig voneinander Wasserstoff, ein vom Rest der Formel Ia, worin n für 1 steht, verschiedenes Acyl oder eine unter physiologischen Bedingungen abspaltbare Gruppe darstellen, $R^3$, $R^4$, $R^5$, $R^7$, $R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie verschiedenes, veräthertes Hydroxy oder Mercapto oder freies oder von einem Rest der Formel Ie verschiedenes substituiertes Amino bedeuten, wobei freie funktionelle Gruppen in geschützter Form vorliegen können, oder Salze, insbesondere pharmazeutisch verwendbare Salze von solchen Verbindungen.

Die vorliegende Erfindung betrifft auch insbesondere (c) Verbindungen der Formel I, worin $X^1$ und $X^2$ die oben gegebenen Bedeutungen haben, $R^{13}$ für den Rest der Formel Ia steht, worin n, $Z^1$, $Y^1$, $X^3$ und $A^1$ die oben gegebenen Bedeutungen haben, $R^1$, $R^2$ und $R^{12}$ unabhängig voneinander Wasserstoff, ein vom Rest der Formel Ia, worin n für 1 steht, verschiedenes Acyl oder eine unter physiologischen Bedingungen abspaltbare Gruppe darstellen, $R^3$, $R^4$, $R^5$, $R^7$, $R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes Niederalkylen mit 3 bis 4 Kohlenstoffatomen in der Kette darstellen, und worin $R^9$ und $R^{11}$ unabhängig voneinander freies Hydroxy oder Mercapto, von einem Rest der Formel Ie verschiedenes veräthertes Hydroxy oder Mercapto oder freies oder von einem Rest der Formel Ie verschiedenes substituiertes Amino bedeuten, wobei freie funktionelle Gruppen in geschützter Form vorliegen können, oder Salze, insbesondere pharmazeutisch verwendbare Salze von solchen Verbindungen.

Die vorliegende Erfindung betrifft auch insbesondere (d) Verbindungen der Formel I, worin $X^1$ und $X^2$ die oben gegebenen Bedeutungen haben, $R^1$, $R^2$, $R^{12}$ und $R^{13}$ unabhängig voneinander Wasserstoff, vom Rest der Formel Ia, worin n für 1 steht, verschiedenes Acyl oder eine unter physiologischen Bedingungen abspaltbare Gruppe darstellen, $R^3$, $R^4$, $R^5$, $R^7$, $R^8$ und $R^{10}$ die oben gegebenen Bedeutungen haben, $R^6$ für durch den Rest der Formel Id substituiertes Niederalkyl steht, worin m, E, $Z^2$, $Y^2$, $X^4$ und $A^2$ die oben gegebenen Bedeutungen haben, oder durch die Gruppe der Formel Ida substituiertes Niederalkyl bedeutet, worin m, E, $Y^2$, $X^4$ und $A^2$ die oben gegebenen Bedeutungen haben, und worin $R^9$ und $R^{11}$ unabhängig voneinander freies Hydroxy oder Mercapto, von einem

Rest der Formel Ie verschiedenes veräthertes Hydroxy oder Mercapto, freies oder von einem Rest der Formel Ie verschiedenes substituiertes Amino bedeuten, wobei freie funktionelle Gruppen in geschützter Form vorliegen können, oder Salze, insbesondere pharmazeutisch verwendbare Salze von solchen Verbindungen.

Die Erfindung umfasst in erster Linie Hexapyranose-Verbindungen, insbesondere D-Glucopyranose-Verbindungen der Formel I, worin $X^1$ und $X^2$ die oben gegebenen Bedeutungen haben, $R^1$, $R^2$, $R^{12}$ und $R^{13}$ unabhängig voneinander für Wasserstoff, den Acylrest einer aliphatischen, cycloaliphatischen, aromatischen oder araliphatischen Carbonsäure, insbesondere einer unsubstituierten oder z.B. durch Hydroxy, Amino und/oder Alkanoylamino, wie Höheralkanoylamino, substituierten Alkancarbonsäure, mit bis zu 90 Kohlenstoffatomen, eine Tri-niederalkyl-silylgruppe oder einen Rest der Formel Ia stehen, worin n und $X^3$ die oben gegebenen Bedeutungen haben, $Z^1$ für Thiocarbonyl oder vorzugsweise Carbonyl steht, $Y^1$ Niederalkylen bedeutet, das durch Iminocarbonyl oder Oxycarbonyl unterbrochen sein kann, und $A^1$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder mit einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, $R^3$, $R^4$, $R^5$, $R^7$ und $R^8$ die oben gegebenen Bedeutungen haben, $R^6$ Wasserstoff oder Niederalkyl bedeutet, das unsubstituiert oder durch einen Rest der Formel Id, worin m, E und $X^4$ die oben gegebenen Bedeutungen haben, $Z^2$ für Thiocarbonyl oder insbesondere Carbonyl steht, $Y^2$ Niederalkylen bedeutet, das durch Iminocarbonyl oder Oxycarbonyl unterbrochen sein kann, und $A^2$ für einen Rest der Formel Ib oder Ic, worin $R^{15}$ einen mindestens 7 Kohlenstoffatome aufweisenden aliphatischen Rest bedeutet, $R^{16}$

Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, durch Hydroxy oder Mercapto, durch mit einem bis zu 90 Kohlenstoffatome enthaltenden aliphatischen Rest veräthertes Hydroxy oder Mercapto, durch mit einem bis zu 90 Kohlenstoffatome aufweisenden aliphatischen Acylrest verestertes Hydroxy oder Mercapto, der von der Gruppe der Formel Id verschieden ist, durch Amino, durch mit einem bis zu 90 Kohlenstoffatome enthaltenden Acylrest substituiertes Amino, der von einem Rest der Formel Id verschieden ist, durch freies Carboxy, Niederalkoxycarbonyl, Carbamoyl, Niederalkylaminocarbonyl, Carboxyniederalkylaminocarbonyl oder amidiertes Carboxyl der Formel Ida, unsubstituiertes oder durch Hydroxy, Niederalkoxy oder Halogen substituiertes Phenyl oder durch Imidazolyl oder Indolyl substituiert ist, oder $R^5$ und $R^6$ zusammen 1,3- oder 1,4-Niederalkylen bedeuten, $R^9$ und $R^{11}$ unabhängig voneinander Hydroxy, Niederalkoxy, Amino, Niederalkylamino, Carboxyniederalkylamino, oder einen Rest der Formel Ie bedeuten, worin $X^5$ und $X^6$ die oben gegebenen Bedeutungen haben, $Y^3$ Niederalkylen bedeutet, das durch Iminocarbonyl oder Oxycarbonyl unterbrochen sein kann, und $A^3$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, und $R^{10}$ für Wasserstoff,

Carboxy, Niederalkoxycarbonyl, Carbamoyl, Niederalkylaminocarbonyl oder Carboxyniederalkylaminocarbonyl steht, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ vom Acylrest einer aliphatischen, cycloaliphatischen, aromatischen oder araliphatischen Carbonsäure, insbesondere einer unsubstituierten oder, z.B. durch Hydroxy, Amino und/oder Alkanoylamino, wie Höheralkanoylamino, substituierten Alkancarbonsäure, mit bis zu 90 Kohlenstoffatomen, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies Hydroxy oder Mercapto, durch mit einem bis zu 90 Kohlenstoffatome enthaltenden aliphatischen Rest veräthertes Hydroxy oder Mercapto, durch von der Gruppe der Formel Id verschiedenes, verestertes Hydroxy oder Mercapto, durch freies oder mit einem bis zu 90 Kohlenstoffatome enthaltenden Acylrest substituiertes und von der Gruppe der Formel Id verschiedenes Amino, durch freies Carboxy, Niederalkylaminocarbonyl, unsubstituiertes oder durch Hydroxy, Niederalkoxy oder Halogen substituiertes Phenyl, oder durch Imidazolyl oder Indolyl substituiert ist, oder $R^7$ von Wasserstoff verschieden ist, und Salze, insbesondere pharmazeutisch verwendbare Salze von solchen Verbindungen.

Die Erfindung betrifft in erster Linie die unter (a), (b), (c) und (d) genannten Hexapyranose-, insbesondere D-Glucopyranose-Verbindungen der Formel I, worin $X^1$, $X^2$, $R^1$, $R^2$, $R^3$, $R^4$, $R^5$, $R^6$, $R^7$, $R^8$, $R^9$, $R^{10}$, $R^{11}$, $R^{12}$ und $R^{13}$ die im vorangegangenen Paragraphen gegebenen Bedeutungen haben, sowie Salze, insbesondere die pharmazeutisch verwendbaren Salze von solchen Verbindungen.

Die Erfindung betrifft in erster Linie D-Glucopyranose-Verbindungen der Formel If,

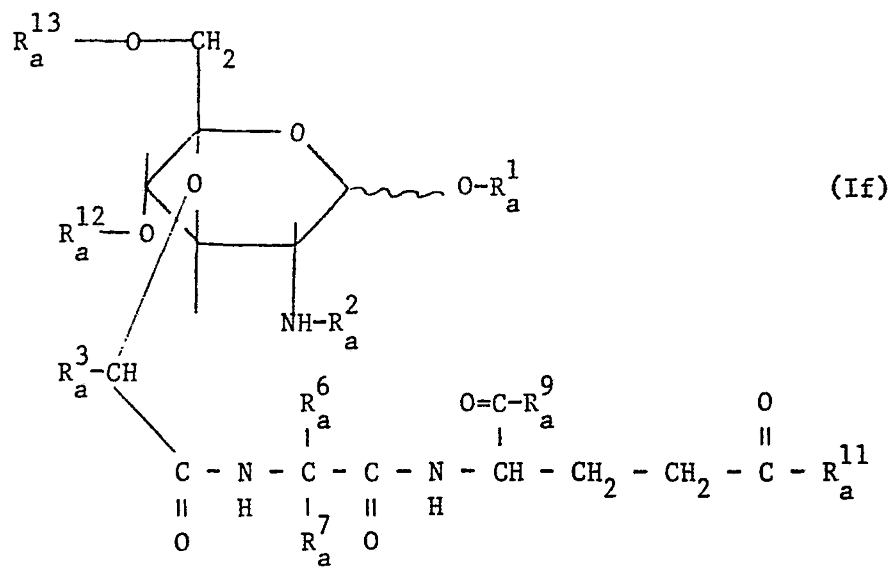

(If)

worin $R_a^1$ Wasserstoff, Niederalkanoyl oder die Gruppe der Formel Ia bedeutet, worin n für 1 steht, $Z^1$ Carbonyl bedeutet, $Y^1$ Niederalkylen darstellt, das durch Iminocarbonyl unterbrochen sein kann, $X^3$ für eine Gruppe der Formel -O- oder -NH- steht, und $A^1$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden Alkylrest, der unsubstituiert oder durch Hydroxy, Amino und/oder Alkanoylamino mit bis zu 30 Kohlenstoffatomen substituiert ist, veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R_a^2$ Niederalkanoyl, Hydroxyniederalkanoyl, Benzoyl oder die Gruppe der

Formel Ia bedeutet, worin n, $Z^1$, $Y^1$, $X^3$ und $A^1$ die oben gegebenen Bedeutungen haben, $R_a^{12}$ Wasserstoff oder Niederalkanoyl darstellt, $R_a^{13}$ Wasserstoff, Alkanoyl oder Hydroxyalkanoyl mit bis zu 90 Kohlenstoffatomen, Alkanoylaminoalkanoyl mit bis zu 30 Kohlenstoffatomen oder die Gruppe der Formel Ia bedeutet, worin n, $Z^1$, $Y^1$, $X^3$ und $A^1$ die oben gegebenen Bedeutungen haben, $R_a^3$ und $R_a^7$ für Wasserstoff oder Methyl stehen, $R_a^6$ Wasserstoff, unsubstituiertes oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl, Indolyl oder durch eine Gruppe der Formel Id substituiertes Niederalkyl bedeutet, worin m für 1 steht, E für eine Gruppe der Formel -O- oder -S- steht, $Z^2$ Carbonyl bedeutet, $Y^2$ Niederalkylen darstellt, das durch Iminocarbonyl unterbrochen sein kann, $X^4$ für eine Gruppe der Formel -O- steht, und $A^2$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem, 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, und jeder der Reste $R_a^9$ und $R_a^{11}$ unabhängig voneinander für Hydroxy, Niederalkoxy, Amino, Niederalkylamino, Carboxyniederalkylamino oder einen Rest der

Formel Ie stehen, worin $X^5$ für eine Gruppe der Formel -O- oder -NH- steht, $Y^3$ Niederalkylen darstellt, das durch Iminocarbonyl unterbrochen sein kann, $X^6$ für eine Gruppe der Formel -O- steht und $A^3$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R_a^9$ vorzugsweise für eine der Aminogruppen und $R_a^{11}$ vorzugsweise für Hydroxy stehen, mit der Massgabe, dass die Verbindungen mindestens einen, und vorzugsweise nur einen, Rest $A^1$, $A^2$ bzw. $A^3$ aufweisen, und mit der weiteren Massgabe, dass in Verbindungen der Formel I, worin mindestens einer der Reste $R^9$ oder $R^{11}$ für die Gruppe der Formel Ie steht, $R_a^1$ von Wasserstoff, oder $R_a^2$ von Niederalkanoyl, Hydroxyniederalkanoyl oder Benzoyl, oder $R_a^{12}$ von Wasserstoff, oder $R_a^{13}$ von Wasserstoff, oder $R_a^6$ von Wasserstoff, unsubstituiertem oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl oder Indolyl substituiertem Niederalkyl, oder $R_a^7$ von Wasserstoff verschieden ist, und Salze, insbesondere pharmazeutisch verwendbare Salze davon.

Die Erfindung betrifft in erster Linie die unter (a), (b), (c) und (d) genannten D-Glucopyranose-Verbindungen der Formel I, worin $X^1$ für die Gruppe der Formel -NH-, $X^2$ für die Gruppe der Formel -O-, $R^4$, $R^5$, $R^8$ und $R^{10}$ für Wasserstoff stehen, und $R^1$, $R^2$, $R^3$, $R^6$, $R^7$, $R^9$, $R^{11}$, $R^{12}$ und $R^{13}$ die im vorangegangenen Abschnitt für die Reste $R^1_a$, $R^2_a$, $R^3_a$, $R^6_a$, $R^7_a$, $R^9_a$, $R^{11}_a$, $R^{12}_a$ bzw. $R^{13}_a$ gegebenen Bedeutungen haben, sowie Salze, insbesondere pharmazeutisch verwendbare Salze,davon.

Insbesondere betrifft die Erfindung die neuen, in den Beispielen beschriebenen Verbindungen der Formel I und ihre Salze, insbesondere ihre pharmazeutisch verwendbaren Salze.

Wenn nicht anders angegeben, haben die im nachfolgenden gebrauchten Substituentenbezeichnungen die obengenannten Bedeutungen.

Die neuen Verbindungen der Formel I und ihre Salze können nach an sich bekannten Methoden erhalten werden.

So lassen sie sich erhalten, wenn man eine Verbindung der Formel II,

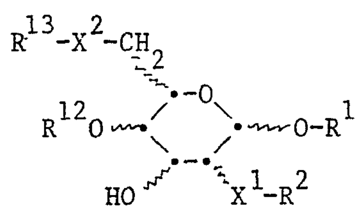

(II)

worin gegebenenfalls vorhandene funktionelle Gruppen, mit Ausnahme der freien Hydroxygruppe in 4-Stellung des Pyranoseteils, in geschützer Form vorliegen können, oder eine Metallverbindung davon mit einer Verbindung der Formel III,

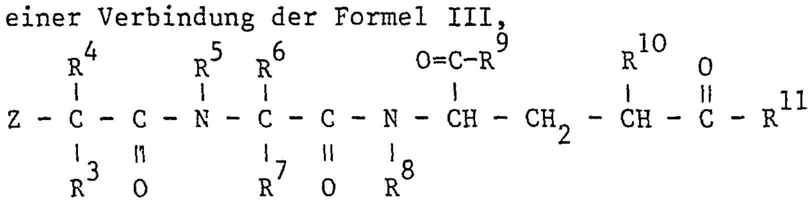

(III)

worin Z eine reaktionsfähige veresterte Hydroxygruppe darstellt und worin gegebenenfalls vorhandene funktionelle Gruppen durch Schutzgruppen geschützt sein können, umsetzt, gegebenenfalls vorhandene Schutzgruppen abspaltet und, wenn erwünscht, eine erhaltene Verbindung

der Formel I in eine andere Verbindung der Formel I umwandelt, und/oder wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder, wenn erwünscht, ein erhaltenes Isomerengemisch in die einzelnen Isomeren auftrennt.

Eine reaktionsfähige veresterte Hydroxygruppe ist insbesondere eine mit einer starken anorganischen oder organischen Säure veresterte Hydroxygruppe, in erster Linie eine mit einer Halogenwasserstoffsäure, wie Chlor-, Brom- oder insbesondere Jodwasserstoffsäure, oder mit einer aliphatischen oder aromatischen Sulfonsäure, wie Methansulfonsäure oder p-Toluolsulfonsäure, veresterte Hydroxygruppe.

Eine Metallverbindung ist insbesondere ein entsprechendes Alkalimetall-, z.B. Natrium- oder Kaliumderivat. Sie kann z.B. durch Behandeln einer Verbindung der Formel II mit einer geeigneten Metallbase, wie einer entsprechenden Alkalimetallverbindung, wie Natriumhydrid, Natriumamid oder Butyllithium, hergestellt werden.

Schutzgruppen von gegebenenfalls vorhandenen funktionellen Gruppen sind z.B. die obengenannten Schutzgruppen.

Die verwendeten Ausgangsstoffe sind bekannt oder lassen sich in an sich bekannter Weise, z.B. analog zu den hier beschriebenen Verfahren, herstellen.

Die neuen Verbindungen können auch erhalten werden, wenn man eine Verbindung der Formel IV,

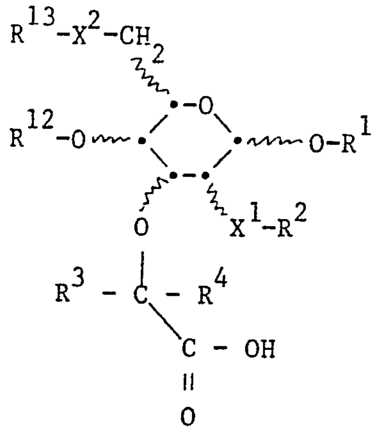

(IV)

worin gegebenenfalls vorhandene funktionelle Gruppen mit Ausnahme der freien Carboxylgruppe, in geschützter Form vorliegen können, oder ein reaktionsfähiges Carboxylderivat davon mit einer Verbindung der Formel V,

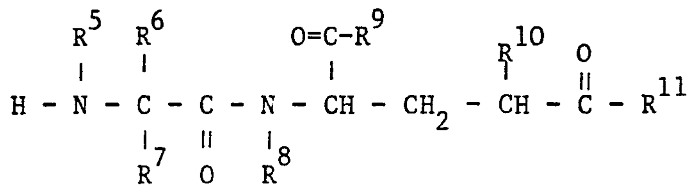

(V)

worin gegebenenfalls vorhandene freie funktionelle Gruppen, mit Ausnahme der Aminogruppe der Formel $HN(R^5)-$, in geschützter Form vorliegen können, oder einem reaktionsfähigen Derivat davon umsetzt und gegebenenfalls vorhandene Schutzgruppen abspaltet, und, wenn erwünscht, die obengenannten zusätzlichen Verfahrensschritte durchführt.

Reaktionsfähige Carboxylderivate sind in erster Linie reaktionsfähige aktivierte Ester oder reaktionsfähige Anhydride, ferner reaktionsfähige cyclische Amide von entsprechenden Säuren der Formel IV; dabei können reaktionsfähige Derivate von Säuren der Formel IV auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch

Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Aethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexyl-carbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenyl-thioester (die man z.B. durch Behandeln der entsprechenden Säure mit gegebenenfalls, z.B. durch Nitro, substituierten Thiophenolen, u.a. mit Hilfe der Anhydrid- oder Carbodiimid-Methode, erhalten kann; Methode der aktivierten Thiolester), oder Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-benztriazol, z.B. nach der Anhydrid- oder Carbodiimid-Methode, erhalten kann; Methode der aktivierten N-Hydroxyester).

Anhydride von Säuren der Formel IV können symmetrische und vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäure-niederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-äthoxy-1,2-dihydrochinolin, erhalten kann;Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diäthylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol; Imidazolid-Methode), oder Pyrazolen,

z.B. 3,5-Dimethyl-pyrazol (die man z.B. über das Säurehydrazid durch Behandeln mit Acetylaceton erhalten kann; Pyrazolid-Methode).

Die an der Reaktion teilnehmende Aminogruppe in einem Ausgangsmaterial der Formel V liegt bevorzugterweise in freier Form vor, insbesondere wenn die Carboxygruppe im Reaktionspartner der Formel IV in reaktionsfähiger Form vorliegt, sie kann aber auch selbst derivatisiert sein, z.B. durch Reaktion mit einem Phosphit, wie Diäthylchlorphosphit, 1,2-Phenylen-chlorphosphit, Aethyl-dichlor-phosphit, Aethylen-chlor-phosphit oder Tetraäthylpyrophosphit, aktiviert sein, oder z.B. als organische Silylamino-, wie Triniederalkylsilyl-, insbesondere Trimethylsilylaminogruppe vorliegen.

Dabei wird die gegebenenfalls derivatisierte Aminogruppe in einem Ausgangsmaterial der Formel V vorzugsweise durch Behandeln mit einem Anhydrid, insbesondere einem gemischten Anhydrid, aber auch mit einem aktivierten Ester oder einem cyclischen Amid einer Säure der Formel IV acyliert. Wie erwähnt können Derivate von Säuren der Formel IV auch in situ gebildet werden. So kann man z.B. N,N'-disubstituierte Amidinoester in situ bilden, indem man das Gemisch des Ausgangsmaterials der Formel V und der Säure der Formel IV in Gegenwart eines geeigneten N,N'-disubstituierten Carbodiimids, z.B. N,N'-Dicyclohexylcarbodiimid, zur Reaktion bringt. Ferner kann man Amino- oder Amidoester von Säuren der Formel IV in Gegenwart des zu acylierenden Ausgangsmaterials der Formel V bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Schutzgruppen von gegebenenfalls vorhandenen funktionellen Gruppen sind z.B. die obengenannten Schutzgruppen.

Die Acylierungsreaktion kann in an sich bekannter Weise durchgeführt werden, wobei die Reaktionsbedingungen in erster Linie von der Art des verwendeten Acylierungsmittels abhängen, üblicherweise in Gegenwart eines geeigneten Lösungs- oder Verdünnungsmittels oder eines

Gemisches von solchen, und, falls notwendig, in Gegenwart eines Kondensationsmittels, das z.B. bei Verwendung eines Anhydrids als Acylierungsmittel gegebenenfalls auch ein Säure-bindendes Mittel sein kann, unter Kühlen oder Erwärmen, z.B. in einem Temperaturbereich von etwa -30°C bis etwa +150°C, in einem geschlossenen Reaktionsgefäss und/oder in der Atmosphäre eines Inertgases, z.B. Stickstoff.

Die verwendeten Ausgangsstoffe sind bekannt oder lassen sich in an sich bekannter Weise herstellen. So kann man z.B. eine Verbindung der Formel II mit einer 2-Halogen-2-$R^3$-2-$R^4$-essigsäure, worin die Carboxylgruppe in geschützter Form vorliegen kann, umsetzen und, wenn notwendig, die Carboxylschutzgruppe abspalten.

Eine weitere Verfahrensweise zur Herstellung der neuen Verbindungen besteht darin, dass man eine Verbindung der Formel VI,

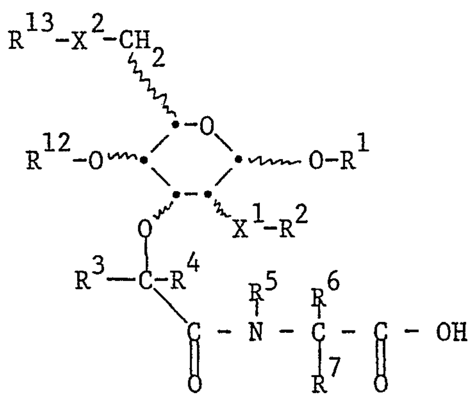

(VI)

worin gegebenenfalls vorhandene freie funktionelle Gruppen, mit Ausnahme der freien Carboxylgruppe, in geschützter Form vorliegen können, oder ein reaktionsfähiges Carboxylderivat davon mit einer Verbindung der Formel VII,

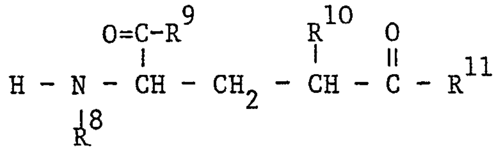

(VII)

worin gegebenenfalls vorhandene freie funktionelle Gruppen, mit Ausnahme der Aminogruppe der Formel $HN(R^8)$-, in geschützter Form vorliegen, oder einem reaktionsfähigen Derivat davon umsetzt, und gegebenenfalls vorhandene Schutzgruppen abspaltet, und, wenn erwünscht, die

obengenannten zusätzlichen Verfahrensschritte durchführt.

Die Kondensation kann analog dem vorangegangenen Verfahren durchführt werden, wobei reaktionsfähige Carboxylderivate von Säuren der Formel VI z.B. Säureanhydride, Säureamide und aktivierte Ester; reaktionsfähige Derivate der Aminoverbindung VII z.B. durch Umsetzen mit einem Phosphit erhältliche Derivate; und Schutzgruppen von gegebenenfalls vorhandenen freien funktionellen Gruppen vorzugsweise die obengenannten Derivate bzw. Schutzgruppen sind.

Die Ausgangsstoffe lassen sich in an sich bekannter Weise herstellen, z.B. indem man eine Verbindung der Formel II, worin gegebenenfalls vorhandene freie funktionelle Gruppen, mit Ausnahme der Hydroxygruppe, in geschützter Form vorliegen können, mit einer Verbindung der Formel VIII

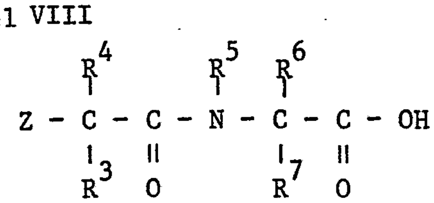

(VIII)

umsetzt, worin Z die oben gegebenen Bedeutung hat und vorhandene freie funktionelle Gruppen in geschützer Form vorliegen können, oder eine Verbindung der Formel IV, worin gegebenenfalls vorhandene freie funktionelle Gruppen, mit Ausnahme der Carboxygruppe, in geschützter Form vorliegen können, oder ein reaktionsfähiges Carboxylderivat davon mit einer Verbindung der Formel IX,

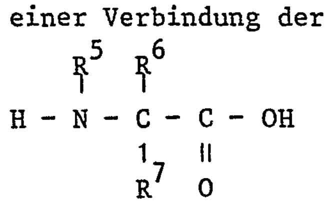

(IX)

worin freie funktionelle Gruppen, mit Ausnahme der Aminogruppe der Formel $HN(R^5)-$, in geschützer Form vorliegen können, oder einem reaktionsfähigen Derivat davon umsetzt. Die Reaktionen können z.B. in der oben beschriebenen Weise durchgeführt werden.

Verbindungen der Formel I, worin mindestens einer der Reste $R^1$, $R^2$, $R^{12}$ und $R^{13}$ eine Gruppe der Formel Ia bedeutet, worin n für 1 steht und $Z^1$, $Y^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, können z.B. hergestellt werden, wenn man eine Verbindung der Formel I, worin mindestens eine der Gruppen $R^1$, $R^2$, $R^{12}$ und $R^{13}$ für Wasserstoff steht und die anderen unabhängig voneinander für Wasserstoff, vom Rest der Formel Ia verschiedenes Acyl, eine unter physiologischen Bedingungen abspaltbare Gruppe oder einen Rest der Formel Ia stehen, und worin die übrigen Substituenten $R^3$, $R^4$, $R^5$, $R^6$, $R^7$, $R^8$, $R^9$, $R^{10}$, $R^{11}$, $X^1$ und $X^2$, die oben gegebenen Bedeutungen haben, wobei gegebenenfalls vorhandene freie funktionelle Gruppen, mit Ausnahme der an der Reaktion teilnehmenden Gruppe(n), in geschützter Form vorliegen können, oder ein reaktionsfähiges Derivat davon, mit einer Säure der Formel $HO - Z^1 - Y^1 - X^3 - A^1$ (X) , worin $Z^1$, $Y^1$, $X^3$ und $A^1$ die oben gegebenen Bedeutungen haben, wobei gegebenenfalls vorhandene freie funktionelle Gruppen, mit Ausnahme der Säuregruppe der Formel $HO-Z^1-$, in geschützter Form vorliegen können, oder einem reaktionsfähigen Säurederivat davon umsetzt und in einer erhaltenen Verbindung der Formel I gegebenenfalls vorhandene Schutzgruppen abspaltet, und, wenn erwünscht, zusätzliche Verfahrensschritte durchführt.

In den Ausgangsstoffen mit mindestens einer freien, an der Reaktion teilnehmenden Hydroxygruppe, z.B. in 1-Stellung des Zuckerteils, kann diese auch als reaktionsfähige veresterte Hydroxygruppe, insbesondere in Form einer mit einer anorganischen Säure veresterten Hydroxygruppe, z.B. als Halogen, wie Chlor, Brom oder Jod, oder in Form einer mit einer starken organischen Säure, wie einer entsprechenden Sulfonsäure veresterten Hydroxygruppe, z.B. als Niederalkylsulfonyloxy, insbesondere Methylsulfonyloxy, oder Arylsulfonyloxy, insbesondere p-Methylphenylsulfonyloxy, vorliegen oder durch Abspaltung des Protons mit einer starken Base aktiviert sein, d.h. als Metalloxy-, z.B. Alkalimetalloxy-, Gruppe vorliegen.

Eine an der Reaktion teilnehmende Aminogruppe in einem Ausgangsmaterial kann z.B. durch Umsetzen mit einem Phosphit, z.B. nach der Phosphit- oder Phosphorazo-Methode, in eine reaktionsfähige phosphorsubstituierte Aminogruppe oder durch Umsetzen mit z.B. Phosgen in eine reaktionsfähige Isocyanat-Gruppe überführt werden.

Schutzgruppen von gegebenenfalls vorhandenen funktionellen Gruppen sind z.B. die obengenannten Schutzgruppen.

Die Acylierung der freien Hydroxy- und/oder Aminogruppe in einer bei der vorliegenden Verfahrensvariante verwendeten Ausgangsverbindung der Formel I kann z.B. nach dem vorstehend beschriebenen Acylierungsverfahren durchgeführt werden, d.h. vorzugsweise mit einem geeigneten, gegebenenfalls in situ gebildeten Derivat einer Säure der Formel X, wobei als Derivat einer solchen auch ein Salz, wie ein Metall-, z.B. Alkalimetall-, wie Natrium- oder Kaliumsalz, oder ein Ammoniumsalz, eingesetzt werden kann.

Je nachdem, ob eine gegebenenfalls derivatisierte Hydroxygruppe oder Aminogruppe ineinem Ausgangsmaterial acyliert wird, eignet sich das eine oder das andere Derivat einer Säure der Formel X besser als Acylierungsmittel. Während die gegebenenfalls derivatisierte Aminogruppe in einem Ausgangsmaterial, vorzugsweise durch Behandeln mit einem Anhydrid, insbesondere einem gemischten Anhydrid, aber auch mit einem aktivierten Ester oder einem cyclischen Amid einer Säure der Formel X acyliert werden kann, verwendet man zur Acylierung der gegebenenfalls derivatisierten Hydroxygruppe in einem Ausgansmaterial insbesondere aktivierte Ester, in erster Linie Amino- oder Amidoester, ferner auch Anhydride, insbesondere gemischte Anhydride von Säuren derFormel X. Ausgangsstoffe mit einer in reaktionsfähiger veresterter Form vorliegenden Hydroxygruppe werden üblicherweise mit einem Salz einer Säure der Formel X umgesetzt.

Die Ausgangsstoffe sind bekannt oder können in an sich bekannter Weise hergestellt werden.

Verbindungen der Formel I, worin mindestens einer der Reste $R^1$, $R^2$, $R^{12}$ und $R^{13}$ eine Gruppe der Formel Ia bedeutet, worin n für 1 steht, $Y^1$ für unsubstituiertes oder substituiertes Alkylen steht, das durch Iminocarbonyl oder Oxycarbonyl unterbrochen ist, und $Z^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder Salze davon können z.B. hergestellt werden, indem man eine Verbindung der Formel I, worin mindestens eine der Gruppen $R^1$, $R^2$, $R^{12}$ und $R^{13}$ für einen Rest der Formel $-Z^1-Y^1_a-R_x$ (Iaa) steht, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel $R_y-Y^1_b-X^3-A^1$ (XI) umsetzt, wobei $Z^1$, $X^3$ und $A^1$ die oben gegebenen Bedeutungen haben, und $Y^1_a$ und $Y^1_b$ unabhängig voneinander unsubstituiertes oder substituiertes Alkylen bedeuten, und worin eine der Gruppen $R_x$ und $R_y$ für freies oder in reaktionsfähiger, derivatisierter Form vorliegendes Hydroxy oder Amino der Formel $-NH(R^{14})$ steht, und die andere für freies oder in reaktionsfähiger, derivatisierter Form vorliegendes Carboxy steht, während die übrigen Substituenten $R^1$, $R^2$, $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion teilnehmenden funktionellen Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Eine Hydroxygruppe $R_x$ bzw. $R_y$ liegt vorzugsweise in freier Form vor, kann aber auch in derivatisierter Form, z.B. wie oben beschrieben in Form einer reaktionsfähigen

veresterten Hydroxygruppe, ferner in Form einer Metalloxy-, wie Alkalimetalloxy-, z.B. Natriumoxy- oder Kaliumoxygruppe,vorliegen. Eine derivatisierte Aminogruppe $R_x$ bzw. $R_y$ kann z.B. eine, wie oben beschrieben, phosphorsubstituierte Aminogruppe darstellen.

Eine Carboxylgruppe $R_x$ bzw. $R_y$ liegt vorzugsweise in reaktionsfähiger derivatisierter Form vor, insbesondere in einer der oben beschriebenen reaktionsfähigen, anhydridisierten, aktivierten veresterten oder cyclisch amidierten Form, ferner in Salzform, z.B. in Alkalimetallsalzform, vor.

Schutzgruppen von gegebenenfalls in den Ausgangsstoffen vorhandenen funktionellen Gruppen sind z.B. die obengenannten Schutzgruppen.

Die Reaktion wird z.B. wie vorstehend beschrieben durchgeführt.

Die Ausgangsstoffe können nach an sich bekannten Verfahren, z.B. analog zu den hier beschriebenen Verfahren, hergestellt werden.

Verbindungen der Formel I, worin mindestens einer der Reste $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder Salze davon können z.B. hergestellt werden, indem man eine Verbindung der Formel I, worin mindestens eine der Gruppen $R^1$, $R^2$, $R^{12}$ und $R^{13}$ für einen Rest der Formel $-Z^1-Y^1-X^3-H$ (Iab) steht, worin $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten,

oder ein reaktionsfähiges Derivat davon mit einer Verbindung der Formel XII oder XIII,

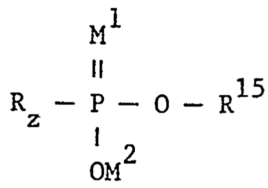

(XII)

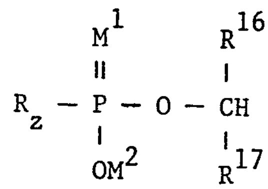

(XIII)

worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig, in Gegenwart eines oxidierenden Mittels, umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht und $M^2$ Wasserstoff bedeutet, können die Verbindungen der Formeln XII und XIII auch in der Form von Tautomeren der Formel XIIa bzw. XIIIa

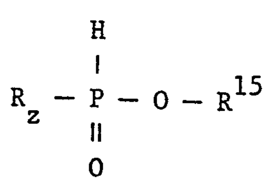

(XIIa)

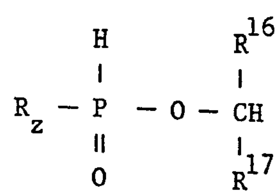

(XIIIa)

vorliegen.

Die Gruppe $R_z$ in den Ausgangsstoffen der Formeln XII und XIII kann ausser einer Hydroxygruppe, die auch in Salzform, z.B. als Metalloxy-, wie Alkalimetalloxy-, z.B. Natriumoxy- oder Kaliumoxygruppe vorliegen kann, für veräthertes Hydroxy, wie Niederalkoxy, z.B. Methoxy oder Aethoxy, oder Phenyloxy, oder verestertes Hydroxy, wie mit einer starken Säure verestertes Hydroxy, z.B. Halogen, wie Chlor, stehen.

Eine abspaltbare Gruppe $M^2$ bildet mit der Oxygruppe eine verätherte Hydroxygruppe, insbesondere Niederalkoxy, z.B. Methoxy oder Aethoxy, ferner Phenyloxy.

In einem Ausgangsmaterial mit einer Gruppe der Formel Iab kann die Hydroxy- oder Aminogruppe der Formel $-X^3-H$ in reaktionsfähiger, derivatisierter Form vorliegen. Falls die Gruppe der Formel $-X^3-H$ für Hydroxy steht, kann dieses z.B. in Form einer reaktionsfähigen veresterten Hydroxygruppe, wie einer der obengenannten Gruppen dieser Art, oder auch in Form einer Metalloxy-, wie Alkalimetalloxy-, z.B. Natriumoxy- oder Kaliumoxygruppe, vorliegen. Eine reaktionsfähige derivatisierte Aminogruppe ist z.B. eine wie oben geschildert phosphorsubstituierte Aminogruppe.

Schutzgruppen von gegebenenfalls in den Ausgangsstoffen vorhandenen funktionellen Gruppen sind die obengenannten Schutzgruppen.

Die Reaktion wird in an sich bekannter Weise, vorzugsweise in Gegenwart eines säurebindenden Mittels, wie Pyridin, Triniederalkylamin, z.B. Trimethylamin oder Triäthylamin, eines Imidazols oder einer anorganischen Base, wie Natrium- oder Kaliumhydroxyd, oder in Gegenwart eines Alkalimetall-, wie Natrium- oder Kaliumniederalkanolates, durchgeführt, wobei man als Lösungsmittel ein aprotisches Lösungsmittel, wie Dimethylsulfoxyd oder Acetonitril, bevorzugt.

Falls in einem Ausgangsmaterial der Formel XII bzw. XIII die Gruppe $=M^5$ ein Elektronenpaar darstellt, wird die Reaktion in Gegenwart eines

geeigneten, insbesondere schwachen Oxidationsmittels, z.B. einer Persäure, wie Perbenzoesäure, oder einem Alkylhydroperoxyd, durchgeführt.

Die Abspaltung einer abspaltbaren Gruppe $M^2$ wird üblicherweise gemeinsam mit der Abspaltung von anderen Schutzgruppen, vorzugsweise hydrogenolytisch oder durch saure Hydrolyse, vorgenommen.

Die Ausgangsstoffe sind bekannt und lassen sich auf an sich bekannte Weise, z.B. analog zu einem der hierin beschriebenen Verfahren herstellen.

Verbindungen der Formel I, worin mindestens einer der Rest $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder Salze davon können z.B. hergestellt werden, indem man eine Verbindung der Formel I, worin mindestens eine der Gruppen $R^1$, $R^2$, $R^{12}$ und $R^{13}$ für einen Rest der Formel

$$-Z^1-Y^1-X^3-\underset{\underset{OM^2}{|}}{\overset{\overset{M^1}{\|}}{P}}-R_z \qquad \text{(Iac)}$$

steht, worin $R_z$ gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel XIV oder XV,

$HO - R^{15}$ 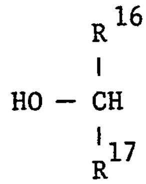

(XIV) (XV)

worin $R^{15}$, $R^{16}$ und $R^{17}$ die oben gegebenen Bedeutungen haben, oder einem Derivat davon, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht, und $M^2$ Wasserstoff bedeutet, kann die Phosphorgruppe in einem Rest der Formel Iac auch in tautomerer Form als Gruppe der Formel

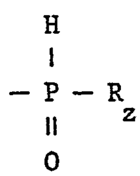

vorliegen.

Die Gruppe $R_z$ kann ausser Hydroxy auch in Salzform vorliegendes Hydroxy oder reaktionsfähiges veräthertes oder verestertes Hydroxy, wie oben beschrieben, darstellen.

Eine abspaltbare Gruppe $M^2$ steht für einen, eine organische Hydroxygruppe veräthernden Rest und ist insbesondere Niederalkyl, wie Methyl oder Aethyl, ferner Phenyl.

In einem Derivat einer Verbindung der Formel XIV bzw. XV liegt die Hydroxygruppe in reaktionsfähiger derivatisierter Form, z.B. als reaktionsfähige veresterte Hydroxygruppe oder als Metalloxy-, z.B. Alkalimetalloxygruppe vor.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren, hergestellt werden.

Verbindungen der Formel I, worin mindestens einer der Reste $R^1$, $R^2$, $R^{12}$ und $R^{13}$ eine Gruppe der Formel Ia bedeutet, worin n für 0 steht, und $A^1$ die obengenannte Bedeutung hat, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder Salze davon, können z.B. hergestellt werden, indem man eine Verbindung der Formel I, worin mindestens eine der Gruppen $R^1$, $R^2$, $R^{12}$ und $R^{13}$ für Wasserstoff steht, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder ein Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, $M^2$ Wasserstoff oder eine

abspaltbare Gruppe bedeutet und $R^{15}$, $R^{16}$ und $R^{17}$ die oben gegebenen Bedeutungen haben, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

In einem Ausgangsmaterial mit mindestens einer Hydroxygruppe der Formel $-O-R^1$, $-X^1-R^2$, $-O-R^{12}$ bzw. $-X^2-R^{13}$ oder mit einer Aminogruppe der Formel $-X^1-R^2$ bzw. $-X^2-R^{13}$ kann eine solche Gruppe in reaktionsfähiger derivatisierter Form vorliegen, eine Hydroxygruppe z.B. als reaktionsfähige veresterte Hydroxygruppe oder als Metalloxy-, z.B. Alkalimetalloxygruppe, und eine Aminogruppe, z.B. in phosphorsubstituierter Form (wie sie z.B. durch Behandeln mit einem Phosphit gebildet werden kann).

Falls in einem Ausgangsmaterial der Formel XII bzw. XIII $=M^1$ für ein Elektronenpaar und $M^2$ für Wasserstoff stehen, kann die Verbindung in der tautomeren Form der Formel XIIa bzw. XIIIa vorliegen.

Die Gruppe $R_z$ kann ausser als Hydroxy auch in Salzform vorliegendes Hydroxy oder als reaktionsfähiges veräthertes oder verestertes Hydroxy, wie oben beschrieben, vorliegen.

Eine abspaltbare Gruppe $M^2$ steht für einen, eine Hydroxygruppe veräthernden organischen Rest und ist insbesondere Niederalkyl, wie Methyl oder Aethyl, ferner Phenyl.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$, für ein Elektronenpaar steht, in Gegenwart eines oxidierenden Mittels, z.B. einem der obengenannten, arbeitet.

Die Ausgangsstoffe können in an sich bekannter Weise, z.B. analog zu den hierin beschriebenen Verfahren, hergestellt werden.

Verbindungen der Formel I, worin $R^6$ Niederalkyl bedeutet, das durch den Rest der Formel Id, worin m für 1 steht, und die Gruppen E, $Z^2$, $Y^2$, $X^4$ und $A^2$ die oben gegebenen Bedeutungen haben, substituiert ist, oder Salze davon, können z.B. hergestellt werden, indem man eine Verbindung der Formel I, worin $R^6$ für Niederalkyl steht, das durch die Gruppe der Formel -E-H (Idb), worin E die oben gegebene Bedeutung hat, substituiert ist, oder ein reaktionsfähiges Derivat davon mit einer Säure der Formel $HO-Z^2-Y^2-X^4-A^2$ (XVI), worin $Z^2$, $Y^2$, $X^4$ und $A^2$ die oben gegebenen Bedeutungen haben, oder einem reaktionsfähigen Derivat davon umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

In einem Derivat eines Ausgangsmaterials mit der Gruppe der Formel Idb liegt eine entsprechende Hydroxygruppe in derivatisierter Form, z.B. wie oben beschrieben in Form einer reaktionsfähigen Hydroxygruppe, oder in Salzform, z.B. in Form einer Metalloxy-, wie Alkalimetalloxy-, z.B. Natriumoxy- oder Kaliumoxygruppe vor, während eine entsprechende Mercaptogruppe in analoger Weise derivatisiert sein kann. Eine derivatisierte Aminogruppe der

Formel Idb ist z.B. eine wie oben beschrieben phosphorsubstituierte Aminogruppe.

Eine reaktionsfähige derivatisierte Säuregruppe der Formel $HO-Z^2-$ (Idc) ist in erster Linie eine entsprechende aktivierte Ester- oder Anhydridgruppe, kann aber auch eine entsprechende cyclische Säureamidgruppe sein, wobei diese reaktionsfähigen, derivatisierten Gruppen auch <u>in situ</u> gebildet werden können. Beispiele von solchen reaktionsfähigen Gruppen sind z.B. die oben beschriebenen Reste.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt.

Die Ausgangsstoffe sind bekannt oder können in an sich bekannter Weise hergestellt werden.

Verbindungen der Formel I, worin $R^6$ für Niederalkyl steht, das durch den Rest der Formel Id, wobei m für 1 steht, $Y^2$ unsubstituiertes oder substituiertes Alkylen bedeutet, das durch Iminocarbonyl oder Oxycarbonyl unterbrochen ist, und die Gruppen E, $Z^2$, $X^4$ und $A^2$ die oben gegebenen Bedeutungen haben, substituiert ist, oder Salze davon können hergestellt werden, indem man eine Verbindung der Formel I, worin $R^6$ für Niederalkyl steht, das durch die Gruppe $-E-Z^2-Y_a^2-R_x$ (Idc) substituiert ist, mit einer Verbindung der Formel $R_y-Y_b^2-X^4-A^2$ (XVII) umsetzt, worin $Y_a^2$ und $Y_b^2$ unabhängig voneinander unsubstituiertes oder substituiertes Alkylen bedeuten, und worin eine der Gruppen $R_x$ und $R_y$ für freies oder in reaktionsfähiger derivatisierter Form vorliegendes Hydroxy oder Amino der Formel $-NH(R^{14})$ steht, und die andere für freies oder in reaktionsfähiger, derivatisierter Form vorliegendes Carboxy

steht, und E, $Z^2$, $X^4$ und $A^2$ die obigen Bedeutungen haben, während die übrigen Substituenten der Ausgangsstoffe $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Eine Hydroxygruppe $R_x$ bzw. $R_y$ kann in derivatisierter Form, z.B. wie oben beschrieben in Form einer reaktionsfähigen veresterten Hydroxygruppe, ferner in Form einer Metalloxy-, wie Alkalimetalloxy-, z.B. Natriumoxy- oder Kaliumoxygruppe vorliegen. Eine derivatisierte Aminogruppe $R_x$ bzw. $R_y$ kann z.B. eine wie oben beschrieben phosphorsubstituierte Aminogruppe darstellen.

Eine Carboxylgruppe $R_x$ bzw. $R_y$ liegt vorzugsweise in reaktionsfähiger derivatisierter Form vor, insbesondere in einer der oben beschriebenen reaktionsfähigen, anhydridisierten, aktivierten veresterten oder cyclisch amidierten Form, ferner in Salzform, z.B. in Alkalimetallsalzform, vor.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt.

Die Ausgangsstoffe können in an sich bekannter Weise, z.B. analog den beschriebenen Verfahren, hergestellt werden.

Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die oben gegebenen Bedeutungen haben, oder Salze davon, können z.B. hergestellt werden, indem man eine Verbindung der Formel I, worin $R^6$ Niederalkyl bedeutet, das durch eine Gruppe der Formel $- E - Z^2 - Y^2 - X^4 - H$ (Idd) substituiert ist, worin E, $Z^2$, $Y^2$ und $X^4$ die oben gegebenen Bedeutungen haben, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmende Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht und $M^2$ Wasserstoff bedeutet, können die Verbindungen der Formeln XII und XIII auch in der Form von Tautomeren der Formel XIIa bzw. XIIIa vorliegen.

Die Gruppe $R_z$ kann ausser Hydroxy auch in Salzform vorliegendes Hydroxy oder reaktionsfähiges veräthertes oder verestertes Hydroxy, wie oben beschrieben, darstellen.

Eine abspaltbare Gruppe $M^2$ steht für einen eine organische Hydroxygruppe veräthernden Rest und ist insbesondere Niederalkyl, wie Methyl oder Aethyl, ferner Phenyl.

In einem Ausgangsmaterial mit einer Gruppe der Formel Idd kann die Hydroxy- oder Aminogruppe der Formel $-X^4-H$ in reaktionsfähiger derivatisierter Form vorliegen. Falls die Gruppe der Formel $-X^4-H$ für Hydroxy steht, kann dieses z.B. in Form einer reaktionsfähigen veresterten Hydroxygruppe, wie einer der obengenannten Gruppen dieser Art, oder auch in Form einer Metalloxy-, wie Alkalimetalloxy-, z.B. Natriumoxy- oder Kaliumoxygruppe, vorliegen, während eine entsprechende derivatisierte Aminogruppe z.B. eine phosphorsubstituierte Aminogruppe, wie eine der obengenannten, sein kann.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren, hergestellt werden.

Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die obengegebenen Bedeutungen haben, oder Salze davon können z.B. hergestellt werden, indem man eine Verbindung der Formel I, worin $R^6$ Niederalkyl bedeutet, das durch eine Gruppe der Formel

$$-E-Z^2-Y^2-X^4-\underset{\displaystyle |\atop OM^2}{\overset{\displaystyle M^1\atop \|}{P}}-R_z \qquad \text{(Ide)}$$

substituiert ist, worin $R_z$ für gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt,

und E, $Z^2$, $Y^2$ und $X^4$ die obengegebenen Bedeutungen haben, mit einer Verbindung der Formel XIV oder XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht und $M^2$ Wasserstoff bedeutet, kann die Phosphorgruppe in einem Rest der Formel Ide auch in tautomerer Form als Gruppe der Formel

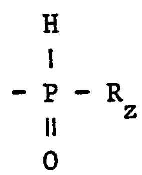

vorliegen.

Die Gruppe $R_z$ kann ausser Hydroxy auch in Salzform vorliegendes Hydroxy oder reaktionsfähiges veräthertes oder verestertes Hydroxy, wie oben beschrieben, darstellen.

Eine abspaltbare Gruppe $M^2$ steht für einen, eine organische Hydroxygruppe veräthernden Rest und ist insbesondere Niederalkyl, wie Methyl oder Aethyl, ferner Phenyl.

In einem Derivat einer Verbindung der Formel XIV bzw. XV liegt die Hydroxygruppe in reaktionsfähiger, derivatisierter Form, z.B. als reaktionsfähige veresterte Hydroxygruppe oder als Metalloxy-, z.B. Alkalimetalloxygruppe, vor.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren, hergestellt werden.

Verbindungen der Formel I, worin $R^6$ Niederalkyl bedeutet, das durch einen Rest der Formel Id substituiert ist, worin m für 0 steht, und $A^2$ die obengenannte Bedeutung hat, oder Salze davon können z.B. hergestellt werden, indem man eine Verbindung der Formel I, worin $R^6$ Niederalkyl bedeutet, das durch eine Gruppe der Formel Idb, worin E die oben gegebene Bedeutung hat, substituiert ist, oder ein reaktionsfähiges Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels,umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

In einem Ausgangsmaterial mit einer Hydroxy- oder Aminogruppe der Formel

Idb kann eine solche Gruppe in reaktionsfähiger,derivatisierter Form vorliegen, eine Hydroxygruppe z.B. als reaktionsfähige veresterte Hydroxygruppe oder als Metalloxy-, z.B. Alkalimetalloxygruppe, und eine Aminogruppe z.B. in phosphorsubstituierter Form (wie sie z.B. durch Behandeln mit einem Phosphit gebildet werden kann).

Falls in einem Ausgangsmaterial der Formel XII bzw. XIII $=M^1$ für ein Elektronenpaar und $M^2$ für Wasserstoff stehen, kann die Verbindung in der tautomeren Form der Formel XIIa bzw. XIIIa vorliegen.

Die Gruppe $R_z$ kann ausser als Hydroxy auch in Salzform vorliegendes Hydroxy oder als reaktionsfähiges veräthertes oder verestertes Hydroxy, wie oben beschrieben, vorliegen.

Eine abspaltbare Gruppe $M^2$ steht für einen, eine Hydroxygruppe veräthernden organischen Rest und ist insbesondere Niederalkyl, wie Methyl oder Aethyl, ferner Phenyl.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren,hergestellt werden.

Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder

Mercapto, freies oder von einem Rest der Formel Ie verschiedenes, substituiertes Amino oder die Gruppe der Formel Ie bedeutet, oder Salze davon können z.B. hergestellt werden, indem man eine Verbindung der Formel I, worin eine der Gruppen $R^9$ und $R^{11}$ Hydroxy oder Mercapto darstellt, und die andere für freies Hydroxy oder Mercapto, von einem Rest der Formel Ie verschiedenes veräthertes Hydroxy oder Mercapto, freies oder von einem Rest der Formel Ie verschiedenes substituiertes Amino oder einen Rest der Formel Ie steht, oder ein reaktionsfähiges Säurederivat davon mit einer Verbindung der Formel XVIII,

$$H - X^5 - Y^3 - X^6 - A^3 \qquad \text{(XVIII)}$$

worin $X^5$, $Y^3$, $X^6$ und $A^3$ die oben gegebenen Bedeutungen haben, oder einem reaktionsfähigen Derivat davon, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet und, wenn erwünscht, zusätzliche Verfahrensschritte durchführt.

Die Säuregruppe in einem Ausgangsmaterial liegt vorzugsweise in reaktionsfähiger derivatisierter Form, insbesondere in einer der oben beschriebenen reaktionsfähigen, anhydrisierten, aktivierten veresterten oder cyclisch amidierten Form, ferner in Salzform, z.B. in Alkalimetallsalzform, vor.

In einem reaktionsfähigen Derivat einer Verbindung der Formel XVIII liegt eine durch die Gruppe der Formel $H-X^5-$ dargestellte Hydroxygruppe in derivatisierter Form, z.B. wie oben beschrieben in Form einer reaktionsfähigen veresterten Hydroxygruppe, oder in Salzform, z.B. in Form einer Metalloxy-, wie Alkalimetalloxy-, z.B. Natriumoxy- oder Kaliumoxygruppe vor, während eine entsprechende Mercaptogruppe in analoger Weise derivatisiert ist. Eine entsprechende Aminogruppe ist z.B. eine wie oben beschrieben phosphorsubstituierte Aminogruppe.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt.

Die Ausgangsstoffe sind bekannt oder können in an sich bekannter Weise hergestellt werden.

Verbindungen der Formel I, worin eine der Gruppen $R^9$ und $R^{11}$ für den Rest der Formel Ie steht, worin $Y^3$ unsubstituiertes oder substituiertes Alkylen darstellt, das durch Iminocarbonyl oder Oxycarbonyl unterbrochen ist, und worin $X^5$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino, oder die Gruppe der Formel Ie bedeutet, oder Salze davon können z.B. hergestellt werden, indem man eine Verbindung der Formel I, worin eine der Gruppen $R^9$ und $R^{11}$ für den Rest der Formel $-X^5-Y^3_a-R_x$ (Iea) steht, mit einer Verbindung der Formel $R_y-Y^3_b-X^6-A^3$ (XIX), worin eine der Gruppen $R_x$ und $R_y$ für ein freies oder in reaktionsfähiger derivatisierter Form vorliegendes Hydroxy oder Amino der Formel $-NH(R^{14})$ steht, und die andere für freies oder in reaktionsfähiger derivatisierter Form vorliegendes Carboxy steht, $Y^3_a$ und $Y^3_b$ unabhängig voneinander unsubstituiertes oder substituiertes Alkylen bedeuten, und $X^5$, $X^6$ und $A^3$ die oben gegebenen Bedeutungen haben, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, umsetzt und in einer erhaltenen Verbindung der Formel I vorhandene Schutzgruppen abspaltet, und, wenn erwünscht, zusätzliche Verfahrensschritte durchführt.

Eine Hydroxygruppe $R_x$ bzw. $R_y$ kann in derivatisierter Form, z.B. wie oben beschrieben in Form einer reaktionsfähigen veresterten Hydroxygruppe, ferner in Form einer Metalloxy-, wie Alkalimetalloxy-, z.B. Natriumoxy- oder Kaliumoxygruppe,vorliegen. Eine derivatisierte Aminogruppe $R_x$ bzw. $R_y$ kann z.B. eine wie oben beschrieben phosphorsubstituierte Aminogruppe darstellen.

Eine Carboxylgruppe $R_x$ bzw. $R_y$ liegt vorzugsweise in reaktionsfähiger derivatisierter Form vor, insbesondere in einer der oben beschriebenen reaktionsfähigen, anhydridisierten, aktivierten veresterten oder cyclisch amidierten Form, ferner in Salzform, z.B. in Alkalimetallsalzform, vor.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt.

Die Ausgangsstoffe sind bekannt oder können in an sich bekannter Weise hergestellt werden.

Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino oder die Gruppe der Formel Ie bedeutet, oder Salze davon können z.B. hergestellt werden, indem man eine Verbindung der Formel I, worin eine der Gruppen $R^9$ und $R^{11}$ für den Rest der Formel $-X^5-Y^3-X^6-H$ (Ieb) steht, worin $X^5$, $Y^3$ und $X^6$ die oben gegebenen Bedeutungen haben, und die andere freies Hydroxy oder Mercapto, von einem Rest der Formel Ie verschiedenes

veräthertes Hydroxy oder Mercapto, freies oder von einem Rest der Formel Ie verschiedenes, substituiertes Amino oder die Gruppe der Formel Ie oder Ieb bedeutet, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht und $M^2$ Wasserstoff bedeutet, können die Verbindungen der Formel XII und XIII auch in der Form vom Tautomeren der Formel XIIa bzw. XIIIa vorliegen.

Die Gruppe $R_z$ kann ausser Hydroxy auch in Salzform vorliegendes Hydroxy oder reaktionsfähiges veräthertes oder verestertes Hydroxy, wie oben beschrieben, darstellen.

Eine abspaltbare Gruppe $M^2$ steht für einen eine organische Hydroxygruppe veräthernden Rest und ist insbesondere Niederalkyl, wie Methyl oder Aethyl, ferner Phenyl.

In einem Ausgangsmaterial mit einer Gruppe der Formel Ieb kann die Hydroxy- oder Aminogruppe der Formel $-X^6-H$ in reaktionsfähiger, derivatisierter Form vorliegen. Falls die Gruppe der Formel $-X^6-H$ für Hydroxy steht, kann dieses z.B. in Form einer reaktionsfähigen veresterten Hydroxygruppe, wie einer der obengenannten Gruppen dieser Art, oder auch in Form einer Metalloxy-, wie Alkalimetalloxy-, z.B. Natriumoxy- oder Kaliumoxygruppe, vorliegen. Eine entsprechende Aminogruppe kann z.B. in phosphorsubstituierter Form vorliegen.

Schutzgruppen von gegebenenfalls in den Ausgangsstoffen vorhandenen funktionellen Gruppen sind z.B. die obengenannten Schutzgruppen.

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren, hergestellt werden.

Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes subsitutiertes Amino oder die Gruppe der Formel Ie bedeutet, oder Salze davon können z.B. hergestellt werden, indem man eine Verbindung der Formel I, worin eine der Gruppen $R^9$ und $R^{11}$ den Rest der Formel

$$-X^5-Y^3-X^6-\underset{\underset{OM^2}{|}}{\overset{\overset{M^1}{\|}}{P}}-R_z \qquad \text{(Iec)}$$

bedeutet, worin $R_z$ für gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und $X^5$, $Y^3$ und $X^6$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes, substituiertes Amino oder die Gruppe der Formel Ie oder Iec bedeutet, mit einer Verbindung der Formel XIV oder

XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, und, wenn erwünscht, zusätzliche Verfahrensschritte durchführt.

Falls $=M^1$ für ein Elektronenpaar steht, und $M^2$ Wasserstoff bedeutet, kann die Phosphorgruppe in einem Rest der Formel Iec auch in tautomerer Form als Gruppe der Formel

$$-\overset{\displaystyle H}{\underset{\displaystyle O}{\overset{|}{\underset{\|}{P}}}}-R_z$$

vorliegen.

Die Gruppe $R_z$ kann ausser Hydroxy auch in Salzform vorliegendes Hydroxy oder reaktionsfähiges veräthertes oder verestertes Hydroxy, wie oben beschrieben, darstellen.

Eine abspaltbare Gruppe $M^2$ steht für einen eine organische Hydroxygruppe veräthernden Rest und ist insbesondere Niederalkyl, wie Methyl oder Aethyl, ferner Phenyl.

In einem Derivat einer Verbindung der Formel XIV bzw. XV liegt die Hydroxygruppe in reaktionsfähiger derivatisierter Form, z.B. als reaktionsfähige veresterte Hydroxygruppe oder als Metalloxy-, z.B. Alkalimetalloxygruppe vor.

Schutzgruppen von gegebenenfalls in den Ausgangsstoffen vorhandenen funktionellen Gruppen sind z.B. die obengenannten Schutzgruppen

Die Reaktion wird in an sich bekannter Weise, z.B. wie oben beschrieben, durchgeführt, wobei man, falls in einem Ausgangsmaterial $=M^1$ für ein Elektronenpaar steht, in Gegenwart eines oxidierenden Mittels, z.B. einem der obengenannten, arbeitet.

Die Ausgangsstoffe können in an sich bekannter Weise, z.B. analog den hierin beschriebenen Verfahren, hergestellt werden.

Die erfindungsgemässen Verbindungen der Formel I, in welcher $R^1$ für Wasserstoff, $X^1$ für -NH-, $R^2$ für den Rest einer organischen Carbonsäure, der Rest der Formel $-X^2—R^{13}$ für Hydroxy und $R^{12}$ für Wasserstoff stehen, und in denen der Pyranosering die Konfiguration der Glucopyranose hat, oder Salze davon können ebenfalls hergestellt werden, indem man in einer Glucofuranose-Verbindung der Formel XX,

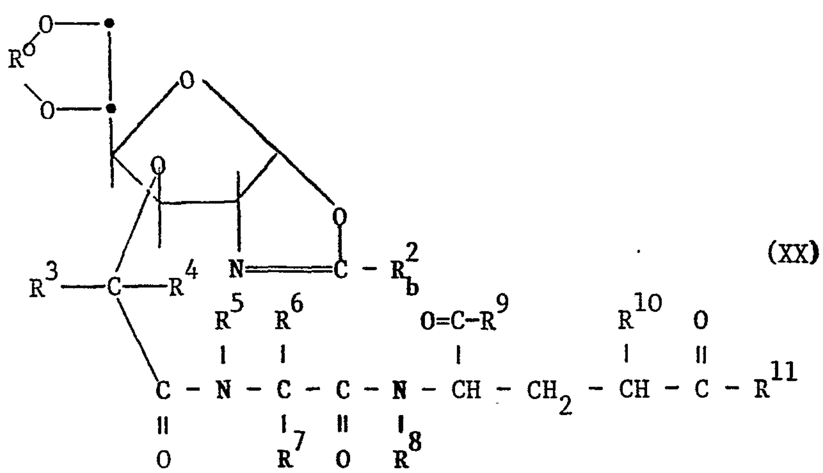

(XX)

worin $R^2_b$ für den Descarbonyl-Rest eines Acylrestes $R^2$ einer organischen Carbonsäure steht, und $R^o$ unsubstituiertes oder substituiertes Methylen bedeutet, und worin $R^3$, $R^4$, $R^5$, $R^6$, $R^7$, $R^8$, $R^9$, $R^{10}$ und $R^{11}$ die oben gegebenen Bedeutungen haben, und gegebenenfalls vorhandene freie funktionelle Gruppen in geschützter Form vorliegen können, den Oxazolin- und den Dioxolanring sauer aufspaltet, und in einer erhaltenen Verbindung der Formel I vorhandene Schutzgruppen abspaltet, und, wenn erwünscht, zusätzliche Verfahrensmassnahmen durchführt.

In einem Ausgangsmaterial der Formel XX ist $R_b^2$ in erster Linie ein aromatischer Rest, z.B. ein Phenylrest.

Ein Methylenrext $R^o$ ist vorzugsweise, z.B. durch Niederalkyl, wie Methyl, Niederalkylen, wie 1,5-Pentylen, oder unsubstituiertes oder, z.B. wie oben beschrieben, substituiertes Phenyl, substituiert und steht z.B. für Niederalkyliden, wie Isopropyliden, Cycloalkyliden, wie Cyclohexyliden, oder Benzyliden.

Schutzgruppen von gegebenenfalls vorhandenen funktionellen Gruppen sind z.B. die obengenannten Schutzgruppen.

Die Spaltung erfolgt in an sich bekannter Weise, z.B. durch Behandeln mit einem sauren Ionenaustauscher, insbesondere einem Sulfonsäuregruppen enthaltenden Ionenaustauscher, wie einem sauren Styrolharz mit Sulfogruppen (z.B. Amberlite IR-120), oder einem sauren Polystyrolsulfonsäureharz (z.B.Dowex 50), oder mit einer starken anorganischen oder organischen Säure, wie einer Mineralsäure, z.B. Salzsäure, Bromwasserstoffsäure oder Schwefelsäure, oder einer organischen Sulfonsäure, wie einer aliphatischen Sulfonsäure, z.B. Methansulfonsäure, oder einer im aromatischen Ring unsubstituierten oder substituierten Benzolsulfonsäure, wie p-Toluolsulfonsäure, oder einer starken organischen Carbonsäure, wie einer halogen-substituierten Niederalkancarbonsäure, z.B. Trifluoressigsäure. Dabei arbeitet man in Gegenwart von Wasser und erhält so Verbindungen der Formel I, worin $X^1$ und $X^2$ für die Gruppe der Formel -O- und $R^1$, $R^{12}$ und $R^{13}$ für Wasserstoff stehen.

Die Ausgangsstoffe können nach an sich bekannten Verfahren, u.a. auch analog zu den hierin beschriebenen Verfahren, hergestellt werden.

In einer erhaltenen Verbindung der Formel I werden, wenn erwünscht, ein oder mehrere der folgenden zusätzlichen Verfahrensschritte durchgeführt.

In einer erhaltenen Verbindung der Formel I, worin eine oder mehrere funktionelle Gruppen geschützt sind, werden diese, z.B. geschützte

Carboxyl-, Amino-, Hydroxy- und/oder Mercaptogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B.durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators,freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Jod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetra-niederalkylammoniumfluorid oder Triniederalkyl-aryl-ammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe, setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure,wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie

eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte

Hydroxy- bzw. Mercaptogruppe wird z.B. durch basische Hydrolyse, eine durch tert.-Niederalkyl oder durch einen 2-oxa- oder 2-thia-aliphatischen oder-cycloaliphatischen Kohlenwasserstoffrest verätherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

Erfindungsgemäss erhältliche Verbindungen der Formel I können, falls erwünscht, in andere Verbindungen der Formel I übergeführt werden. So kann man z.B. Säuregruppen, wie Carboxylgruppen, verestern, Hydroxy- und Mercaptogruppen z.B. veräthern oder verestern, oder Aminogruppen z.B. acylieren. Diese Zusatzreaktionen können in an sich bekannter Weise, z.B. analog den hierin beschriebenen Verfahren, durchgeführt werden.

Salze von Verbindungen der Formel I können in an sich bekannter Weise hergestellt werden. So kann man Salze von Verbindungen der Formel I z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallverbindungen, wie entsprechenden Hydroxiden, Carbonaten und

Hydrogencarbonaten, wie Natrium- oder Kaliumhydroxid, -carbonat oder -hydrogencarbonat, oder entsprechenden Calciumverbindungen oder mit Ammoniak oder geeigneten organischen Aminen bilden, wobei man vorzugsweise stöchiometrische Mengen oder einen kleinen Ueberschuss des salzbildenden Mittels verwendet. Säureadditionssalze von Verbindungen der Formel I, die salzbildende basische Gruppen enthalten, erhält man in üblicher Weise, z.B. durch Behandeln mit einer Säure oder einem geeigneten Anionenaustauschreagens. Innere Salze von Verbindungen der Formel I, welche saure und basische salzbildende Gruppen enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren, Racemate z.B. unter Bildung von Derivaten mit optisch aktiven Verbindungen und Trennung der so erhältlichen Diastereomerengemische in die optisch aktiven Antipoden, aufgetrennt werden.

Die oben beschriebenen Verfahren, inklusive die Verfahren zur Abspaltung von Schutzgruppen und die zusätzlichen Verfahrensmassnahmen, werden in an sich bekannter Weise, z.B. in An- oder Abwesenheit von Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die Erfindung betrifft ebenfalls neue Zwischenprodukte, insbesondere neue Verbindungen der Formel V, worin $R^5$, $R^6$, $R^7$, $R^8$, $R^9$,$R^{10}$ und $R^{11}$ die oben gegebenen Bedeutungen haben, oder Salze davon, ferner Verbindungen der Formel V, worin $R^5$, $R^6$, $R^7$, $R^8$, $R^9$, $R^{10}$ und $R^{11}$ die oben gegebenen Bedeutungen haben, oder pharmazeutisch verwendbare Salze davon zur pharmakologischen Verwendung, insbesondere als immunomodulierende, z.B. immunstimulierende, Mittel, sowie pharmazeutische Präparate enthaltend Verbindungen der Formel V oder pharmazeutisch verwendbare Salze davon. Es hat sich herausgestellt, dass auch Zwischenprodukte, insbesondere diejenigen der Formel V, immunomodulierende Wirkungen aufweisen und deshalb analog den Verbindungen der Formel I verwendet werden können.
Die Erfindung betrifft ebenfalls pharmazeutische Präparate, welche eine pharmakologisch wirksame Menge der Aktivsubstanz, gegebenenfalls zusammen mit pharmazeutisch verwendbaren Trägerstoffen enthalten, die sich zur enteralen, z.B. oralen, oder parenteralen Verabreichung

eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyäthylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 1% bis 100%, insbesondere von etwa 5% bis etwa 50%, Lyophilisate bis zu 100%, des bzw. der Aktivstoffe.

Die Dosierung kann von verschiedenen Faktoren, wie Applikationsweise, Spezies, Alter und/oder individuellem Zustand abhängen. So liegen die täglich zu verabreichenden Dosen bei oraler Applikation an Warmblütern von etwa 70 kg vorzugsweise zwischen etwa 0,001 und 0.1 g.

Die nachfolgenden Beispiele illustrieren die Erfindung, ohne sie in irgendeiner Form einzuschränken. Die $R_f$-Werte werden auf Kieselgeldünnschichtplatten der Firma Merck ermittelt. Das Verhältnis der Laufmittel in den verwendeten Laufmittelgemischen zueinander ist in Volumenanteilen (V/V), Temperaturen sind in Grad Celsius angegeben. Die Phosphorylverbindungen werden üblicherweise, auch wenn es nachstehend nicht in jedem einzelnen Falle angegeben ist, in Form von oder im Gemisch mit ihren Salzen, z.B. Natriumsalzen, wie Mono- oder Bisnatriumsalzen, erhalten.

Beispiel 1: Zu einer Lösung von 1 mMol 2-(1,2-Dipalmitoyl-sn-glycero-3-hydroxyphosphoryloxy)-äthylamin und von 2 mMol Triäthylamin in 20 ml eines Gemisches aus Chloroform : Methanol : Wasser = 65:25:4, werden 1,5 mMol N-Hydroxysuccinimidester von N-Acetyl-6-O-succinoyl-normuramyl-L-alanyl-D-isoglutamin-γ-diphenylmethylester, gelöst in 5 ml Dimethylacetamid, bei Raumtemperatur zugetropft. Nach 20 Stunden Rühren bei Raumtemperatur wird die Lösung bei reduziertem Druck auf ca. 15 ml eingeengt; dabei entsteht eine Emulsion. Diese wird mit 200 ml Wasser verdünnt und gefriergetrocknet. Der Rückstand wird in 25 ml Wasser suspendiert und 24 Stunden gegen 0,1molaren Natriumphosphatpuffer, pH 7, danach 2 Tage gegen Wasser dialysiert. Das Innendialysat, das das gewünschte Produkt (Gemisch mit 40-55 % des Natriumsalzes) enthält, wird gefriergetrocknet. Zur Abspaltung des Diphenylmethyl-Restes wird der gefriergetrocknete Rückstand in 50 ml eines Gemisches (1:1) aus 1,2-Dimethoxyäthan und Methanol gelöst und über $Pd/BaSO_4$ (10%ig) hydriert. Der Katalysator wird abfiltriert und das Lösungsmittel bei reduziertem Druck abdestilliert.

Das N-Acetyl-6-O-{[N-2-(1,2-dipalmitoyl-sn-glycero-3-hydroxyphosphoryloxy)-äthyl]-succinamoyl}-normuramyl-L-alanyl-D-isoglutamin, das teilweise in Natriumsalzform vorliegt, wird durch Chromatographie an einer Sephadex LH-20 Säule gereinigt, Elutionsgemisch Chloroform : Methanol : Essigsäure : Wasser = 25:15:4:2, $R_f$ = 0.36 (Chloroform : Methanol : Wasser = 65:25:4), $R_f$ = 0.66 (Chloroform : Methanol : Essigsäure : Wasser = 25:15:4:2).

Die neue Verbindung wird analytisch dadurch charakterisiert, dass die Bausteine - N-Acetylmuraminsäure, Palmitinsäure, Phosphat, L-Alanin und D-Glutaminsäure - quantitativ bestimmt werden: N-Acetylmuraminsäure wird mit Hilfe des Morgan-Elson-Reaktion nach der Modifikation von J.M. Ghuysen et al. [in "Methods in Enzymology" 8, 629 (1966)] spektrophotometrisch bestimmt.

Phosphat wird nach Lowry et al. [J.Biol.Chem. 207, 1 (1954)] quantitativ bestimmt.

Palmitinsäure und die Aminosäuren werden in einem Totalhydrolysat (6N HCl, 24 Std. 110°C) gaschromatographisch bzw. mit Hilfe eines Aminosäureanalysators unter Verwendung von Pentadecansäure bzw. Norleucin als interne Standards quantitativ bestimmt.

Die gefundenen molaren Verhältnisse bezogen auf Phosphat sind wie folgt:

$PO_4'''$ : N-Acetyl-muraminsäure : Alanin : Glutaminsäure : Palmitinsäure = 1:0,90:0,93:0,95:2,16.

Die Ausgangsstoffe sind wie folgt erhältlich:

2 mMol N-Acetyl-6-O-succinoyl-normuramyl-L-alanyl-D-isoglutamin-γ-diphenylmethylester, 2,1 mMol N-Hydroxysuccinimid und 2,1 mMol Dicyclohexylcarbodiimid werden in 6,6 ml Dimethylacetamid gelöst und 18 Stunden bei 20°C gerührt. Der ausgefallene Dicyclohexylharnstoff wird abgetrennt und die Lösung, die den N-Hydroxysuccinimidester von N-Acetyl-6-O-succinoyl-normuramyl-L-alanyl-D-isoglutamin-γ-diphenylmethylester enthält, wird direkt für die Kondensation mit dem Phospholipid verwendet.

2-(1,2-Dipalmitoyl-sn-glycero-3-hydroxyphosphoryloxy)-äthylamin ist ein kommerziell erhältliches synthetisches Präparat.

5,1 g (7,8 mMol) N-Acetyl-normuramyl-L-alanyl-D-isoglutamin-γ-diphenylmethylester werden in 40 ml abs. Pyridin gelöst und 2,34 g (23,4 mMol) Bernsteinsäureanhydrid, gelöst in 40 ml abs. Essigsäureäthylester, bei -5° innerhalb von 20 Minuten eingetropft. Nach zweitägigem Stehen bei 0° wird mit 4normaler Essigsäure auf pH = 4 angesäuert und nach einstündigem Rühren eingedampft. Der Rückstand wird in 100 ml Acetonitril:Wasser = 1:4 gelöst, auf 100 Kieselgel $UPC_{12}$ (ANTEC) gegeben und mit Acetonitril:Wasser = 1:1 erst die Bernsteinsäure, dann mit Acetonitril:Wasser = 3:1 die Hauptmenge des Produktes eluiert. Man erhält 3,1 g N-Acetyl-6-O-succinoyl-normuramyl-L-alanyl-D-isoglutamin-γ-diphenylmethylester als amorphes Pulver, $[\alpha]_D^{20}$ = +16° (c = 0,243; Methanol), $R_f$ = 0,43 (n-Butanol:Essigsäure:Wasser = 75:7,5:21), $R_f$ = 0,70 (Essigsäureäthylester:n-Butanol:Pyridin:Essigsäure:Wasser = 42:21:21:6:10).

Eine Lösung von 22,8 g (40 mMol) N-Acetyl-normuramyl-L-alanyl-D-isoglutamin in 500 ml eines 1:1-Gemisches von 1,2-Dimethoxy-äthan und Methanol wird mit 11,6 g (60 mMol) Diphenyldiazomethan versetzt, und die Lösung während 16 Stunden bei Raumtemperatur gerührt. Die rote Suspension wird bei 20° und unter vermindertem Druck eingedampft, und der Rückstand mehrfach mit Diäthyläther verrieben, bis ein fast farbloses Produkt erhalten wird. Dieses wird in 100 ml Methanol gelöst und durch portionenweise Zugabe eines 2:1-Gemisches von Diäthyläther/Petroläther zur Kristallisation gebracht. Nach mehrstündigem Rühren bei Raumtemperatur, dann im Eisbad wird die Kristallmasse abfiltriert und unter vermindertem Druck getrocknet. Man erhält so den N-Acetyl-normuramyl-L-alanyl-D-isoglutamin-diphenylmethylester in Form von würfelförmigen Kristallen, Smp. 170° (mit Zersetzung), $[\alpha]_D^{20}$ ± 14 ± 1° (c = 1,5; Methanol), $R_f$ = 0.40 (Chloroform:Methanol:Wasser = 70:30:5), $R_f$ = 0.66 (Essigsäureäthylester:n-Butanol:Pyridin:Eisessig:Wasser = 42:21:21:6:10).

Beispiel 2: Analog Beispiel 1 erhält man folgende Verbindungen, die in Natriumsalzform oder im Gemisch mit dieser vorliegen:

N-Acetyl-6-O-{N-[2-(1,2-dipalmitoyl-sn-glycero-3-hydroxyphosphoryloxy)-äthyl]-succinamoyl}- muramyl-L-α-aminobutyryl-D-isoglutamin,

N-Acetyl-6-O-{N-[2-(tetradecyloxy-hydroxyphosphoryloxy)-äthyl]-succinamoyl}-muramyl-L-α-aminobutyryl-D-isoglutamin,

N-Propionyl-6-O-{N-[2-(1,2-dipalmitoyl-sn-glycero-3-hydroxyphosphoryloxy)-äthyl]-succinamoyl}-normuramyl-L-alanyl-D-isoglutamin,

N-Propionyl-6-O-{N-[2-(tetradecyloxy-hydroxyphosphoryloxy)-äthyl]-succinamoyl}-normuramyl-L-alanyl-D-isoglutamin,

N-Acetyl-6-O-{N-[2-(1,2-dipalmitoyl-sn-glycero-3-hydroxyphosphoryloxy)-äthyl]-succinamoyl}-muramyl-L-valyl-D-isoglutamin,

N-Acetyl-6-O-{N-[2-(tetradecyloxy-hydroxyphosphoryloxy)-äthyl]-succinamoyl}-muramyl-L-valyl-D-isoglutamin,

N-Acetyl-6-O-{N-[2-(1,2-dipalmitoyl-sn-glycero-3-hydroxyphosphoryloxy)-äthyl]-succinamoyl}-normuramyl-L-alanyl-D-glutamyl-glycinamid,

N-Acetyl-6-O-{N-[2-(tetradecyloxy-hydroxyphosphoryloxy)-äthyl]-succinamoyl}-normuramyl-L-alanyl-D-glutamyl-glycinamid,

N-Benzoyl-6-O-{N-[1,2-dipalmitoyl-sn-glycero-3-hydroxyphosphoryloxy)-äthyl]-succinamoyl}-muramyl-L-alanyl-D-isoglutamin,

N-Benzoyl-6-O-{N-[2-(tetradecyloxy-hydroxyphosphoryloxy)-äthyl]-succinamoyl}-muramyl-L-alanyl-D-isoglutamin,

N-Benzoyl-6-O-{N-[2-(1,2-dipalmitoyl-sn-glycero-3-hydroxyphosphoryloxy)-äthyl]-succinamoyl}-normuramyl-L-α-aminobutyryl-D-isoglutamin,

N-Benzoyl-6-O-{N-[2-(tetradecyloxy-hydroxyphosphoryloxy)-äthyl]-succinamoyl}-normuramyl-L-α-aminobutyryl-D-isoglutamin,

N-Acetyl-6-O-{N-[2-(1,2-dipalmitoyl-sn-glycero-3-hydroxyphosphoryloxy)-äthyl]-succinamoyl}-muramyl-L-alanyl-D-(γ-carboxy)-isoglutamin,

N-Acetyl-6-O-{N-[2-(tetradecyloxy-hydroxyphosphoryloxy)-äthyl]-succinamoyl}-muramyl-L-alanyl-D-(γ-carboxy)-isoglutamin,

2-Acetylamino-2-desoxy-6-O-{N-[2-(1,2-dipalmitoyl-sn-glycero-3-hydroxyphosphoryloxy)-äthyl]-succinamoyl}-D-mannopyranosyl-(3-O)-D-propionyl-L-alanyl-D-isoglutamin,

N-Acetyl-6-O-{N-[2-(1,2-dipalmitoyl-sn-glycero-3-hydroxyphosphoryl-oxy)-äthyl]-succinamoyl}-muramyl-L-α-aminobutyryl-D-isoglutamin-γ-n-butylester,
2-Acetylamino-2-desoxy-6-O-{N-[2-(tetradecyloxy-hydroxyphosphoryloxy)-äthyl]-succinamoyl}-D-mannopyranosyl-(3-O)-D-propionyl-L-alanyl-D-isoglutamin,
2-Acetylamino-2-desoxy-6-O-{N-[2-(1,2-dipalmitoyl-sn-glycero-3-hydroxy-phosphoryloxy)-äthyl]-succinamoyl}—D-galactopyranosyl-(3-O)-D-propionyl-L-alanyl-D-isoglutamin,
2-Acetylamino-2-desoxy-6-O-{N-[2-(tetradecyloxy-hydroxyphosphoryloxy)-äthyl]-succinamoyl}-D-galactopyranosyl-(3-O)-D-propionyl-L-alanyl-D-isoglutamin,
N-Acetyl-6-O-{N-[2-(1,2-dipalmitoyl-sn-glycero-3-hydroxyphosphoryloxy)-äthyl]-succinamoyl}-muramyl-L-prolyl-D-isoglutamin,
N-Acetyl-6-O-{N-[2-(tetradecyloxy-hydroxyphosphoryloxy)-äthyl]-succinamoyl}-muramyl-L-prolyl-D-isoglutamin,
N-Acetyl-6-O-{N-[2-(1,2-dipalmitoyl-sn-glycero-3-hydroxyphosphoryloxy)-äthyl]-succinamoyl}-normuramyl-L-α-aminobutyryl-D-isoglutamin,
N-Acetyl-6-O-{N-[2-(tetradecyloxy-hydroxyphosphoryloxy)-äthyl]-succinamoyl}-normuramyl-L-α-aminobutyryl-D-isoglutamin,
N-Acetyl-6-O-{N-[2-(1,2-dipalmitoyl-sn-glycero-3-hydroxyphosphoryloxy)-äthyl]-succinamoyl}-muramyl-D-α-aminobutyryl-D-isoglutamin,
N-Acetyl-6-O-{N-[2-(tetradecyloxy-hydroxyphosphoryloxy)-äthyl]-succinamoyl}-muramyl-D-α-aminobutyryl-D-isoglutamin,
N-Acetyl-6-O-{N-[2-(1,2-dipalmitoyl-sn-glycero-3-hydroxyphosphoryl-oxy)-äthyl]-succinamoyl}-muramyl-α-amino-isobutyryl-D-isoglutamin,
N-Acetyl-6-O-{N-[2-(tetradecyloxy-hydroxyphosphoryloxy)-äthyl]-succinamoyl}-muramyl-α-amino-isobutyryl-D-isoglutamin.

Beispiel 3: In analoger Weise zu Beispiel 1, ausgehend von 2-(Tetra-decyloxy-hydroxyphosphoryloxy)-äthylamin und N-Hydroxysuccinimidester von N-Acetyl-normuramyl-L-O-(succinoyl)-seryl-D-isoglutamin-γ-di-phenylmethylester erhält man N-Acetyl-normuramyl-L-O-{N-[2-(tetra-decyloxy-hydroxyphosphoryloxy)-äthyl]-succinamoyl}-seryl-D-isoglutamin, das teilweise in Natriumsalzform vorliegt.

Beispiel 4: Zu einer Lösung von 1 mMol 2-[{(3'R)-Hydroxy-(2'S)-palmitoylamino-octadecyloxy}-hydroxyphosphoryloxy]-äthylamin und von 2 mMol Triäthylamin in 200 ml eines Gemisches aus Chloroform:Methanol: Wasser = 65:25:4, wird eine Lösung von 1,5 mMol N-Acetyl-6-O-(N-stearoyl-glycyl)-normuramyl-L-alanyl-D-isoglutamin-N-hydroxysuccinimidester in 5 ml 1,2-Dimethoxy-äthan bei Raumtemperatur zugetropft. Nach 20 Stunden Rühren bei Raumtemperatur wird die Lösung bei reduziertem Druck auf ca. 15 ml eingeengt; dabei entsteht eine Emulsion. Diese wird mit 200 ml Wasser verdünnt und gefriergetrocknet. Der Rückstand wird in 50 ml Wasser suspendiert und 24 Stunden gegen 0,1 M Natriumphosphatpuffer pH = 7, danach 3 Tage gegen Wasser dialysiert. Das Innendialysat, das N-Acetyl-6-O-(N-stearoyl-glycyl)-normuramyl-L-alanyl-D-isoglutamin-2-[{(3'R)-hydroxy-(2'S)-palmitoylamino-octadecyloxy}-hydroxyphosphoryloxy]-äthylamid in Form von oder im Gemisch mit seinem Natriumsalz enthält, wird gefriergetrocknet und durch Chromatographie an einer Sephadex LH-20 Säule gereinigt. Elutionsgemisch: Chloroform: Methanol:Wasser = 65:25:4.

Die neue Verbindung wird analytisch dadurch charakterisiert, dass die Bausteine (Stearinsäure, Palmitinsäure, Glycin, Alanin, Glutaminsäure und Phosphat) in analoger Weise zu Beispiel 1 bestimmt werden.

Die gefundenen molaren Verhältnisse bezogen auf Phosphat sind wie folgt: $PO_4'''$:Glycin:Alanin:Glutaminsäure:Palmitinsäure:Stearinsäure = 1:0.89:0.92:0.95:1.09:0.98.

2-[{(3'R)-Hydroxy-(2'S)-palmitoylamino-octadecyloxy}-hydroxyphosphoryloxy]-äthylamin, das als Ausgangsstoff verwendet wird, ist ein kommerziell erhältliches synthetisches Präparat.

N-Acetyl-6-O-(N-stearoyl-glycyl)-normuramyl-L-alanyl-D-isoglutamin-N-hydroxysuccinimidester, der als Ausgangsstoff verwendet wird, lässt sich z.B. wie folgt herstellen:

2 mMol N-Acetyl-6-O-(N-stearoyl-glycyl)-normuramyl-L-alanyl-D-isoglutamin, 2,1 mMol N-Hydroxysuccinimid und 2,1 mMol Dicyclohexylcarbodiimid werden in 6,6 ml 1,2-Dimethoxy-äthan gelöst und 18 Stunden bei 20°C gerührt. Der ausgefallene Dicyclohexylharnstoff wird abgetrennt und die Lösung direkt für die Kondensation mit dem Phospholipid verwendet.

Der Ausgangsstoff wird wie folgt hergestellt:

0,60 g (0,7 mMol) N-Acetyl-6-O-(N-stearoyl-glycyl)-normuramyl-L-alanyl-D-isoglutamin-γ-tert.butylester werden bei 0° in 7 ml 95%iger Trifluoressigsäure gelöst und während 20 Minuten bei derselben Temperatur stehengelassen. Die klare Lösung wird eingedampft und der Rückstand über Natronasbest (Merck) im Hochvakuum getrocknet. Der Rückstand wird in 15 ml eines Gemisches aus tert.Butanol:Wasser = 9:1 gelöst, über 40 ml gereinigten starksauren Ionenaustauscher (H-Form) filtriert und mit 80 ml des obigen Gemisches nachgewaschen. Das Filtrat wird auf ca. 20 ml eingeengt, durch ein Milliporefilter (0,45 μ) filtriert und lyophilisiert. Man erhält 0,265 g N-Acetyl-6-O-(N-stearoyl-glycyl)-normuramyl-L-alanyl-D-isoglutamin als farbloses Pulver, $[\alpha]_D^{20} = +5\pm1°$ (c = 0,40; Methanol), $R_f$ = 0,26 (Chloroform:Methanol:Wasser = 70:30:5), $R_f$ = 0,40 (n-Butanol:Essigsäure:Wasser = 75:75:21).

Das Ausgangsmaterial erhält man wie folgt:

0,90 g (1,01 mMol) N-Acetyl-1α-O-benzyl-6-O-(N-stearoyl-glycyl)-normuramyl-L-alanyl-D-isoglutamin-γ-tert.butylester, gelöst in 40 ml 1,2-Dimethoxyäthan:Wasser = 20:1 werden in Gegenwart von 1,8 g Pd auf Kohle (100%ig) während 39 Stunden mit Wasserstoff behandelt. Der Katalysator wird abfiltriert, das Filtrat eingedampft und der Rückstand nach Aufnahme in einem Gemisch von 80 ml Chloroform:Essigsäureäthylester = 1:5 4mal mit je 20 ml Wasser extrahiert. Nach Trocknen

über Natriumsulfat und Eindampfen verbleiben 0,65 g N-Acetyl-6-O-(N-stearoyl-glycyl)-normuramyl-L-alanyl-D-isoglutamin-γ-tert.butylester als farbloser Schaum. $R_f$ = 0,62 (Chloroform:Methanol:Wasser = 70:30:5), $R_f$ = 0,52 (n-Butanol:Essigsäure:Wasser = 75:7,5:21).

Das Ausgangsmaterial erhält man wie folgt:

Eine Suspension von 0,70 g (1 mMol) N-Acetyl-1α-O-benzyl-6-O-glycyl-normuramyl-L-alanyl-D-isoglutamin-γ-tert.butylester-hydrochlorid, 0,446 g (1,15 mMol) Stearinsäure-p-nitrophenylester und 0,111 g (1 mMol) N-Methyl-morpholin in 2 ml Dimethylformamid wird während 16 Stunden bei Raumtemperatur gerührt, dann unter vermindertem Druck zur Trockene eingedampft. Der Rückstand wird in Chloroform gelöst und in der Kälte mehrfach mit einer 1N Zitronensäurelösung, dann mit Wasser gewaschen. Die getrocknete organische Phase wird eingedampft und an Silikagel im System Chloroform:Isopropanol = 7:3 gereinigt. Man erhält den N-Acetyl-1α-O-benzyl-6-O-(N-stearoyl-glycyl)-normuramyl-L-alanyl-D-isoglutamin-γ-tert.butylester als amorphes, stark hygroskopisches Pulver, $R_f$ = 0,86 (Chloroform:Methanol:Wasser = 70:30:5), $R_f$ = 0,11 (Chloroform:Isopropanol:Essigsäure = 70:8:2).

Das verwendete Ausgangsmaterial erhält man wie folgt:

Eine Lösung von 16,8 g (20 mMol) N-Acetyl-1α-O-benzyl-6-O-(N-benzyloxycarbonyl-glycyl)-normuramyl-L-alanyl-D-isoglutamin-tert.butylester in 200 ml eines 20:1-Gemisches von 1,2-Dimethoxy-äthan und Wasser wird nach Zugabe von 4,8 g eines Palladium-auf-Kohle-Katalysators (10%ig) mit Wasserstoff behandelt. Der pH-Wert der Lösung wird während der Hydrierung durch Zugabe von 2normaler wässriger Salzsäure mittels pH-Stat auf 3 gehalten. Die Suspension wird filtriert, das Filtrat bei Raumtemperatur und unter vermindertem Druck eingedampft, und der weisse Rückstand durch Chromatographie an 400 g Silikagel (Merck AG, 0,06-0,2 mm) im System Chloroform:Isopropanol = 7:3 gereinigt. Man wäscht mit 2 Fraktionen zu je 1000 ml des Lösungsmittelgemisches vor und eluiert dann mit 190 Fraktionen zu je 10 ml, gefolgt von 4 Fraktionen zu je 800 ml; das reine Produkt wird in diesen 4 letzten Fraktionen erhalten. Der Rückstand aus diesen wird in einem 10:1-Gemisch von 1,2-Dimethoxy-äthan und Wasser gelöst; der pH-Wert der Lösung wird unter Kühlen durch Zugabe von 2normaler Salzsäure auf 4,5 gestellt, und die Lösung unter vermindertem Druck und in der Kälte eingedampft. Der Rückstand wird über Natronasbest (Merck AG) getrocknet und ergibt N-Acetyl-1α-O-benzyl-6-O-glycyl-normuramyl-L-alanyl-D-isoglutamin-tert.-butylester-hydrochlorid als weissen Schaum, $R_f$ = 0,47 (Chloroform:Methanol:Wasser = 70:30:5), $R_f$ = 0,10 (Chloroform:Isopropanol = 7:3).

Das Ausgangsmaterial wird wie folgt hergestellt:

Eine Lösung von 13,74 g (22 mMol) N-Acetyl-1α-O-benzyl-normuramyl-L-alanyl-D-isoglutamin-tert.-butylester, 4,2 g (20 mMol) N-Benzyloxycarbonyl-glycin, 5,4 g (40 mMol) 1-Hydroxy-benztriazol und 2,44 g (20 mMol) 4-Dimethylamino-pyridin in 100 ml Dimethylformamid wird auf 0° abgekühlt und 5,0 g (24 mMol) Dicyclohexylcarbodiimid hinzugefügt. Nach 24stündigem Rühren bei Raumtemperatur wird die Suspension filtriert und das Filtrat unter vermindertem Druck eingedampft. Der Rückstand wird in 800 ml Essigsäureäthylester aufgenommen und in der Kälte dreimal mit je 100 ml Wasser und dreimal mit je 100 ml 2normaler wässriger Zitronensäurelösung gewaschen. Die organische Phase wird mit Wasser neutralgewaschen und über Natriumsulfat getrocknet. Beim Einengen auf ein Volumen von etwa 100 ml beginnt die Kristallisation. Nach mehrstündigem Rühren unter Kühlen im Eisbad wird die Kristallmasse abfiltriert, gewaschen und aus Essigsäureäthylester umkristallisiert. Man erhält so den N-Acetyl-1α-O-benzyl-6-O-(N-benzyloxycarbonyl-glycyl)-normuramyl-L-alanyl-D-isoglutamin-tert.-butylester in Form von farblosen Nadeln, Smp. 155-160°; $[\alpha]_D^{20}$ = +60±1° (c = 1,2; Dimethylformamid), $R_f$ = 0,86 (Chloroform:Methanol:Wasser = 70:30:5), $R_f$ = 0,93 (n-Butanol:Eisessig:Wasser = 75:7,5:21).

Die obige Verbindung kann auch unter Verwendung des symmetrischen Anhydrids von N-Benzyloxycarbonyl-glycin in Gegenwart von Pyridin erhalten werden.

Das obengenannte geschützte N-Acetyl-6-O-glycyl-normuramyl-L-alanyl-D-isoglutamin lässt sich mit anderen Schutzgruppen versehen auch wie folgt herstellen:

6,8 g (7,2 mMol) N-Acetyl-6-O-[{2-(p-biphenylyl)-isopropyloxycarbonyl}-glycyl]-normuramyl-L-alanyl-D-isoglutamin-γ-diphenylmethylester, gelöst in 100 ml 90%igem Trifluoräthanol, werden bei Raumtemperatur mit 0,1 N HCl in 90%igem Trifluoräthanol bei pH = 0,5 (pH-Stat) innerhalb von einer Stunde gespalten. Das Lösungsmittel wird verdampft, der Rückstand nach dem Trocknen mehrfach mit 1,2-Dimethoxyäthan verrieben und das Überstehende abdekantiert. Man erhält 4,7 g (88%) amorphes N-Acetyl-6-O-glycyl-normuramyl-L-alanyl-D-isoglutamin-γ-diphenylmethylester-hydrochlorid, $[\alpha]_D^{20} = +17°$ (c = 0,396; Methanol), $R_f$ = 0,17 (Isopropanol:Wasser = 80:20), $R_f$ = 0,27 (Essigsäureäthylester:n-Butanol:Pyridin:Essigsäure:Wasser = 42:21:21:6:10).

Das verwendete Ausgangsmaterial erhält man wie folgt:

3,77 g (12 mMol) 2-(p-Biphenylyl)-isopropyloxycarbonyl-glycin und 7,33 g (12 mMol) N-Acetyl-normuramyl-L-alanyl-D-isoglutamin-γ-diphenylmethylester, gelöst in 100 ml Dimethylformamid, werden nach Zusatz von 3,2 g (24 mMol) Hydroxybenztriazol, 1,44 g (12 mMol) 4-Dimethylaminopyridin und 2,72 g (13,2 mMol) Dicyclohexylcarbodiimid wie oben beschrieben kondensiert. Der nach üblicher Aufarbeitung anfallende Rückstand (9,8 g) wird zur Entfernung der 4,6-Diacylverbindung durch Gegenstromverteilung nach Craig im System Methanol:Wasser:Chloroform:Tetrachlormethan = 13,5:3,5:4,5:8) gereinigt. Nach 600 Stufen wird das reine Material gesammelt. Man erhält 6,9 g (61%) N-Acetyl-6-O-[{2-(p-biphenylyl)-isopropyloxycarbonyl}-glycyl]-normuramyl-L-alanyl-D-iso-

glutamin-γ-diphenylmethylester, $[\alpha]_D^{20}$ = +16° (c = 0,837; Methanol), $R_f$ = 0,72 (Chloroform:Methanol:Wasser = 70:30:5).

Beispiel 5: In analoger Weise zu Beispiel 4, ausgehend von 2-[{(3'R)-Hydroxy-(2'S)-palmitoylamino-octadecycloxy}-hydroxyphosphoryloxy]-äthylamin bzw. 2-(1,2-Dipalmitoyl-sn-glycero-3-hydroxyphosphoryloxy)-äthylamin und N-Acetyl-4,6-O-distearoyl-normuramyl-L-alanyl-D-isoglutamin-N-hydroxysuccinimidester bzw. N-Acetyl-1,4,6-O-triacetyl-muramyl-L-alanyl-D-isoglutamin-N-hydroxy-succinimidester, erhält man

N-Acetyl-4,6-O-distearoyl-normuramyl-L-alanyl-D-isoglutamin-2-[{(3'R)-hydroxy-(2'S)-palmitoylamino-octadecyloxy}-hydroxyphosphoryloxy]-äthylamid,

N-Acetyl-4,6-O-distearoyl-normuramyl-L-alanyl-D-isoglutamin-2-(1',2'-dipalmitoyl-sn-glycero-3'-hydroxyphosphoryloxy)-äthylamid,

N-Acetyl-1,4,6-O-triacetyl-muramyl-L-alanyl-D-isoglutamin-2-[{(3'R)-hydroxy-(2'S)-palmitoylamino-octadecyloxy}-hydroxyphosphoryloxy]-äthylamid und

N-Acetyl-1,4,6-O-triacetyl-muramyl-L-alanyl-D-isoglutamin-2-(1',2'-dipalmitoyl-sn-glycero-3'-hydroxyphosphoryloxy)-äthylamid, jeweils in Form von oder im Gemisch mit dem Natriumsalz.

Die Ausgangssubstanzen werden wie folgt hergestellt:

Eine Lösung von 3,29 g (5mMol) N-Acetyl-1α-O-benzyl-normuramyl-L-alanyl-D-isoglutamin-γ-benzylester in 40 ml abs. Pyridin wird in der Kälte unter Rühren und Feuchtigkeitsausschluss tropfenweise mit einer Lösung von 3,63 g (12mMol) Stearinsäurechlorid in 30 ml abs. Tetrahydrofuran versetzt. Nach 16stündigem Stehen bei Raumtemperatur wird auf Eiswasser gegossen, das ausgefallene Produkt gesammelt und an Kieselgel (1:40) mit einem Gemisch von Chloroform:Essigsäureäthylester = 1:1 gereinigt.

1,6 g des im Dünnschichtchromatogramm einheitlichen Materials werden in 40 ml eines Gemisches von tert. Butanol : Wasser = 98:2 gelöst und

in Gegenwart von 0,4 g Pd auf Kohle (10%ig) während 30 Stunden hydriert. Der Katalysator wird abfiltriert, mit dem obengenannten Lösungsmittelgemisch mehrmals bei 40° extrahiert und die vereinigten Filtrate eingedampft. Der Rückstand wird in 6 ml Chloroform gelöst, durch ein PTFE-Milliporefilter (0,2 μ) filtriert und das Produkt durch Zugabe von 80 ml Aether : Petroläther = 4:1 in der Kälte (-10°) ausgefällt. Man erhält 1,2 g N-Acetyl-4,6-O-distearoyl-normuramyl-L-alanyl-D-isoglutamin als amorphes Pulver,

$[\alpha]_D^{20} = + 23 \pm 1°$ (c = 1; Chloroform),

$R_f$ = 0,37 (Choroform:Methanol:Wasser = 70:30:5),

$R_f$ = 0,77 (Essigsäureäthylester:n-Butanol:Pyridin:Essigsäure:Wasser = 42:21:21:6:10).

3,69 g (5,6mMol) N-Acetyl-muramyl-L-alanyl-D-isoglutamin-γ-diphenylmethylester werden in 40 ml abs. Pyridin gelöst, 2,04 g (20mMol) Essigsäureanhydrid zugegeben und das Ganze während einer Stunde stehen gelassen. Die klare Lösung wird bei 30° eingeengt, mit 200 ml Essigsäureäthylester verdünnt und die Essigesterphase 4mal mit je 50 ml Wasser extrahiert. Der nach dem Trocknen und Eindampfen verbleibende Rückstand wird in 40 ml Methanol : Tetrahydrofuran = 1:1 gelöst und nach Zugabe von 0,8 g eines Palladium-auf-Kohle-Katalysators (10%ig) während einer Stunde mit Wasserstoff behandelt. Der Katalysator wird abgesaugt, das Filtrat zur Trockne verdampft und zwischen n-Butanol und Wasser verteilt (6mal). Die im Dünnschichtchromatogramm einheitlichen Phasen werden vereinigt und bei 30° stark eingeengt. Man versetzt mehrfach mit Wasser und engt wieder ein. Schliesslich wird mit dest. Wasser auf total 100 ml verdünnt, die Lösung durch ein Milliporefilter (0,45 μ) filtriert und lyophilisiert. Es verbleiben 2 g N-Acetyl-1,4,6-O-triacetyl-muramyl-L-alanyl-D-isoglutamin als farbloses Pulver,

$[\alpha]_D^{20} = + 57 \pm 1°$ (c = 0,92; Wasser),

$R_f$ = 0,4 (Dichlormethan : Methanol : Essigsäure = 20:5:1).

Den als Ausgangsmaterial benötigten Diphenylmethylester erhält man wie folgt:

Eine Lösung von 5,1 g N-Acetyl-muramyl-L-alanyl-D-isoglutamin in 130 ml 1,2-Dimethoxyäthan : Wasser = 1:1 wird mit 2,9 g Diphenyldiazomethan versetzt und während 24 Stunden bei 40° gerührt. Zu der entfärbten Lösung werden nochmals 1,5 g Reagens gegeben und weitere 24 Stunden bei obiger Temperatur gerührt. Die erkaltete Suspension wird filtriert, der Niederschlag mit Aether verrieben und farblos gewaschen. Der feste Rückstand wird in 50 ml Wasser suspendiert, nach 30 Minuten abfiltriert und getrocknet. Es verbleiben 5 g N-Acetyl-muramyl-L-alanyl-D-isoglutamin-γ-diphenylmethylester, $R_f$ = 0,5 (Dichlormethan:Methanol:Wasser = 14:6:1).

Beispiel 6: In analoger Weise zu Beispiel 4 ausgehend vom N-Hydroxy-succinimidester des Bernsteinsäure-2-(1'-palmitoyl-2'-oleoyl-sn-glycero-3'-hydroxyphosphoryloxy)-äthylamids und N-Acetyl-normuramyl-L-(O-glycyl)-seryl-D-isoglutamin-γ-diphenylmethylester-hydrochlorid erhält man nach Spaltung des Diphenylmethylesters N-Acetyl-normuramyl-L-O- {N-[2-(1'-palmitoyl-2'-oleoyl-sn-glycero-3'-hydroxyphosphoryloxy)-äthyl ]-succinamoyl--glycyl }-seryl-D-isoglutamin, das teilweise in Natriumsalzform vorliegt.

Analog werden die folgenden Verbindungen, die teilweise in Natriumsalzform vorliegen, hergestellt:

N-Acetyl-muramyl-L-O- {(N-[2-(1'-palmitoyl-2'-oleoyl-sn-glycero-3'-hydroxyphosphoryloxy)-äthyl]-succinamoyl)-glycyl }-seryl-D-isoglutamin,

N-Acetyl-muramyl-D-O- {(N-[2-(1'-palmitoyl-2'-oleoyl-sn-glycero-3'-hydroxyphosphoryloxy)-äthyl]-succinamoyl)-glycyl }-seryl-D-isoglutamin,

N-Acetyl-muramyl-D-O-{(N-[2-(1'-palmitoyl-2'oleoyl-sn-glycero-3'-hydroxyphosphoryloxy)-äthyl]-succinamoyl)-leucyl}-seryl-D-isoglutamin,

2-Acetylamino-2-desoxy-D-glucopyranosyl-(3)-L-propionyl-D-O- {(N-[2-(1'-palmitoyl-2'-oleoyl-sn-glycero-3'-hydroxyphosphoryloxy)-äthyl]-succinamoyl)-glycyl }-seryl-D-isoglutamin,

2-Acetylamino-2-desoxy-D-mannopyranosyl-(3)-D-propionyl-L-O-{(N-[2-(1'-palmitoyl-2'-oleoyl-sn-glycero-3'-hydroxyphosphoryloxy)-äthyl]-succinamoyl)-glycyl}-seryl-D-isoglutamin,

2-Acetylamino-2-desoxy-D-galactopyranosyl-(3)-D-propionyl-L-O-{(N-2-(1'-palmitoyl-2'-oleoyl-sn-glycero-3'-hydroxyphosphoryloxy)-äthyl]-succinamoyl)-glycyl}-seryl-D-isoglutamin,

N-Acetyl-normuramyl-L-O-{(N-[2-(1'-palmitoyl-2'-oleoyl-sn-glycero-3'-hydroxyphosphoryloxy)-äthyl]-succinamoyl)-glycyl}-seryl-D-glutaminsäure-diamid,

N-Propionyl-normuramyl-L-O-{(N-[2-(1'-palmitoyl-2'-oleoyl-sn-glycero-3'-hydroxyphosphoryloxy)-äthyl]-succinamoyl)-glycyl}-seryl-D-isoglutamin,

N-Benzoyl-normuramyl-L-O-{(N-[2-(1'-palmitoyl-2'-oleoyl-sn-glycero-3'-hydroxyphosphoryloxy)-äthyl]-succinamoyl)-glycyl}-seryl-D-glutaminsäure-dimethylester,

N-Acetyl-muramyl-L-O-{(N-[2-(1'-palmitoyl-2'-oleoyl-sn-glycero-3'-hydroxyphosphoryloxy)-äthyl]-succinamoyl)-alanyl}-threonyl-D-isoglutamin,

N-Acetyl-muramyl-L-O-{(N-[2-(1'palmitoyl-2'-oleoyl-sn-glycero-3'-hydroxyphosphoryloxy)-äthyl]-succinamoyl)-alanyl}-hydroxyprolyl-D-isoglutaminyl-alanin,

N-Acetyl-muramyl-L-O-{(N-[2-(1'-palmitoyl-2'-oleoyl-sn-glycero-3'-hydroxyphosphoryloxy)-äthyl]-succinamoyl)-glycyl}-(N-methyl)-seryl-D-isoglutamin,

N-Acetyl-muramyl-L-O- {(N-[2-(1'-palmitoyl-2'-oleoyl-sn-glycero-3'-hydroxyphosphoryloxy)-äthyl]-succinamoyl)-glycyl}-seryl-D-(γ-carboxy)-isoglutamin,

N-Acetyl-muramyl-L-O- {(N-[2-(1'-palmitoyl-2'oleoyl-sn-glycero-3'-hydroxyphosphoryloxy)-äthyl]-succinamoyl)-glycyl}-seryl-D-(γ-carboxy)-glutamyl-glycinamid.

Beispiel 7: N-Acetyl-1α-O-benzyl-4,6-O-isopropyliden-normuramyl-L-seryl-D-isoglutamin-benzylester wird in Gegenwart von Pyridin und 2,4,6-Triisopropyl-benzolsulfonylchlorid mit (1,2-Dipalmitoyl-rac-glycero-3)-phosphorsäureester-di-Natrium-Salz zu N-Acetyl-1α-O-benzyl-4,6-O-isopropyliden-normuramyl-L-O-(1,2-dipalmitoyl-rac-glycero-3-hydroxyphosphoryl)-seryl-D-isoglutamin, das teilweise in Salzform vorliegt, umgesetzt, woraus zunächst durch saure Hydrolyse die Isopropylidengruppe, dann durch katalytische Hydrierung der Benzylrest, wie jeweils oben beschrieben, abgespalten werden, wonach man N-Acetyl-normuramyl-L-O-(1,2-dipalmitoyl-rac-glycero-3-hydroxyphosphoryl)-seryl-D-isoglutamin erhält, das teilweise in Natriumsalzform vorliegt.

Beispiel 8: Aus N-Benzyloxycarbonyl-1α-O-benzyl-muramyl-L-alanyl-D-isoglutamin-tert.butylester wird die Benzyloxycarbonylgruppe wie üblich abgespalten. Das erhaltene Produkt mit freier 2-Amino-Gruppe wird mit 2-(1,2-Dipalmitoyl-rac-glycero-3-hydroxyphosphoryloxy)-essigsäure-N-succinimidester zu 1α-O-Benzyl-N-(1,2-dipalmitoyl-rac-glycero-3-hydroxyphosphoryloxy)-acetyl-muramyl-L-alanyl-D-isoglutamin-tert.-butylester umgesetzt. Nach Verseifung des tert.Butylesters mit Trifluoressigsäure in Methylenchlorid und nachfolgender Abspaltung der Benzylschutzgruppe mit Wasserstoff in Gegenwart eines Palladiumkatalysators erhält man N-(1,2-Dipalmitoyl-rac-glycero-3-hydroxyphosphoryloxy)-acetyl-muramyl-L-alanyl-D-isoglutamin.

Beispiel 9: N-Acetyl-1α-O-benzyl-normuramyl-L-alanyl-D-isoglutamin-benzylester wird nach der Steglich-Methode (Dimethylaminopyridin, Dicyclohexylcarbodiimid und Pyridin) mit (1,2-Dipalmitoyl-rac-glycero-hydroxyphosphoryloxy)-essigsäure-N-succinimidester umgesetzt. Nach Abspaltung der Benzylschutzgruppen mit Wasserstoff in Gegenwart eines Palladiumkatalysators erhält man N-Acetyl-6-O-([1,2-dipalmitoyl-rac-glycero-3-hydroxyphosphoryloxy]-acetyl)-normuramyl-L-alanyl-D-isoglutamin.

Beispiel 10: N-Acetyl-normuramyl-1α-O-benzyl-L-alanyl-D-isoglutamin-benzylester wird in Gegenwart von Pyridin und 2,4,6-Triisopropylbenzolsulfonylchlorid mit (1,2-Dipalmitoyl-rac-glycero-3)-phosphorsäureester-di-Natriumsalz umgesetzt. Aus dem erhaltenen Produkt erhält man nach Abspaltung der Benzylschutzgruppen mit Wasserstoff in Gegenwart eines Palladiumkatalysators N-Acetyl-6-O-(1,2-dipalmitoyl-rac-glycero-3-hydroxyphosphoryloxy)-normuramyl-L-alanyl-D-isoglutamin.

Beispiel 11: N-Acetyl-1α-O-benzyl-4,6-O-benzyliden-muraminsäure-äthylester wird mit siedender Kaliumhydroxidlösung zum Kaliumsalz der 1α-O-Benzyl-4,6-O-benzyliden-muraminsäure verseift. Nach Umsetzung des erhaltenen Produkts mit Benzyloxycarbonylchlorid bei pH 8 erhält man N-Benzyloxycarbonyl-1α-O-benzyl-4,6-O-benzyliden-muraminsäure, die mit L-Alanyl-D-isoglutamin-tert.butylester in Gegenwart von Dicyclohexylcarbodiimid und N-Hydroxysuccinimid zu N-Benzyloxycarbonyl-1α-O-benzyl-4,6-O-benzyliden-muramyl-L-alanyl-D-isoglutamin-tert.butylester umgesetzt wird. Der Benzyloxycarbonylrest und die Benzylidengruppe werden durch milde, kurze Hydrierung am Palladium-auf-Kohle-Katalysator selektiv abgespalten. Das erhaltene Produkt wird an der freien 2-Amino-Gruppe mit Bernsteinsäuremonobenzylester in Gegenwart von Dicyclohexylcarbodiimid und N-Hydroxy-succinimid zu N-Benzyloxysuccinyl-1α-O-benzyl-muramyl-L-alanyl-D-isoglutamin-tert.butylester acyliert. Daraus wird durch längere Hydrierung am Pd/C-Kontakt die 1α-O-Benzyl-Schutzgruppe abgespalten.

Das erhaltene Produkt wird mit 2-(1,2-Dipalmitoyl-sn-glycero-3-hydroxy-phosphoryloxy)-äthylamin in Gegenwart von Dicyclohexylcarbodiimid und N-Hydroxy-succinimid zu N-(2-[1,2-Dipalmitoyl-sn-glycero-hydroxyphosphoryloxy]-äthyl)-succinamoyl-muramyl-L-alanyl-D-isoglutamin-tert.-butylester umgesetzt, aus dem nach saurer Abspaltung der tert.Butylgruppe N-(2-[1,2-Dipalmitoyl-sn-glycero-hydroxyphosphoryloxy]-äthyl)-succinamoyl-muramyl-L-alanyl-D-isoglutamin erhalten wird.

Beispiel 12: Herstellung von 1000 Kapseln mit 260 mg der aktiven Ingredienzen pro Kapsel:

Zusammensetzung:

| | |
|---|---|
| Rifampicin | 250 g |
| N-(2-[1,2-Dipalmitoyl-sn-glycero-hydroxy-phosphoryloxy]-äthyl)-succinamoyl-muramyl-L-alanyl-D-isoglutamin, das teilweise in Natriumsalzform vorliegt | 10 g |
| Talk | 36 g |
| Weizenstärke | 24 g |
| Magnesiumstearat | 16 g |
| Laktose | 4 g |
| | 340 g |

Zubereitung: Die pulverförmigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

Beispiel 13: Herstellung von 1000 Kapseln enthaltend 105 mg der aktiven Stoffe pro Kapsel:

Zusammensetzung

| | |
|---|---|
| Rifampicin – | 100 g |
| N-Acetyl-normuramyl-L-O-(1,2-dipalmitoyl-rac-glycero-3-hydroxyphosphoryl)-seryl-D-isoglutamin, das teilweise in Natriumsalzform vorliegt | 5 g |
| Aethyl-Cellulose | 3 g |
| Stearinsäure | 3 g |
| | 111 g |

Zubereitung: Die Aethyl-Cellulose und die Stearinsäure werden in 120 ml Methylenchlorid gelöst, mit dem Antibiotikum versetzt, und die Masse wird durch ein Sieb von 0,6 mm Maschenweite bei einer Temperatur von ca. 40° geschlagen, wobei das Methylenchlorid verdampft. 156 mg des erhaltenen Granulates werden in Gelatine-Kapseln zu 0,5 ml mit Hilfe einer Kapsel-Abfüllmaschine abgefüllt.

Beispiel 14: Herstellung eines Futtermittels enthaltend 0,005 % der aktiven Stoffe:

Vor-Mischung:

| | |
|---|---|
| Rifampicin oder Chlortetracyclin | 30 g |
| N-Acetyl-6-O-{N-[2-(1,2-dipalmitoyl-sn-glycero-3-hydroxyphosphoryloxy)-äthyl]-succinamoyl}-normuramyl-L-alanyl-D-isoglutamin, das teilweise in Natriumsalzform vorliegt | 10 g |
| Staubzucker | 50 g |
| Sojabohnen Futter (mit Lösungsmitteln extrahiert) | 275 g |
| | 365 g |

Zusatzstoffe:

| | |
|---|---|
| Maismehl | 500,0 kg |
| Sojabohnenmehl, 44 % Protein | 300,0 kg |
| Alfalfamehl | 13,5 kg |
| Dicalciumphosphat | 18,0 kg |

| | |
|---|---|
| Calciumcarbonat (gemahlen) | 4,5 kg |
| Salz | 2,3 kg |
| Fischmehl, 60 % Protein | 18,0 kg |
| Stab. Fett | 27,0 kg |
| trockener Molkerückstand | 18,0 kg |
| Mangansulfat | 0,2 kg |
| Zinkoxid | 1,3 kg |
| d,l-Methionin | 0,7 kg |
| Vitamin-Vormischung | 4,5 kg |
| | 908,0 kg |

Die Vitamin-Vormischung enthält in 4,5 kg: 16 000 000 I.E. Vit. A, 1 000 000 I.E. Vit. $D_3$, 5 000 I.E. Vit. E Acetat, 6 g Vit. $K_3$, 6 mg Vit. $B_{12}$, 3 g Riboflavin, 30 g Niacin, 5 g Calcium Pantothenat und 100 g Ethoxyquin (1,2-Dihydro-6-äthoxy-2,2,4-trimethyl-chinolin) und Maismehl bis auf 4,5 kg.

Herstellungsweise: Die Wirkstoffe und Zucker werden gründlich miteinander gemischt, durch ein Sieb von 0,6 mm Maschenweite geschlagen und dann mit dem Sojabohnenmehl vermischt. Die Vormischung wird dann in der der gewünschten Endkonzentration entsprechenden Menge zum Futtermittel zugegeben und in einem horizontalen Trommelmischer homogenisiert.

Beispiel 15: 120 mg N-{N-[2-(1',2'-dipalmitoyl-sn-glycero-3'-hydroxyphosphoryloxy)-äthyl]-succinamoyl}-muramyl-L-alanyl-D-isoglutamin-tert.-butylester-natriumsalz löst man in 12 ml einer Mischung aus 3 ml Trifluoressigsäure und 9 ml Methylenchlorid und lässt 3 Stunden bei Raumtemperatur stehen. Man dampft im Vakuum bei 40° zur Trockne, verreibt den Rückstand mehrmals mit Aether und reinigt den farblosen krist. Rückstand an Kieselgel im Laufmittel Chloroform: Methanol:

Natriumphosphatpuffer (pH = 7) = 55:45:2.

Man erhält ein farbloses Kristallisat von N-{N-[2-(1',2'-dipalmitoyl-sn-glycero-3'-hydroxyphosphoryloxy)-äthyl]-succinamoyl}-muramyl-L-alanyl-D-isoglutamin, das teilweise in Natriumsalzform vorliegt, mit dem Zers.-Punkt: 196-197°; $R_f$ = 0,23 (Chloroform:Methanol:Wasser = 60:40:2), $[\alpha]_D^{20}$ = +15° (c = 0,9; Chloroform:Methanol = 1:1).

Die Substanz reagiert positiv mit Phosphorspray [V.E. Vaskovsky et al., J. Lipid Research 9, 396 (1968)], mit Anilinhydrogenphthalat, mit verdünnter Schwefelsäure und mit Natriumhypochlorit/N,N-Tetramethyl-4,4'-diaminodiphenylmethan (TDM). 360 MHz-$^1$H-NMR-Spektrum ($CDCl_3$: $CD_3OD$ = 1:1) :

$\delta$ = 1.40 ($CH_3$ von Alanin und der Propionylgruppe),
0.88, 1.27 und 1.60 (Methyl- und Methylengruppen [ausser α-Methylengruppen] der Palmitoylreste),
2.32 (α-Methylengruppen der Palmitoylreste),
1.9-2.2 (Methylengruppen der Succinamoyl- und Isoglutaminreste),
4.15 (γ-H des Glycerorestes),
5.22 (β-H des Glycerorestes),
5.36 (1-H des Muramylrestes).

Das Ausgangsmaterial erhält man wie folgt:
404,6 mg (0,733 mMol) des Ameisensäureadditionssalzes an Muramyl-L-alanyl-D-isoglutamin-γ-tert.-butylester, 720 mg (0,807 mMol) N-[2-(1',2'-Dipalmitoyl-sn-glycero-3'-hydroxyphosphoryloxy)-äthyl]-succinamid-Natriumsalz, 118 mg (1,026 mMol) N-Hydroxysuccinimid, 211 mg (1,026 mMol) Dicyclohexylcarbodiimid und 142 ml (1,026 mMol) Triäthylamin löst man in einer Mischung aus 12 ml Dimethylacetamid und 4 ml Chloroform und lässt 18 Stdn. bei Raumtemperatur reagieren. Nach Zusatz von 1 ml Wasser wird die Reaktionslösung im Vakuum eingedampft, mit Chloroform aufgenommen und von Kristallen (Dicyclohexylharnstoff) abgesaugt.

Das Filtrat wird an Kieselgel Merck chromatographiert. Man eluiert nacheinander mit je 200 ml der Lösungsmittelgemische Chloroform: Methanol = 9:1, Chloroform:Methanol = 8:2, Chloroform:Methanol = 7:3, Chloroform:Methanol:Wasser = 70:30:1 und Chloroform:Methanol:Wasser = 70:30:10.

Die Fraktionen der gesuchten Substanz, die positiv reagieren mit Phosphorspray, Anilinhydrogenphthalat, verdünnter Schwefelsäure und Natriumhypochlorit/TDM werden gesammelt und liefern nach dem Eindampfen ein farbloses Pulver mit dem Zersetzungsbereich 144-152° (Anomerengemisch) und $R_f$ = 0,455 (Chloroform:Methanol:Waser = 70:30:3).

Das als Ausgangsmaterial für obigen Versuch verwendete Muramylpeptid erhält man wie folgt:

1,6 g N-tert.-Butoxycarbonyl-4,6-O-isopropyliden-muramyl-L-alanyl-D-isoglutamin-γ-tert.-butylester löst man in einer Mischung aus 1 ml Dimethoxyäthan und 25 ml 40proz. wässriger Ameisensäure. Nach 36 Stunden bei Raumtemperatur (25°) und 18 Stunden bei 4° verdünnt man mit 75 ml destilliertem Wasser und lyophilisiert. Man erhält ein farbloses, amorphes Pulver, das an Kieselgel, Merck, chromatographiert wird, wobei man nacheinander jeweils Elutionsmischungen aus Chloroform: Methanol: Wasser in den folgenden Mengenverhältnissen verwendet: 90:10:1; 80:20:1; 70:30:1 und 60:40:1.

Man erhält nach dem Eindampfen der reinen Fraktionen ein farbloses, amorphes Pulver des Ameisensäureadditionssalzes an Muramyl-L-alanyl-D-isoglutamin-γ-tert.-butylester mit $R_f$ = 0,31 (Chloroform:Methanol: Wasser = 60:40:2), $[\alpha]_D^{20}$ = +57° (c = 0.956, Methanol) und Zersetzungsbereich 70-90° (Anomerengemisch). Die Substanz reagiert positiv mit Ninhydrin, Anilinhydrogenphthalat, verdünnter Schwefelsäure und Natriumhypochlorit/TDM.

Das als Ausgangsmaterial verwendete Succinoylkephalin erhält man auf folgende Weise:

4 g 2-(1',2'-Dipalmitoyl-sn-glycero-3'-hydroxyphosphoryloxy)-äthylamin (Kephalin) und 1,16 g Bernsteinsäureanhydrid suspendiert man in 150 ml einer Mischung aus Chloroform:Methanol:Wasser = 8:10:4 und gibt unter Rühren 2,4 ml Triäthylamin zu. Man erhält eine klare, farblose Lösung, die nach 2 Stunden bei Raumtemperatur mit weiteren 0,3 g Bernsteinsäureanhydrid und 0,6 ml Triäthylamin versetzt wird. Nach einer weiteren Stunde bei Raumtemperatur dampft man die Lösung im Vakuum ein. Man löst den Rückstand in wenig Tetrahydrofuran, stellt mit 1normaler Salzsäure auf pH = 2 und extrahiert das Phospholipid mit Essigester. Die organische Phase wäscht man mit Wasser, dann mit Natriumchloridlösung. Nach dem Trocknen der Essigesterphase mit Natriumsulfat erhält man chromatographisch reines N-[2-(1',2'-Dipalmitoyl-sn-glycero-3'-hydroxyphosphoryloxy)-äthyl]-succinamid, das teilweise in Natriumsalzform vorliegt, mit $R_f$ = 0,3 (Chloroform:Methanol:Wasser = 70:30:3; zum Vergleich: $R_f$ von Kephalin = 0,535).

Der Schmelzpunkt ist unscharf (82-110°), da sich die Verbindung gemäss DC-Kontrolle beim Erhitzen in eine lipophile Verbindung teilweise umwandelt.

Den N-tert.-Butoxycarbonyl-4,6-O-isopropyliden-muramyl-L-alanyl-D-isoglutamin-γ-tert.-butylester erhält man durch katalytische Hydrierung des entsprechenden α-Benzylglykosids:

3,6 g Benzylglykosid hydriert man bei Normaldruck in 90 ml einer Mischung aus Dimethoxyäthan:Methylenchlorid = 8:2 unter Zusatz von 2,2 g 10proz. Palladium-auf-Kohlenstoff. Nach 90 Stdn. ist die Hydrierung beendet; man filtriert den Katalysator ab und dampft im Vakuum

zur Trockne ein. Es resultiert ein farbloses, amorphes Pulver mit Smp. 180-188° und $R_f$ = 0,36 (Chloroform:Methanol = 9:1; zum Vergleich: $R_f$ von Benzylglykosid = 0,6). Die Verbindung reagiert positiv mit Anilinhydrogenphthalat, verdünnter Schwefelsäure oder Natriumhypochlorit/TDM.

Das als Ausgangsmaterial eingesetzte Benzylglykosid gewinnt man wie folgt:

6 g (12,5 mMol) 1α-O-Benzyl-N-tert.-butoxycarbonyl-4,6-O-isopropyliden-muraminsäure, 4,6 g (15 mMol) Hydrochlorid des L-Alanyl-D-isoglutamin-γ-tert.-butylesters, 3,1 g (15 mMol) Dicyclohexylcarbodiimid, 1,7 g N-Hydroxysuccinimid und 2,07 ml (15 mMol) Triäthylamin löst man in 120 ml einer Mischung aus Dimethylformamid:Methylenchlorid = 3:2.

Die klare, farblose Lösung wird 5 Stunden bei Raumtemperatur gerührt, wobei Dicyclohexylharnstoff ausfällt. Man saugt vom Niederschlag ab und dampft das Filtrat im Vakuum zur Trockne. Den Rückstand nimmt man mit Methylenchlorid auf und schüttelt einmal mit 0,1normaler Salzsäure, dann sofort mehrmals mit Wasser aus, bis die Probe auf Chloridionen negativ ausfällt. Man trocknet die organische Phase über Natriumsulfat und dampft die Lösung zur Trockne. Der Rückstand wird mit Essigester bei Raumtemperatur extrahiert, vom ungelösten Dicyclohexylharnstoff abgesaugt, und das Filtrat mit Petroläther versetzt. Der 1α-O-Benzyl-N-tert.-butoxycarbonyl-4,6-O-isopropyliden-muramyl-L-alanyl-D-isoglutamin-γ-tert.-butylester mit Smp. 93-97° und $[\alpha]_D^{20}$ = +80° (c = 0.98; Chloroform) fällt farblos kristallin aus.

Das Kaliumsalz der 1α-O-Benzyl-N-tert.-butoxycarbonyl-4,6-O-isopropyliden-muraminsäure erhält man wie folgt:

22.7 g 1α-O-Benzyl-N-acetyl-4,6-O-isopropyliden-muraminsäure-äthylester lässt man bei Raumtemperatur in einer Lösung aus 5,1 g Kaliumhydroxid in 200 ml Methanol und 5 ml Wasser 12 Stdn. stehen. Danach ist der Aethylester verseift. Man engt im Vakuum ein, versetzt den Rückstand mit 80 ml Hydrazinhydrat und lässt 48 Stunden bei 80° reagieren. Dann dampft man die Lösung bei 0,1 Torr ein und verteilt den Rückstand zwischen gesättigter Kaliumchloridlösung und Tetrahydrofuran. Das Kaliumsalz der 1α-O-Benzyl-4,6-O-isopropyliden-muraminsäure mit $R_f$ = 0,5 (Chloroform:Methanol = 3:1) befindet sich in der organischen Phase zusammen mit etwas unverseifter N-Acetyl-Verbindung mit $R_f$ = 0.6.

Das so erhaltene rohe Amin wird direkt mit Di-O-tert.-butyl-kohlensäureanhydrid umgesetzt:

9.4 g des rohen Amins löst man in einer Mischung aus 35 ml Dioxan und 25 ml Wasser, gibt eine Lösung von 3.2 g Kaliumcarbonat in 20 ml Wasser zu und tropft zu dieser Mischung bei 10° eine Lösung von Di-O-tert.-butyl-kohlensäureanhydrid in 20 ml Dioxan. Dann lässt man bei Raumtemperatur noch 2 Stunden zu Ende reagieren. Der pH-Wert der Lösung wird durch Zugabe von Kaliumcarbonatlösung dauernd bei 9-10 gehalten. Die entstandene Suspension stellt man mit 2normaler Salzsäure auf pH = 7, sättigt die wässrige Phase mit Kaliumchlorid und extrahiert mit Essigester. Die organische Phase wird mit Wasser gewaschen,über Natriumsulfat getrocknet und eingedampft. Der so erhaltene Rückstand wird an Kieselgel,Merck,chromatographisch nacheinander mit den Lösungsmittelgemischen Aether: Essigester = 1:1 und Chloroform: Aceton = 8:2 gereinigt.

Das so erhaltene Kaliumsalz der 1α-O-Benzyl-N-tert.-butoxycarbonyl-4,6-O-isopropyliden-muraminsäure zersetzt sich bei 250-255°; $R_f$ = 0.82 (Aether: Tetrahydrofuran = 9:1),$R_f$ = 0.64 (Methylen-

chlorid:Methanol = 9:1), $[\alpha]_D^{20}$ = +105° (c = 1.07; Chloroform).

Beispiel 16: 828 mg (1,494 mMol) des in Beispiel 15 beschriebenen Ameisensäureadditionssalzes an Muramyl-L-alanyl-D-isoglutamin-γ-tert.-butylester, 726 mg (0.996 mMol) 2-(1',2'-Dipalmitoyl-rac-glycero-3'-hydroxyphosphoryloxy)-essigsäure-mononatriumsalz, und 620 mg (2,49 mMol) 2-Aethoxy-N-carbäthoxy-1,2-dihydrochinolin (EEDQ) löst man in 17 ml einer Mischung aus Chloroform:Methanol:Wasser = 75:25:4 und stellt den pH-Wert mit 138 µl N-Aethylmorpholin auf 7 ein. Nach 24 Stunden bei Raumtemperatur ist die Reaktion nach DC-Kontrolle beendet. Man dampft im Vakuum zum Sirup ein und reinigt durch Chromatographie an Kieselgel Merck mit dem Lösungsmittelgemisch Chloroform:Methanol:Wasser = 70:30:1. Das Mono-natriumsalz des N-{2-[1',2'-dipalmitoyl-rac-glycero-3'-hydroxyphosphoryloxy]-acetyl}-muramyl-L-alanyl-D-isoglutamin-γ-tert.-butylesters erhält man als farbloses, amorphes Pulver mit $R_f$ = 0,68 (Chloroform:Methanol:Wasser = 70:30:1) und $[\alpha]_D^{20}$ = +10° (c = 0.8; Chloroform:Methanol = 1:1).

200 mg des erhaltenen tert.-Butylesters löst man in 16 ml einer Mischung aus Trifluoressigsäure:Methylenchlorid = 1:3. Nach 3 Stunden bei Raumtemperatur dampft man im Vakuum zur Trockne und extrahiert den Rückstand mehrmals mit Aether. Man erhält so ein farbloses, amorphes Pulver des Mononatriumsalzes des N-{2-[1',2'-dipalmitoyl-rac-glycero-3'-hydroxyphosphoryloxy]-acetyl}-muramyl-L-alanyl-D-isoglutamins mit $R_f$ = 0.095 (Chloroform: Methanol: Wasser=70:30:1) und $R_f$ = 0.25 (Chloroform: Methanol: Wasser = 55:45:1).

Die Substanz reagiert positiv mit Anilinhydrogenphthalat, Phosphorspray, Natriumhypochlorit/TDM und verdünnter Schwefelsäure.

Das als Ausgangsmaterial verwendete Natriumsalz der 2-(1,2-Dipalmitoyl-rac-glycero-3-hydroxyphosphoryloxy)-essigsäure erhält man durch katalytische Hydrierung des entsprechenden Benzylesters mit 10proz. Pd-Kohle in einer Mischung aus Dimethoxyäthan:Chloroform = 1:1. Die Verbindung fällt als farbloses Kristallisat an mit $R_f$ = 0,1 (Chloroform: Methanol: Wasser = 70:30:3) und Smp. 105-108°.

Den als Ausgangsmaterial dienenden Benzylester dieser Verbindung stellt man wie folgt her:

Zu 0,704 ml (7,7 mMol) Phosphorylchlorid ($POCl_3$) in 10 ml Tetrahydrofuran tropft man bei 10° 4,0 g (7 mMol) 1,2-O-Dipalmitoyl-rac-glycerin in 20 ml Tetrahydrofuran und anschliessend bei 0-5° 1,06 ml Triäthylamin in 10 ml Tetrahydrofuran. Nach 1 Stunde bei Raumtemperatur kühlt man wieder auf 5° und tropft bei dieser Temperatur weitere 1,06 ml Triäthylamin in 10 ml Tetrahydrofuran und 1,28 g (7.7 mMol) Glykolsäurebenzylester in 20 ml Tetrahydrofuran zu. Nach 1.5 Stunden bei Raumtemperatur kühlt man wieder auf 0° und tropft 10 ml Wasser und eine Lösung von 2,03 ml Triäthylamin in 10 ml Tetrahydrofuran zu.

Nach weiteren 2 Stunden bei Raumtemperatur dampft man die Lösung im Vakuum ein, nimmt den Rückstand mit Chloroform auf und schüttelt mehrmals mit gesättigter Natriumchloridlösung durch. Nach Trocknen der Chloroform-Phase über Natriumsulfat und Eindampfen erhält man das Rohprodukt als Mononatriumsalz, das durch Chromatographie an Kieselgel (Merck) in Methylenchlorid:Methanol = 9:1 gereinigt wird. Man erhält das Diastereomerengemisch des 2-(1,2-Dipalmitoyl-rac-glycero-3-hydroxyphosphoryloxy)-essigsäure-benzylesters als Mononatriumsalz mit dem Schmp. 65-68°. Durch eine zweite Chromatographie gelingt die Isolierung einer Teilmenge mit identischem $R_f$-Wert (0,3, Methylenchlorid: Methanol = 9:1) und identischer Elementaranalyse, jedoch dem Smp. 160-165°. Für die weiteren Umsetzungen wurde das

1:1-Diastereomerengemisch eingesetzt.

Beispiel 17: Zu einer Lösung von 0,322 g (1,0 mMol) Phosphorsäurehexadecylester in 10 ml absolutem Pyridin gibt man 0,227 g (1,1 mMol) Dicyclohexylcarbodiimid und rührt 1 Stunde bei Raumtemperatur unter Argon. Anschliessend werden 0,673 g (1,0 mMol) N-Acetyl-1α-O-benzyl-muramyl-L-alanyl-D-isoglutamin-γ-benzylester, gelöst in 10 ml eines Gemisches von Dimethylacetamid:Pyridin = 1:1, und 0,061 g (0,5 mMol) 4-Dimethylaminopyridin zugegeben und weitere 18 Stunden bei Raumtemperatur unter Argon gerührt. Danach wird die so erhaltene Suspension bei 30° im Hochvakuum eingedampft.
Der feste, weisse Rückstand wird in 50 ml Methanol suspendiert, von einem Teil nicht umgesetztem γ-Benzylesterderivat abfiltriert und erneut eingedampft.

Der so erhaltene Rückstand wird nun in 50 ml Chloroform gelöst und diese Lösung mit 25 ml 10proz. Natriumchloridlösung ausgeschüttelt. Die Chloroformphase wird über Natriumsulfat getrocknet, filtriert und erneut eingedampft.

Das so erhaltene Rohprodukt aus N-Acetyl-1α-O-benzyl-6-O-hexadecyloxy-hydroxyphosphoryl-muramyl-L-alanyl-D-isoglutamin-γ-benzylester-mono-natriumsalz und 1α-O-Benzyl-N-acetyl-4,6-O-di-hexadecyloxyhydroxy-phosphoryl-muramyl-L-alanyl-D-isoglutamin-γ-benzylester-dinatriumsalz wird durch Säulenchromatographie an Kieselgel 60 (1:50; 0,063-0,002 mm, Merck) im System Chloroform:Methanol =9:1 von den Verunreinigungen befreit und dann mit Chloroform:Methanol =6:1 die gewünschten Produkte eluiert. Die entsprechenden Fraktionen werden gesammelt und im Hochvakuum eingedampft.

Man erhält N-Acetyl-1α-O-benzyl-4,6-O-di-hexadecyloxyhydroxyphosphoryl-muramyl-L-alanyl-D-isoglutamin-γ-benzylester-dinatriumsalz mit $R_f$ = 0,52 (Chloroform:Methanol:Wasser = 70:30:5) und N-Acetyl-1α-O-benzyl-6-O-hexadecyloxyhydroxyphosphoryl-muramyl-L-alanyl-D-isoglutamin-γ-benzylester-mononatriumsalz mit $R_f$ = 0,71 (Chloroform: Methanol: Wasser = 70:30:5), beide in Form eines amorphen, farblosen Pulvers.

0,25 g (0,25 mMol) N-Acetyl-1α-O-benzyl-6-O-hexadecyloxyhydroxyphosphoryl-muramyl-L-alanyl-D-isoglutamin-γ-benzylester-mononatriumsalz werden in 25 ml Chloroform : 1,2-Dimethoxyäthan:Wasser=1:10:1 in Gegenwart von insgesamt 0,25 g 10proz. Palladium-auf-Kohlenstoff 60 Stunden bei Raumtemperatur und Normaldruck hydriert. Danach filtriert man vom Katalysator ab und dampft das Filtrat bei 40° im Hochvakuum zur Trockne. Der so erhaltene, feste weisse Rückstand wird in 50 ml bidestilliertem Wasser gelöst und diese Lösung durch ein PTFE (Polytetrafluoräthylen, Teflon)-Milliporefilter (0,2 µ) filtriert und lyophilisiert.

Man erhält 0,2 g N-Acetyl-6-O-hexadecyloxy-hydroxyphosphoryl-muramyl-L-alanyl-D-isoglutamin-mononatriumsalz als amorphes, farbloses Pulver mit $R_f$ = 0,7 (Chloroform:Methanol:Wasser = 5:5:1).

0,4 g (0,31 mMol) N-Acetyl-1α-O-benzyl-4,6-O-dihexadecyloxyhydroxyphosphoryl-muramyl-L-alanyl-D-isoglutamin-γ-benzylester-dinatriumsalz werden in 40 ml Chloroform:1,2-Dimethoxyäthan:Wasser = 1:10:1 in Gegenwart von insgesamt 0,4 g 10proz. Palladium-auf-Kohlenstoff 60 Stunden bei Raumtemperatur und Normaldruck hydriert. Danach filtriert man vom Katalysator ab und dampft das Filtrat bei 40° im Hochvakuum zur Trockne. Der so erhaltene feste weisse Rückstand wird in 60 ml bidestilliertem Wasser gelöst und diese Lösung durch ein PTFE-Milliporefilter (0,2 µ) filtriert und lyophilisiert. Man erhält 0,3 g

N-Acetyl-4,6-O-di-hexadecyloxyhydroxyphosphoryl-muramyl-L-alanyl-D-isoglutamin-dinatriumsalz als amorphes, farbloses Pulver mit $R_f$ = 0,48 (Chloroform:Methanol:Wasser = 5:5:1).

Beispiel 18: Zu einer Lösung von 0,644 g (2,0 mMol) Phosphorsäurehexadecylester in 20 ml absolutem Pyridin gibt man 0,454 g (2,2 mMol) Dicyclohexylcarbodiimid und rührt 1 Stunde bei Raumtemperatur unter Argon.
Anschliessend werden 0,673 g (1,0 mMol) N-Acetyl-1α-O-benzyl-muramyl-L-alanyl-D-isoglutamin-γ-benzylester, gelöst in 10 ml eines Gemisches von Dimethylacetamid:Pyridin=1:1, und 0,061 g (0,5 mMol) 4-Dimethylaminopyridin zugegeben und weitere 22 Stunden bei Raumtemperatur unter Argon gerührt. Danach wird die so erhaltene trübe Lösung bei 30° im Hochvakuum eingedampft.
Der Rückstand wird in 100 ml Chloroform aufgenommen, vom Unlöslichen abfiltriert, und die Chloroformlösung zweimal mit je 25 ml 10proz. Natriumchloridlösung ausgeschüttelt. Die Chloroformphase wird über Natriumsulfat getrocknet, filtriert und erneut eingedampft.

Das so erhaltene rohe N-Acetyl-1α-O-benzyl-4,6-O-di-hexadecycloxy-hydroxyphosphoryl-muramyl-L-alanyl-D-isoglutamin-γ-benzylester-dinatriumsalz wird von wenig N-Acetyl-1α-O-benzyl-6-O-hexadecyloxy-hydroxyphosphoryl-muramyl-L-alanyl-D-isoglutamin-γ-benzylester-mononatriumsalz, Ausgangsstoffen und Verunreinigungen durch Säulenchromatographie an Kieselgel 60 (1:50; 0,063 - 0,002 mm , Merck) gereinigt.
Zuerst werden mit Chloroform:Methanol=9:1 die Verunreinigungen, dann wird mit Chloroform:Methanol = 6:1 das gewünschte Produkt eluiert. Die entsprechenden Fraktionen werden gesammelt und im Hochvakuum eingedampft.
Man erhält 0,97 g N-Acetyl-1α-O-benzyl-4,6-O-di-hexadecyloxyhydroxy-phosphoryl-muramyl-L-alanyl-D-isoglutamin-γ-benzylester-dinatriumsalz als amorphes, farbloses Pulver mit $R_f$ = 0,52 (Chloroform:Methanol:Wasser =70:30:5), aus dem die Schutzgruppen durch katalytische

Hydrierung analog Beispiel 17 abgespalten werden.

Beispiel 19: Zu einer Lösung von 0,5 g (0,38 mMol) N-Acetyl-muramyl-L-alanyl-D-isoglutaminyl-L-alanyl-2-(1',2'-dipalmitoyl-sn-glycero-3'-hydroxyphosphoryloxy)-äthylamid-natriumsalz, das 3 Mol Wasser enthält, in 10 ml absolutem Pyridin gibt man 5 ml (50 mMol) Essigsäureanhydrid (purissimum) und rührt das Ganze 24 Stunden bei Raumtemperatur. Anschliessend wird die so erhaltene Lösung bei 40° im Hochvakuum eingedampft und dann der so gewonnene weisse Rückstand mehrmals in 50 ml Wasser suspendiert und lyophilisiert. Das schneeweisse Lyophilisat wird in 100 ml bidestilliertem Wasser gelöst und diese Lösung zuerst über Hyflo, dann durch ein PTFE (Polytetrafluoräthylen)-Milliporefilter (0,2 µ) filtriert und erneut lyophilisiert.

Man erhält N-Acetyl-1,4,6-O-triacetyl-muramyl-L-alanyl-D-isoglutaminyl-L-alanyl-2-(1',2'-dipalmitoyl-sn-glycero-3'-hydroxyphosphoryloxy)-äthylamid-natriumsalz in Form eines schneeweissen Pulvers, das noch 3 Mol Wasser enthält, mit $[\alpha]_D^{20} = +17,6 \pm 1°$ (c = 0,165; Wasser) und $R_f$ = 0,40 (Chloroform:Methanol:Wasser =65:25:4).

Beispiel 20: 0,176 g N-Acetyl-1α-O-benzyl-6-O-{N-[2-(1',2'-dipalmitoyl-sn-glycero-3'-hydroxyphosphoryloxy)-äthyl]-succinamoyl -muramyl-L-α-aminobutyryl-D-isoglutamin-γ-benzylester, gelöst in 160 ml Dimethoxyäthan:Wasser =4:1,werden in Gegenwart von 0,2 g Palladium-auf-Kohle (10 %ig) während 46 Stunden mit Wasserstoff behandelt. Der Katalysator wird abfiltriert, das Filtrat zur Trockne eingedampft und aus tert.Butanol:Wasser = 4:1 gefriergetrocknet. Das Rohprodukt wird durch Dickschichtchromatographie auf Kieselgel (fünf 20 x 20 cm PSC-Fertigplatten, Kieselgel 60, F 254, Stärke: 2 mm, Merck) aufgetrennt. Die aufgrund der UV-Fluoreszenz identifizierte, das Produkt enthaltende Schicht wird mit Chloroform: tert. Butanol = 1:1 extrahiert und nach Filtration (PTFE-Millipore, 0,2 μ) aus tert. Butanol:Wasser = 20:1 gefriergetrocknet. Nach kontinuierlicher Diafiltration (Amicon-Filter YM 10, nominelle Trenngrenze 10 000 dalton) gegen ein 1:1-Gemisch von 0,1molarem Phosphatpuffer pH = 7 und 0.1molarer Natriumchloridlösung sowie anschliessender Lyophilisation erhält man N-Acetyl-6-O-{N-[2-(1',2'-dipalmitoyl-sn-glycero-3'-hydroxyphosphoryloxy)-äthyl]-succinamoyl}-muramyl-L-α-aminobutyryl-D-isoglutamin, das teilweise in Natriumsalzform vorliegt, als farbloses Pulver mit $[\alpha]_D^{20} = +11 \pm 1°$ (c= 0,253; Dimethylformamid), $R_f$ = 0,38 (Chloroform:Methanol:Wasser = 70:30:5) und $R_f$ = 0,72 (Chloroform:Methanol:Essigsäure:Wasser = 25:15:4:2).

Das Ausgangsmaterial erhält man wie folgt:
0,234 g (0,3 mMol) N-Acetyl-1α-O-benzyl-6-O-succinoyl-muramyl-L-α-aminobutyryl-D-isoglutamin-γ-benzylester und 0,092 g (0,36 mMol) Di-(N-succinimidyl)-carbonat (Fluka puriss.) werden in 10 ml abs. Dimethylformamid gelöst und unter Ausschluss von Feuchtigkeit nach Zugabe von 0,1 ml (1,2 mMol) abs. Pyridin während drei Stunden bei 35° gehalten. Nun gibt man 0,208 g (0,30 mMol) 2-(1',2'-Dipalmitoyl-sn-glycero-3'-hydroxyphosphoryloxy)-äthylamin (=Kephalin), gelöst in 3 ml Chloroform: Methanol =7:3, zu und lässt das Ganze unter Rühren während sechs Stunden bei 35° reagieren. Zur Aufarbeitung wird die dünne Suspension eingedampft. Das Rohprodukt wird auf einer OPTI-UPC$_{12}$-Säule (mit Dodecyl-trichlorsilan behandeltes Kieselgel, 80 g, 4fach belegt, 40 - 63 μm, ANTEC) nacheinander mit Wasser, wässerigem Acetonitril und Acetonitril gewaschen. Schliesslich wird das Produkt mit Methanol:Wasser = 9:1 eluiert. Nach Eindampfen und

Lyophilisation aus tert. Butanol erhält man N-Acetyl-1α-O-benzyl-6-O-{N-[2-(1',2'-dipalmitoyl-sn-glycero-3'-hydroxyphosphoryloxy)-äthyl]-succinamoyl}-muramyl-L-α-aminobutyryl-D-isoglutamin-γ-benzylester als lockeres Pulver mit $[\alpha]_D^{20} = +8 \pm 1°$ (c=0,435; Chloroform) und $R_f$ = 0,27 (Chloroform:Methanol=7:3).

Der Ausgangsstoff wird wie folgt hergestellt:

2,00 g (3,1 mMol) N-Acetyl-1α-O-benzyl-4,6-O-isopropyliden-muramyl-L-α-aminobutyryl-D-isoglutamin-γ-benzylester, gelöst in 30 ml abs. Pyridin, werden in der Kälte mit 0,80 g (7,85 mMol) Bernsteinsäureanhydrid in 15 ml abs. Essigsäureäthylester versetzt und während drei Tagen bei Raumtemperatur stehen gelassen. Die klare Lösung wird auf 0° abgekühlt, 50 ml 0,5normale Zitronensäurelösung zugetropft und die Reaktionslösung während zwei Stunden gerührt. Man engt auf etwa 5 ml ein, setzt 40 ml tert. Butanol zu und lyophilisiert. Die weitere Reinigung erfolgt an einer $UPC_{12}$-Säule (80 g), analog wie vorher beschrieben, beginnend mit einem Gemisch aus Acetonitril: Wasser = 1:3 (20 ml Fraktionen). Das Produkt wird mit Acetonitril: Wasser = 3:1 eluiert, die vereinigten Fraktionen stark eingeengt und aus tert. Butanol:Wasser = 4:1 lyophilisiert.

Man erhält N-Acetyl-1α-O-benzyl-6-O-succinoyl-muramyl-L-α-amino-butyryl-D-isoglutamin-γ-benzylester als farbloses, lockeres Pulver mit $[\alpha]_D^{20} = +36 \pm 1°$ (c=0,618; Dimethylformamid), $R_f$= 0,45 (Chloroform:Methanol:Wasser = 70:30:5) und $R_f$ = 0,68 (Essigsäureäthylester:Essigsäure:Wasser:Methanol = 67:10:23:12).

Das verwendete Ausgangsmaterial erhält man auf folgende Weise:

2,80 g (4,4 mMol) N-Acetyl-1α-O-benzyl-4,6-O-isopropyliden-muramyl-L-α-aminobutyryl-D-isoglutamin-γ-benzylester, gelöst in 30 ml 60%iger Essigsäure, werden während 24 Stunden bei Raumtemperatur stehengelassen. Die Reaktionslösung wird am Rotationsverdampfer bei

30° auf ca.5 ml eingeengt und nach Zugabe von 35 ml tert. Butanol lyophilisiert.

Man erhält 2,1 g N-Acetyl-1α-O-benzyl-muramyl-L-α-aminobutyryl-D-isoglutamin-γ-benzylester mit $[\alpha]_D^{20} = +44 \pm 1°$ (c = 0,635; Methanol),

$R_f$ = 0,62 (Acetonitril:Wasser = 3:1) und

$R_f$ = 0,72 (Essigsäureäthylester: n-Butanol:Pyridin:Essigsäure: Wasser =42:21:21:6:10).

Beispiel 21: 0,20 g (0,21 mMol) N-Acetyl-1α-O-benzyl-normuramyl-L-O-{N-[2-tetradecyloxy- hydroxyphosphoryloxy)-äthyl]-succinamoyl}-seryl-D-isoglutamin-γ-tert.butylester, gelöst in 50 ml Dimethoxyäthan:Wasser = 10:1 werden nach Zugabe von 0,2 g eines Palladium-auf-Kohle-Katalysators (10%ig) wie üblich hydriert. Der Katalysator wird abfiltriert und das Filtrat zur Trockene eingedampft. Der Rückstand wird analog wie unten beschrieben durch präparative Schichtchromatographie gereinigt. Zur Abspaltung der tert.Butylester-Schutzgruppe wird der Rückstand in der Kälte in 2 ml 95%iger Trifluoressigsäure gelöst. Nach einstündigem Stehen bei Raumtemperatur wird die Trifluoressigsäure bei Raumtemperatur abgedampft und der Rückstand zweimal aus tert.Butanol:Wasser=10:1 gefriergetrocknet. Nach kontinuierlicher Diafiltration (Amicon-Filter YM 10) der 1%igen Lösung gegen ein 1:1-Gemisch von 0,1molarem Phosphatpuffer pH=7 und 0,1molarer Natriumchloridlösung sowie anschliessender Lyophilisation erhält man N-Acetyl-normuramyl-L-O-{N-[2-tetradecycloxy-hydroxy-phosphoryloxy)-äthyl]-succinamoyl}-seryl-D-isoglutamin, das teilweise in Natriumsalzform vorliegt,mit

$R_f$ = 0,05 (Chloroform:Methanol:Wasser = 70:30:5) und

$R_f$ = 0,11 (Essigsäureäthylester: n-Butanol:Pyridin: Essigsäure:Wasser=42:21:21:6:10).

Das Ausgangsmaterial erhält man wie folgt:

0,370 g (0,5 mMol) N-Acetyl-1α-O-benzyl-normuramyl-L-O-(succinoyl)-seryl-D-isoglutamin-γ-tert.butylester und 0,115 g (1mMol) N-Hydroxysuccinimid, beide über Phosphorpentoxyd im Hochvakuum getrocknet, werden in einem Gemisch aus 2ml abs. Dimethylacetamid (getrocknet über Molekularsieb der Firma Merck,3Å) und 1,5 ml abs. Chloroform gelöst. Man kühlt auf 0° ab, gibt 0,124 g (0,6 mMol) Dicyclohexylcarbodiimid zu und rührt das Ganze während 4 Stunden bei Raumtemperatur. Der abgeschiedene Dicyclohexylharnstoff kann durch Zentrifugation entfernt werden. Nun werden 0,111 g (0,3 mMol) 2-(Tetradecycloxy-hydroxyphosphoryloxy)-äthylamin, gelöst in einem Gemisch aus 6 ml Chloroform, 1,5 ml Isopropanol und 0,3 ml Wasser hinzugefügt und unter gutem Rühren bei Raumtemperatur während zwei Stunden 0,084 ml (0,396 mMol) Triäthylamin in 0,5 ml Chloroform eingetropft, wobei teilweise ungelöste Aminkomponente langsam in Lösung geht. Nach zwei weiteren Stunden wird die klare Lösung im Hochvakuum bei 30° eingedampft.

Der ölige Rückstand wird in wenig Chloroform:Methanol=2:1 aufgenommen und durch Dickschichtchromatographie auf Kieselgel (fünf 20 x 20 cm PSC-Fertigplatten, Kieselgel 60, F 254, Stärke 2 mm, Merck) im System Chloroform:Methanol:Wasser=70:30:5 (15 cm) aufgetrennt.

Die das Phospholipid-Derivat enthaltende Schicht (Detektion am Rand der Platte mit Hilfe von Phosspray® 3-3047, Supelco, CH-1299 Crans) wird mit Dimethylacetamid:Chloroform=1:1 dreimal extrahiert. Die Lösung wird bei 30° im Vakuum eingedampft und der Rückstand aus tert. Butanol:Wasser=9:1 lyophilisiert. Man erhält N-Acetyl-1α-O-benzyl-normuramyl-L-O-{N-[2-(tetradecycloxy-hydroxy-phosphoryloxy)-äthyl)-succinamoyl)-seryl-D-isoglutamin-γ-tert.butylester als farbloses Pulver mit

$R_f$ = 0,26 (Chloroform:Methanol:Wasser=70:30:5) und

$R_f$ = 0,45 (Essigsäureäthylester:n-Butanol:Pyridin:Esssigsäure: Wasser=42:21:21:6:10).

Den Ausgangsstoff erhält man folgendermassen:

6,25 g (9,2 mMol) N-Acetyl-1α-O-benzyl-4,6-O-isopropyliden-normuramyl-L-seryl-D-isoglutamin-γ-tert.butylester werden in 40 ml abs. Pyridin gelöst, in der Kälte mit 3,00 g (30 mMol) Bernsteinsäureanhydrid versetzt und während 48 Stunden bei Raumtemperatur stehengelassen. Man kühlt auf 0° ab und gibt zur Zersetzung des überschüssigen Anhydrids 20 ml Methanol hinzu. Nach zweistündigem Stehen im Eisbad werden Methanol und Pyridin im Vakuum bei 30° entfernt. Der ölige Rückstand, gelöst in 40 ml Acetonitril:Wasser = 1:3, wird auf eine $UPC_{12}$ Säule (200 g; $UPC_{12}$ ist mit Dodecyl-trichlorsilan behandeltes Kieselgel, 40-63 µm, Warenzeichen der Firma Opti) vom gleichen Lösungsmittelgemisch gegeben. Mit Acetonitril: Wasser = 1:1 (20 ml Fraktionen) werden erst die sauren Verunreinigungen, dann das Produkt eluiert. Das in den Fraktionen 8-25 enthaltene Material wird gesammelt. Das nach dem Verdampfen der Lösungsmittel verbleibende kristalline Material zeigt einen kleineren $R_f$-Wert als ursprünglich und erweist sich als die Des-isopropyliden-Verbindung, N-Acetyl-1α-O-benzyl-normuramyl-L-O-(succinoyl)-seryl-D-isoglutamin-γ-tert.butylester, mit

$[\alpha]_D^{20} = +72\pm1°$ (c = 0,811; Methanol),

$R_f$ = 0,60 (Chloroform:Methanol:Wasser=70:30:5) und

$R_f$ = 0,61 (Acetonitril:Wasser=3:1).

Das Endprodukt der obengenannten Reaktionsfolge, N-Acetyl-normuramyl-L-O-{N-[2-tetradecyloxy-hydroxyphosphoryloxy)-äthyl]-succinamoyl}-seryl-D-isoglutamin, das teilweise in Natriumsalzform vorliegt, kann auch ausgehend von dem oben beschriebenen N-Acetyl-1α-O-benzyl-normuramyl-L-O-(succinoyl)-seryl-D-isoglutamin-γ-tert.butylester erhalten werden, indem man, wie nachfolgend beschrieben, zunächst die Benzylschutzgruppe abspaltet, das erhaltene Produkt analog wie oben beschrieben mit N-Hydroxy-succinimid /Dicyclohexylcarbodiimid / 2-(Tetradecyloxy—hydroxyphosphoryloxy)-äthylamin umsetzt, und aus dem erhaltenen Produkt die tert.Butylschutzgruppe analog wie oben beschrieben sauer abspaltet:

2,00 g N-Acetyl-1α-O-benzyl-normuramyl-L-O-(succinoyl)-seryl-D-iso-glutamin-γ-tert.butylester, gelöst in 100 ml Dimethoxyäthan:Wasser = 20:1, werden nach Zugabe von 1 g eines Palladium-auf-Kohle-Katalysators (10%ig) während 20 Stunden mit Wasserstoff behandelt. Der Katalysator wird abfiltriert, das Filtrat zur Trockne eingedampft und der Rückstand nach Lösen in Wasser gefriergetrocknet. Man erhält N-Acetyl-normuramyl-L-O-(succinoyl)-seryl-D-isoglutamin-γ-tert.butylester als amorphes Pulver mit $[\alpha]_D^{20} = +20° \pm 1°$ (c = 0,199 Wasser),
$R_f$ = 0,15 (Chloroform:Methanol:Wasser = 70:30:5) und
$R_f$ = 0,45 (Essigsäureäthylester:n-Butanol:Pyridin:Essigsäure:Wasser = 42:21:21:6:10).

Beispiel 22: 0,110 g (0,12 mMol) Bernsteinsäure-2-(1'-palmitoyl-2'-oleoyl-sn-glycero-3'-hydroxyphosphoryloxy)-äthylamid und 0,028 g (0,24 mMol) N-Hydroxysuccinimid werden in einem Gemisch aus 1,2 ml abs. Dimethylacetamid (getrocknet über Molekularsieb Merck, 3 Å) und 0,4 ml abs. Chloroform gelöst. Man fügt 0,030 g (0,144 mMol) Dicyclohexyl-carbodiimid zu und rührt das Ganze während drei Stunden bei Raumtemperatur. Dann werden 0,054 g (0,08 mMol) N-Acetyl-muramyl-L-O-(L-phenylalanyl)-seryl-D-isoglutamin hinzugegeben und während zwei Stunden 0,028 ml (0,16 mMol) Triäthylamin, gelöst in 0,4 ml Chloroform, eingetropft. Nach weiteren zwei Stunden wird die Suspension im Vakuum bei 30° eingedampft, in wenig Chloroform- Methanol-Gemisch aufgenommen und durch Dickschichtchromatographie auf Kieselgel (drei 20x20 cm PSC-Fertigplatten, Kieselgel 60, F 254, Stärke 2 mm, Merck) im System Chloroform:Methanol:Wasser = 70:30:5 (15 cm Laufstrecke) aufgetrennt. Aus der gewünschten Schicht erhält man nach dreimaliger Elution mit Dimethylacetamid:Chloroform = 1:1 und Dialyse des Rückstandes nacheinander gegen 0,1molaren Natriumphosphatpuffer pH 7 und Wasser N-Acetyl-muramyl-L-O-{[N-(1'-palmitoyl-2'-oleoyl-sn-glycero-3'-hydroxyphosphoryloxy)-äthyl]-succinamoyl-L-phenylalanyl}-seryl-D-isoglutamin, das teilweise in Natriumsalzform vorliegt, als farbloses Pulver mit $R_f$ = 0,27 (Chloroform:Methanol:Wasser = 70:30:5).

Die Ausgangsstoffe erhält man wie folgt:

2-(1'-Palmitoyl-2'-oleoyl-sn-glycero-3'-hydroxyphosphoryloxy)-äthylamin wird analog, wie für Kephalin im Beispiel 15 beschrieben, mit Bernsteinsäureanhydrid in Pyridin in Bernsteinsäure-2-(1'-palmitoyl-2'-oleoyl-sn-glycero-3'-hydroxyphosphoryloxy)-äthylamid mit $R_f$ = 0.35 (Chloroform: Methanol:Wasser=70:30:3) überführt.

Das benötigte Muramylpeptidderivat kann folgendermassen gewonnen werden:

0,40 g (0,36 mMol) N-Acetyl-1α-O-benzyl-4,6-O-isopropyliden-muramyl-L-O-{[2-(p-biphenylyl)-isopropyloxycarbonyl]-L-phenylalanyl}-seryl-D-isoglutamin-γ-benzylester werden in 6,4 ml Eisessig gelöst, 1,6 ml Wasser hinzugefügt und während 24 Stunden bei Raumtemperatur stehen gelassen. Die klare Lösung wird mit 8 ml Eisessig verdünnt, lyophilisiert, der stark hygroskopische Rückstand in tert.Butanol aufgenommen und wieder lyophilisiert. Der Rückstand wird zur Entfernung von 2-(p-Biphenylyl)-isopropanol mehrfach mit Petroläther verrieben und letzterer abdekantiert.
Der Rückstand [$R_f$ = 0,55 (Chloroform:Methanol:Wasser=70:30:5)] wird schliesslich in 20 ml Eisessig gelöst und nach Zugabe von 0,2 g eines Palladium-auf-Kohle-Katalysators während 28 Stunden mit Wasserstoff behandelt. Der Katalysator wird abfiltriert und die Essigsäure durch zweimalige Gefriertrocknung entfernt. Man erhält N-Acetyl-muramyl-L-O-(L-phenylalanyl)-seryl-D-isoglutamin als farbloses, stark hygroskopisches Pulver mit
$R_f$ = 0,11 (Chloroform:Methanol:Wasser=70:30:5),
$R_f$ = 0,26 (Acetonitril:Wasser=3:1) und
$R_f$ = 0,24 (Essigsäureäthylester:n-Butanol:Pyridin:Essigsäure:Wasser =42:21:21:6:10).

Das verwendete Ausgangsmaterial erhält man wie folgt:

1,895 g (2,6 mMol) N-Acetyl-1α-O-benzyl-4,6-O-isopropyliden-muramyl-L-seryl-D-isoglutamin-γ-benzylester, 1,05 g (2,6 mMol) 2-(p-Biphenylyl)-isopropyloxycarbonyl-L-phenylalanin, 0,703 g (5,2 mMol) N-Hydroxybenztriazol-monohydrat und 0,635 g (5,2 mMol) 4-Dimethylaminopyridin werden in einem Gemisch aus 4,5 ml abs. Dimethylformamid und 1,5 ml Acetonitril gelöst. Man kühlt im Eisbad ab und gibt 0,70 g (3,4 mMol) Dicyclohexylcarbodiimid hinzu. Es wird 24 Stunden bei 0 bis 5° und weitere 24 Stunden bei Raumtemperatur gerührt. Die dicke Suspension wird mit 50 ml Essigsäureäthylester verdünnt, das Ungelöste abgesaugt und das Filtrat zur Trockne eingedampft. Der Rückstand wird in 200 ml Essigsäureäthylester aufgenommen, mehrfach mit je 10 ml Wasser extrahiert, die organische Phase getrocknet und das Lösungsmittel verdampft. Der verbleibende ölige Rückstand (3,63 g) wird schliesslich an 200 g Silikagel (Merck AG, 0,06-0,2 mm Ø) im System Chloroform:Methanol= 98:2 (6 ml Fraktionen) gereinigt.
Das in den Fraktionen 65-215 enthaltene Material wird gesammelt. Man erhält 2,3 g (80 % d.Th.) N-Acetyl-1α-O-benzyl-4,6-O-isopropyliden-muramyl-L-O-{[2-(p-biphenylyl)-isopropyl-oxycarbonyl]-L-phenyl-alanyl}-seryl-D-isoglutamin-γ-benzylester als farblosen Schaum mit $[\alpha]_D^{20} = + 62 \pm 1°$ (c=0,892; Chloroform),
$R_f$ = 0,45 (Chloroform: Isopropanol:Essigsäure=70:8:2) und
$R_f$ = 0,85 (Acetonitril:Wasser=3:1).

Das Ausgangsmaterial wird wie folgt hergestellt:

8,01 g (16,1 mMol) N-Acetyl-1α-O-benzyl-4,6-O-isopropyliden-muraminsäure-natriumsalz,5,80 g (16,1 mMol) L-Seryl-D-isoglutamin-γ-benzylester-hydrochlorid und 2,78 g (24,2 mMol) N-Hydroxysuccinimid werden in 60 ml abs. Dimethylformamid gelöst und in der Kälte mit 3,99 g

(19.3 mMol) Dicyclohexylcarbodiimid versetzt. Nach 16stündigem Rühren bei Raumtemperatur wird die Suspension mit 100 ml Essigsäureäthylester versetzt, 30 Minuten im Eisbad gerührt und das Unlösliche (Dicyclohexylharnstoff, Natriumchlorid) abfiltriert. Das Filtrat wird mit 400 ml Essigsäureäthylester verdünnt, die Lösung 10mal mit je 100 ml Wasser extrahiert, über Natriumsulfat getrocknet und eingedampft. Der schwach gelbliche Rückstand wird mit einer Mischung aus 150 ml Essigsäureäthylester, 30 ml Methanol und 20 ml Aether digeriert, wobei Kristallisation eintritt. Die Abscheidung wird durch portionenweise Zugabe von total 300 ml Aether und durch Kühlung vervollständigt. Nach dem Abfiltrieren und Trocknen verbleiben 8,4 g (71,5 % d.Th.) N-Acetyl-1α-O-benzyl-4,6-O-isopropyliden-muramyl-L-seryl-D-isoglutamin-γ-benzylester mit Smp. 178-179°, $[\alpha]_D^{20} = +96 \pm 1°$ (c = 0,695; Methanol), $R_f$ = 0,77 (Chloroform:Methanol:Wasser = 70:30:5) und $R_f$ = 0,14 (Chloroform: Isopropanol:Essigsäure=70:8:2).

Beispiel 23: In analoger Weise zu Beispiel 4, ausgehend vom N-Hydroxy-succinimidester des Bernsteinsäure-2-(1',2'-dipalmitoyl-2'-oleoyl-sn-glyero-3'-hydroxyphosphoryloxy)-äthylamids und N-Acetyl-muramyl-L-O-(L-phenylalanyl)-seryl-D-isoglutamin-γ-diphenylmethyl-ester-hydrochlorid, erhält man nach Spaltung des Diphenylmethylesters N-Acetyl-muramyl-L-O-{N-[2-(1',2'-dipalmitoyl-sn-glycero-3'-hydroxyphosphoryloxy)-äthyl]-succinamoyl-L-phenylalanyl}-seryl-D-isoglutamin in Form von oder im Gemisch mit seinem Natriumsalz.

Beispiel 24: Zu einer Lösung von 0,387 g (1,2 mMol) Phosphorsäurehexadecylester in 20 ml absolutem Pyridin gibt man 0,269 g (1,3 mMol) Dicyclohexylcarbodiimid und rührt 1 Stunde bei Raumtemperatur unter Argon.

Anschliessend werden 0,583 g (0,8 mMol) N-Acetyl-1-α-O-benzyl-4,6-O-isopropyliden-muramyl-L-seryl-D-isoglutamin-γ-benzylester, gelöst in

20 ml absolutem Pyridin, und 0,098 g (0,8 mMol) 4-Dimethylamino-pyridin zugegeben und weitere 65 Stunden bei Raumtemperatur unter Argon gerührt.
Danach wird die so erhaltene Suspension bei 40° im Hochvakuum eingedampft. Der Rückstand wird in 100 ml Chloroform aufgenommen, vom Unlöslichen abfiltriert, und die Chloroformphase mit insgesamt 50 ml 10%iger Natriumchloridlösung ausgeschüttelt. Die Chloroformphase wird über Natriumsulfat getrocknet, filtriert und erneut eingedampft.
Das so erhaltene Rohprodukt wird durch Dickschichtchromatographie an Kieselgel (PF 254, Firma Merck) im System Chloroform:Methanol:Wasser = 70:30:5 gereinigt. Die gewünschte Fraktion wird mit Chloroform: Methanol = 1:1 vom Träger gelöst und die so erhaltene Lösung bei 30° im Hochvakuum eingedampft. Der feste, weisse Rückstand wird in 50 ml Chloroform aufgenommen, vom Unlöslichen abfiltriert und erneut eingedampft. Man erhält N-Acetyl-1-α-O-benzyl-4,6-O-isopropyliden-muramyl-L-O-(hexadecyloxy-hydroxyphosphoryl)-seryl-D-isoglutamin-γ-benzylester-mononatriumsalz als farbloses, amorphes Pulver mit $R_f$ = 0,49 (Chloroform:Methanol:Wasser = 70:30:5).

Aus dem erhaltenen Produkt wird die Schutzgruppe wie folgt abgespalten:

0,4 g (0,38 mMol) N-Acetyl-1-α-O-benzyl-4,6-O-isopropyliden-muramyl-L-O-(hexadecyloxy-hydroxyphosphoryl)-seryl-D-isoglutamin-γ-benzylester-mononatriumsalz werden in 8 ml Chloroform:1,2-Dimethoxyäthan: Wasser = 1:10:1 in Gegenwart von insgesamt 0,4 g 10%igem Palladium-auf-Kohlenstoff 90 Stunden bei Raumtemperatur und Normaldruck hydriert. Danach filtriert man vom Katalysator ab und dampft das Filtrat bei 40° im Hochvakuum zur Trockne ein.
Der so erhaltene feste, weisse Rückstand wird zur vollständigen Abspaltung der Isopropylidengruppe noch 12 Stunden in 10 ml 50%iger Essigsäure stehengelassen und dann erneut im Hochvakuum eingedampft.

Der Rückstand wird in 50 ml bidestilliertem Wasser gelöst und diese Lösung durch ein PTFE-Milliporefilter (0,2 u) filtriert und lyophilisiert.
Man erhält 0,25 g N-Acetyl-muramyl-L-O-(hexadecyloxy-hydroxyphosphoryl)-seryl-D-isoglutamin-mononatriumsalz als amorphes, farbloses Pulver mit
$R_f$ = 0,45 (Chloroform:Methanol:Wasser = 5:5:1).

In analoger Weise erhält man
N-Acetyl-muramyl-L-O-(1,2-dipalmitoyl-3-sn-glycero-hydroxyphosphoryl)-seryl-D-isoglutamin-mononatriumsalz.

Patentansprüche (für alle benannten Länder ausser Oesterreich)

1. Hexapyranoseverbindungen der Formel I, worin

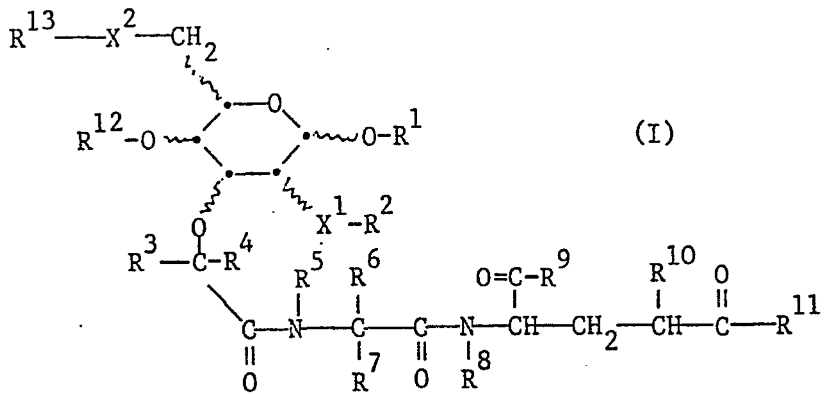

(I)

$X^1$ und $X^2$ unabhängig voneinander eine Gruppe der Formel -O- oder $-N(R^{14})-$, wobei $R^{14}$ für Wasserstoff oder Niederalkyl steht, $R^1$, $R^2$, $R^{12}$ und $R^{13}$ unabhängig voneinander einen Rest der Formel Ia,

$$-(Z^1-Y^1-X^3)_n-A^1 \qquad \text{(Ia)}$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der Formel -O- oder $-N(R^{14})-$, worin $R^{14}$ die oben gegebene Bedeutung hat, und $A^1$ für einen Rest der Formel Ib,

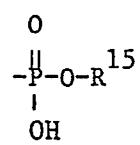

(Ib)

worin $R^{15}$ einen mindestens 7 Kohlenstoffatome aufweisenden aliphatischen oder cycloaliphatischen Rest darstellt, oder für eine Gruppe der Formel Ic,

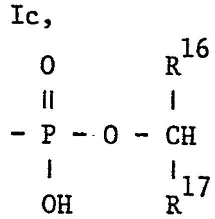

(Ic)

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder einen unter physiologischen Bedingungen abspaltbaren Rest,

$R^3$, $R^4$, $R^5$, $R^7$ und $R^8$ unabhängig voneinander Wasserstoff oder Niederalkyl,

$R^6$ Wasserstoff oder Niederalkyl, das unsubstituiert oder durch eine Gruppe der Formel Id,

$$-E-(Z^2-Y^2-X^4)_m-A^2 \qquad \text{(Id)}$$

worin m für 0 oder 1, E für eine Gruppe der Formel -O-, -S- oder $-N(R^{14})-$, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel -O- oder $-N(R^{14})-$, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder

$R^5$ und $R^6$ zusammen unsubstituiertes oder substituiertes 1,3- oder

1,4-Niederalkylen,

$R^9$ und $R^{11}$ unabhängig voneinander einen Rest der Formel Ie,

$$-X^5-Y^3-X^6-A^3 \qquad (Ie)$$

worin $X^5$ für eine Gruppe der Formel -O-, -S- oder $-N(R^{14})-$ und $X^6$ für eine Gruppe der Formel -O- oder $-N(R^{14})-$, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und

$R^{10}$ Wasserstoff oder freies, verestertes oder amidiertes Carboxy bedeuten, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ von Acyl, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies oder veräthertes Hydroxy oder Mercapto, durch von der Gruppe der Formel Id verschiedenes verestertes Hydroxy oder Mercapto, durch freies oder von der Gruppe der Formel Id verschiedenes substituiertes Amino, oder durch Cycloalkyl, carbocyclisches Aryl oder stickstoffhaltiges Heteroaryl mit 5 bis 6 Ringgliedern im heterocyclischen Ring substituiert ist, oder $R^7$ von Wasserstoff verschieden ist, und Salze von solchen Verbindungen.

2. Verbindungen der Formel I nach Anspruch 1, worin $R^1$ für den Rest der Formel Ia gemäss Anspruch 1 steht, $R^2$, $R^{12}$ und $R^{13}$ unabhängig voneinander Wasserstoff, vom Rest der Formel Ia, worin n für 1 steht, verschiedenes Acyl oder eine unter physiologischen Bedingungen abspaltbare Gruppe bedeuten, $R^5$ Wasserstoff oder Niederalkyl bedeutet, $R^6$ für Wasserstoff, unsubstituiertes oder durch freies oder veräthertes Hydroxy oder Mercapto, von einer Gruppe der Formel Id gemäss Anspruch 1 verschiedenes, verestertes Hydroxy oder Mercapto, freies oder von einer entsprechenden Gruppe der Formel Id verschiedenes, substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie gemäss Anspruch 1 verschiedenes, veräthertes Hydroxy oder Mercapto oder freies oder von einem Rest der Formel Ie verschiedenes, substituiertes Amino bedeuten, wobei freie funktionelle Gruppen in geschützter Form vorliegen können, und Salze von solchen Verbindungen.

3. Verbindungen der Formel I nach Anspruch 1, worin $R^2$ für den Rest der Formel Ia gemäss Anspruch 1 steht, $R^1$, $R^{12}$ und $R^{13}$ unabhängig voneinander Wasserstoff, vom Rest der Formel Ia, worin n für 1 steht, verschiedenes Acyl oder eine unter physiologischen Bedingungen abspaltbare Gruppe darstellen, $R^5$ Wasserstoff oder Niederalkyl bedeutet, $R^6$ für Wasserstoff, unsubstituiertes oder durch freies oder veräthertes Hydroxy oder Mercapto, von einer entsprechenden Gruppe der Formel Id gemäss Anspruch 1 verschiedenes, verestertes Hydroxy oder Mercapto, freies oder von einer Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von

einem Rest der Formel Ie gemäss Anspruch 1 verschiedenes, veräthertes Hydroxy oder Mercapto oder freies oder von einem Rest der Formel Ie verschiedenes substituiertes Amino bedeuten, wobei freie funktionelle Gruppen in geschützter Form vorliegen können, und Salze von solchen Verbindungen.

4. Verbindungen der Formel I nach Anspruch 1, worin $R^{13}$ für den Rest der Formel Ia gemäss Anspruch 1 steht, $R^1$, $R^2$ und $R^{12}$ unabhängig voneinander Wasserstoff, vom Rest der Formel Ia, worin n für 1 steht, verschiedenes Acyl oder eine unter physiologischen Bedingungen abspaltbare Gruppe darstellen, $R^5$ Wasserstoff oder Niederalkyl bedeutet, $R^6$ für Wasserstoff, unsubstituiertes oder durch freies oder veräthertes Hydroxy oder Mercapto, von einer Gruppe der Formel Id gemäss Anspruch 1 verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie gemäss Anspruch 1 verschiedenes veräthertes Hydroxy oder Mercapto oder freies oder von einem Rest der Formel Ie verschiedenes substituiertes Amino bedeuten, wobei freie funktionelle Gruppen in geschützter Form vorliegen können, und Salze von solchen Verbindungen.

5. Verbindungen der Formel I nach Anspruch 1, worin $R^1$, $R^2$, $R^{12}$ und $R^{13}$ unabhängig voneinander Wasserstoff, vom Rest der Formel Ia gemäss Anspruch 1, worin n für 1 steht, verschiedenes Acyl oder eine unter physiologischen Bedingungen abspaltbare Gruppe darstellen, $R^6$ für durch einen Rest der Formel Id oder Ida substituiertes Niederalkyl steht,

$$-\overset{O}{\overset{\|}{C}} - E - (Y^2 - X^4)_m - A^2 \qquad \text{(Ida)}$$

worin m, E, $Z^2$, $Y^2$, $X^4$ und $A^2$ die in Anspruch 1 gegebenen Bedeutungen haben, und $R^9$ und $R^{11}$ unabhängig voneinander freies Hydroxy oder Mercapto, von einem Rest der Formel Ie gemäss Anspruch 1 verschiedenes veräthertes Hydroxy oder Mercapto, freies oder von einem Rest der Formel Ie verschiedenes substituiertes Amino bedeuten, wobei freie funktionelle Gruppen in geschützter Form vorliegen können, und Salze von solchen Verbindungen.

6. Verbindungen der Formel I nach Anspruch 1, worin $R^1$, $R^2$, $R^{12}$ und $R^{13}$ unabhängig voneinander für Wasserstoff, den Acylrest einer aliphatischen, cycloaliphatischen, aromatischen oder araliphatischen Carbonsäure mit bis zu 90 Kohlenstoffatomen, Tri-niederalkyl-silyl oder einen Rest der Formel Ia stehen, worin n und $X^3$ die in Anspruch 1 gegebenen Bedeutungen haben, $Z^1$ für Carbonyl steht, $Y^1$ Niederalkylen bedeutet, das durch Iminocarbonyl oder Oxycarbonyl unterbrochen sein kann, und $A^1$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder mit einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, $R^6$ Wasserstoff oder Niederalkyl bedeutet, das unsubstituiert oder durch einen Rest der Formel Id substituiert ist, worin m, E und $X^4$ die oben gegebenen Bedeutungen haben, $Z^2$ für Carbonyl steht, $Y^2$ Niederalkylen bedeutet, das durch Iminocarbonyl oder Oxycarbonyl unterbrochen sein kann, und $A^2$ für einen Rest der Formel Ib oder Ic, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Acylrest verestertes Hydroxymethyl bedeuten, oder $R^6$ Niederalkyl bedeutet, das durch Hydroxy oder Mercapto, durch mit einem bis zu 90 Kohlenstoffatome enthaltenden aliphatischen Rest veräthertes Hydroxy oder Mercapto, durch mit einem bis zu 90 Kohlenstoffatome aufweisenden aliphatischen Acylrest verestertes Hydroxy oder Mercapto, der von der Gruppe der Formel Id verschieden ist, durch Amino, durch mit einem bis zu 90 Kohlenstoffatome enthaltenden Acylrest substituiertes Amino, der von einem Rest der Formel Id verschieden ist, durch freies Carboxy, Niederalkoxycarbonyl, Carbamoyl, Niederalkylaminocarbonyl, Carboxyniederalkylaminocarbonyl oder amidiertes Carboxyl der Formel Ida gemäss Anspruch 5, unsubstituiertes oder durch Hydroxy, Niederalkoxy oder Halogen substituiertes Phenyl oder durch Imidazolyl oder Indolyl substituiert ist, oder $R^5$ und $R^6$ zusammen 1,3- oder 1,4-Niederalkylen bedeuten, $R^9$ und $R^{11}$ unabhängig voneinander Hydroxy, Niederalkoxy, Amino, Niederalkylamino, Carboxyniederalkylamino, oder einen Rest der Formel Ie bedeuten, worin $X^5$ und $X^6$ die oben gegebenen Bedeutungen haben, $Y^3$ Niederalkylen bedeutet, das durch Iminocarbonyl oder Oxycarbonyl unterbrochen sein kann, und $A^3$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen

Acylrest verestertes Hydroxymethyl bedeuten, und $R^{10}$ für Wasserstoff, Carboxy, Niederalkoxycarbonyl, Carbamoyl, Niederalkylaminocarbonyl oder Carboxyniederalkylaminocarbonyl steht, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ vom Acylrest einer aliphatischen, cycloaliphatischen, aromatischen oder araliphatischen Carbonsäure mit bis zu 90 Kohlenstoffatomen, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies Hydroxy oder Mercapto, durch mit einem bis zu 90 Kohlenstoffatome enthaltenden aliphatischen Rest veräthertes Hydroxy oder Mercapto, durch von der Gruppe der Formel Id verschiedenes, verestertes Hydroxy oder Mercapto, durch freies oder mit einem bis zu 90 Kohlenstoffatome enthaltenden Acylrest substituiertes und von der Gruppe der Formel Id verschiedenes Amino, durch freies Carboxy, Niederalkylaminocarbonyl, unsubstituiertes oder durch Hydroxy, Niederalkoxy oder Halogen substituiertes Phenyl, oder durch Imidazolyl oder Indolyl substituiert ist, oder $R^7$ von Wasserstoff verschieden ist, und Salze von solchen Verbindungen.

7. Verbindungen der Formel I nach Anspruch 6, worin $R^1$, $R^2$, $R^{12}$ und $R^{13}$ unabhängig voneinander für Wasserstoff, den Acylrest einer unsubstituierten oder durch Hydroxy, Amino und/oder Alkanoylamino substituierten Alkancarbonsäure mit bis zu 90 Kohlenstoffatomen, Tri-niederalkyl-silyl oder einen Rest der Formel Ia gemäss Anspruch 6 stehen.

8. Verbindungen der Formel I, nach Anspruch 6, worin sich der Zuckerteil von der D-Glucose ableitet,
$X^1$ für die Gruppe NH und
$X^2$ für Sauerstoff stehen,
$R^1$ Wasserstoff, Niederalkanoyl oder die Gruppe der Formel Ia bedeutet, worin n für 1 steht, $Z^1$ Carbonyl bedeutet, $Y^1$ Niederalkylen darstellt, das durch Iminocarbonyl unterbrochen sein kann, $X^3$ für eine Gruppe der Formel -O- oder -NH- steht, und $A^1$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden Alkylrest, der unsubstituiert oder durch Hydroxy, Amino und/oder Alkanoylamino mit bis zu 30 Kohlenstoffatomen substituiert ist, veräthert oder mit einem 7 bis 90 Kohlenstoffatome aufweisenden, unsubstituierten oder durch Hydroxy, Amino- und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R^2$ Niederalkanoyl, Hydroxyniederalkanoyl, Benzoyl oder die Gruppe der Formel Ia bedeutet, worin n, $Z^1$, $Y^1$, $X^3$ und $A^1$ die oben in diesem Anspruch gegebenen Bedeutungen haben, $R^{12}$ Wasserstoff oder Niederalkanoyl darstellt, $R^{13}$ Wasserstoff, Alkanoyl oder Hydroxyalkanoyl mit bis zu 90 Kohlenstoffatomen,

Alkanoylaminoalkanoyl mit bis zu 30 Kohlenstoffatomen oder die Gruppe der Formel Ia bedeutet, worin n, $Z^1$, $Y^1$, $X^3$ und $A^1$ die oben in diesem Anspruch gegebenen Bedeutungen haben, $R^3$ und $R^7$ für Wasserstoff oder Methyl stehen, $R^4$, $R^5$, $R^8$ und $R^{10}$ für Wasserstoff stehen, $R^6$ Wasserstoff, unsubstituiertes oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl, Indolyl oder durch eine Gruppe der Formel Id substituiertes Niederalkyl bedeutet, worin m für 1 steht, E für eine Gruppe der Formel -O- oder -S- steht, $Z^2$ Carbonyl bedeutet, $Y^2$ Niederalkylen darstellt, das durch Iminocarbonyl unterbrochen sein kann, $X^4$ für eine Gruppe der Formel -O- steht, und $A^2$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem, 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, und jeder der Reste $R^9$ und $R^{11}$ unabhängig voneinander für Hydroxy, Niederalkoxy, Amino, Niederalkylamino, Carboxyniederalkylamino oder einen Rest der Formel Ie stehen, worin $X^5$ für eine

Gruppe der Formel -O- oder -NH- steht, $Y^3$ Niederalkylen darstellt, das durch Iminocarbonyl unterbrochen sein kann, $X^6$ für eine Gruppe der Formel -O- steht und $A^3$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R^9$ für Amino, Niederalkylamino oder Carboxyniederalkylamino und $R^{11}$ für Hydroxy stehen, mit der Massgabe, dass die Verbindungen mindestens einen Rest aus der Gruppe $A^1$, $A^2$ und $A^3$ aufweisen, und mit der weiteren Massgabe, dass in Verbindungen der Formel I, worin mindestens einer der Reste $R^9$ oder $R^{11}$ für die Gruppe der Formel Ie steht, $R^1$ von Wasserstoff, oder $R^2$ von Niederalkanoyl, Hydroxyniederalkanoyl oder Benzoyl, oder $R^{12}$ von Wasserstoff, oder $R^{13}$ von Wasserstoff, oder $R^6$ von Wasserstoff, unsubstituiertem oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl oder Indolyl substituiertem Niederalkyl, oder $R^7$ von Wasserstoff verschieden ist, und Salze dieser Verbindungen.

9. Verbindungen der Formel I nach Anspruch 6, worin
$X^1$ eine Gruppe der Formel $-N(R^{14})-$, wobei
$R^{14}$ für Wasserstoff oder $C_{1-4}$-Alkyl steht,
$X_2$ Sauerstoff,
$R^1$ Wasserstoff, Niederalkanoyl oder die Gruppe der Formel Ia, worin n für 0 oder 1, $Z^1$ für Carbonyl, $Y^1$ für Niederalkylen, das durch Iminocarbonyl unterbrochen sein kann, $X^3$ für Sauerstoff und $A^1$ für einen Rest der Formel Ib oder Ic stehen, worin $R^{15}$ für einen 7 - 30 Kohlenstoffatome aufweisenden unsubstituierten oder durch Hydroxy, Amino und/oder Alkanoylamino mit bis zu 22 Kohlenstoffatomen substituierten Alkyl- oder Alkenylrest, $R^{16}$ für Wasserstoff und $R^{17}$ für 2-Hydroxy- oder 1,2-Dihydroxyäthyl, wobei in einem Rest $R^{17}$ mindestens eine Hydroxygruppe mit einem 7 - 30 Kohlenstoffatome aufweisenden Alkyl- oder Alkenylrest veräthert oder mit einem 7 - 30 Kohlenstoffatome aufweisenden Alkanoyl- oder Alkenoylrest verestert ist, oder $R^{16}$ und $R^{17}$ unabhängig voneinander für Hydroxymethyl, wobei die Hydroxygruppe mit einem 7 - 30 Kohlenstoffatome aufweisenden Alkyl- oder Alkenylrest veräthert oder mit einem 7-30 Kohlenstoffatome aufweisenden Alkanoyl- oder Alkenoylrest verestert ist, stehen,
$R^2$ Niederalkanoyl, Hydroxyniederalkanoyl, Benzoyl oder unabhängig von $R^1$, $R^{12}$ und $R^{13}$ eine wie vorstehend in diesem Anspruch definierte Gruppe der Formel Ia,
$R^3$, $R^4$, $R^5$, $R^7$ und $R^8$ unabhängig voneinander Wasserstoff, Methyl oder Aethyl,
$R^6$ Wasserstoff oder unsubstituiertes oder durch freies Hydroxy, freies Mercapto, Niederalkoxy, Niederalkylthio, Alkanoyloxy mit 2 - 30 Kohlenstoffatomen, Alkenoyloxy mit 6 - 30 Kohlenstoffatomen, Phenyl, 4-Hydroxy-phenyl oder durch eine Gruppe der Formel Id substituiertes Niederalkyl, worin m für 0 oder 1, E für Sauerstoff oder Schwefel, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der wie vorstehend in diesem Anspruch definierten Formeln Ib oder Ic stehen,
$R^9$ und $R^{11}$ unabhängig voneinander Hydroxy, Niederalkoxy, Amino, Niederalkylamino, Carboxyniederalkylamino, Niederalkoxycarbonylniederalkylamino, Carbamoylniederalkylamino oder einen Rest der Formel Ie, worin $X^5$ für Sauerstoff oder NH, $Y^3$ für Niederalkylen, das durch Iminocarbonyl unterbrochen sein kann, $X^6$ für Sauerstoff und $A^3$ unabhängig von $A^1$ und $A^2$ für einen Rest der wie vorstehend in diesem Anspruch definierten Formeln Ib oder Ic stehen, $R^{10}$ Wasserstoff,
$R^{12}$ Wasserstoff, Niederalkanoyl oder den gleichen Rest wie $R^{13}$, und
$R^{13}$ Wasserstoff, Alkanoyl oder Alkenoyl mit jeweils bis zu 30 Kohlenstoffatomen oder unabhängig von $R^1$ und $R^2$ einen wie vorstehend in diesem Anspruch definierten Rest der Formel Ia bedeuten, mit der Massgabe, dass die Verbindungen mindestens einen und höchstens zwei Reste aus der Gruppe $A^1$, $A^2$ und $A^3$ aufweisen und mit der weiteren Massgabe, dass in denjenigen Verbindungen, in denen mindestens ein Rest aus der Gruppe $R^9$ und $R^{11}$ für die Gruppe der Formel Ie steht, $R^1$ von Wasserstoff, oder $R^2$ von Niederalkanoyl, Hydroxyniederalkanoyl oder Benzoyl, oder $R^4$ von Wasserstoff, oder $R^6$ von Wasserstoff oder von unsubstituiertem oder durch freies Hydroxy, freies Mercapto, Niederalkoxy, Niederalkylthio, Alkanoyloxy, Alkenoyloxy, Phenyl oder 4-Hydroxy-phenyl substituiertem Niederalkyl, oder $R^7$, $R^{12}$ oder $R^{13}$ von Wasserstoff verschieden ist, und Salze dieser Verbindungen.

10. Verbindungen der Formel I nach Anspruch 9, worin
$X^1$ die Gruppe NH,
$X^2$ Sauerstoff,
$R^1$ Wasserstoff oder Niederalkanoyl,
$R^2$ Niederalkanoyl, Benzoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen Alkylrest mit 7 bis 22 C-Atomen, $R^{16}$ für Wasserstoff und $R^{17}$ für 1,2-Dihydroxyäthyl stehen, wobei beide Hydroxygruppen unabhängig voneinander jeweils mit einem 10 - 22 Kohlenstoffatome aufweisenden Alkanoyl- oder Alkenoylrest verestert sind,
$R^3$ Wasserstoff oder Methyl,
$R^4$, $R^5$, $R^7$ und $R^8$ Wasserstoff,
$R^6$ Wasserstoff oder unsubstituiertes oder durch freies Hydroxy, Alkanoyloxy mit 2 bis 22 Kohlenstoffatomen, Alkenoyloxy mit 6 - 22 Kohlenstoffatomen, Phenyl oder durch eine Gruppe der Formel Id substituiertes Niederalkyl, worin m für 0 oder 1, E für Sauerstoff, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der vorstehend definierten Formeln Ib und Ic stehen, $R^9$ und $R^{11}$ unabhängig voneinander Hydroxy, Niederalkoxy, Amino, Niederalkylamino, α-Carboxy-niederalkylamino, α-Niederalkoxycarbonyl-niederalkylamino, α-Carbamoyl-niederalkylamino oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhänig von $A^1$ und $A^2$ für einen Rest der vorstehend definierten Formeln Ib oder Ic stehen,
$R^{10}$ Wasserstoff,
$R^{12}$ Wasserstoff, Niederalkanoyl oder den gleichen Rest wie $R^{13}$ und
$R^{13}$ Wasserstoff, Alkanoyl mit 2 bis 22 Kohlenstoffatomen, Alkenoyl mit 6 bis 22 Kohlenstoffatomen oder unabhängig von $R^2$ einen wie vorstehend definierten Rest der Formel Ia bedeuten, und Salze dieser Verbindungen.

11. Verbindungen der Formel I nach Anspruch 10, worin
$X^1$ die Gruppe NH,
$X^2$ Sauerstoff,
$R^1$ Wasserstoff oder $C_{2-4}$-Alkanoyl,
$R^2$ $C_{2-4}$-Alkanoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und

$A^1$ für einen Rest der Formel Ib oder Ic stehen, worin $R^{15}$ für einen unverzweigten Alkylrest mit 12 bis 18 C-Atomen, $R^{16}$ für Wasserstoff und $R^{17}$ für 1,2-Dipalmitoyloxy-äthyl oder 2-Oleoyloxy-1-palmitoyloxy-äthyl stehen,
$R^3$ Wasserstoff oder Methyl,
$R^4$, $R^5$, $R^7$, $R^8$ und $R^{10}$ Wasserstoff,
$R^6$ unsubstituiertes oder durch einen Rest der Formel Id substituiertes Methyl, Aethyl oder Isopropyl, worin m für 0 oder 1, E für Sauerstoff, $Z^2$ für Carbonyl, $Y^2$ für unsubstituiertes oder durch Phenyl substituiertes Niederalkylen, das durch ein oder zwei Iminocarbonylgruppen unterbrochen sein kann, $X^4$ für Sauerstoff und $A^2$ unabhängig von $A^1$ und $A^3$ für einen Rest der vorstehend definierten Formeln Ib oder Ic stehen,
$R^9$ Amino,
$R^{11}$ Hydroxy oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhängig von $A^1$ und $A^2$ für einen Rest der vorstehend definierten Formeln Ib oder Ic stehen,
$R^{12}$ Wasserstoff, Acetyl oder den gleichen Rest wie $R^{13}$ und
$R^{13}$ Wasserstoff, Acetyl oder, unabhängig von $R^2$, einen wie vorstehend definierten Rest der Formel Ia bedeuten, und Salze dieser Verbindungen.

12. Verbindungen der Formel I nach einem der Ansprüche 1 bis 11, worin die Reste $A^1$, $A^2$ und $A^3$ für einen Rest der Formel Ic stehen, und ihre Salze.

13. Verbindungen der Formel I nach einem der Ansprüche 1 bis 7 und 9 bis 12, worin sich der Zuckerteil von (D)-Galactose oder (D)-Mannose ableitet, und ihre Salze.

14. Verbindungen der Formel I nach einem der Ansprüche 1 bis 7 und 9 bis 12, worin sich der Zuckerteil von (D)-Glucose ableitet, und ihre Salze.

15. Verbindungen der Formel I, nach einem der Ansprüche 1 bis 14, die an einem asymmetrisch substituierten C(-$R^3$)-Atom die (D)-Konfiguration, an einem asymmetrisch substituierten C(-$R^6$)-Atom die (L)-Konfiguration und am C(-N-$R^8$)-Atom die (D)-Konfiguration aufweisen, und ihre Salze.

16. Verbindungen der Formel I nach einem der Ansprüche 1 bis 14, die an einem asymmetrisch substituierten C(-$R^3$)-Atom die (L)-Konfiguration, an einem asymmetrisch substituierten C(-$R^6$)-Atom die (L)-Konfiguration und am C(-N-$R^8$)-Atom die (D)-Konfiguration aufweisen, und ihre Salze.

17. Verbindungen der Formel I nach einem der Ansprüche 1 bis 16, die einen Rest der Formel Ia, worin n für 1 steht, oder einen Rest der Formel Id, worin m für 1 steht, enthalten, und ihre Salze.

18. Verbindungen der Formel I nach einem der Ansprüche 1 bis 16, die einen Rest der Formel Ia, worin n für 0 steht, oder einen Rest der Formel Id, worin m für 0 steht, enthalten, und ihre Salze.

19. Verbindungen der Formel I nach einem der Ansprüche 1 bis 18, die nur einen Phosphorylsubstituenten tragen, und zwar im Rest $R^{13}$, und ihre Salze.

20. Verbindungen der Formel I nach einem der Ansprüche 1 bis 18, die nur einen Phosphorylsubstituenten tragen, und zwar im Rest $R^6$, und ihre Salze.

21. Verbindungen der Formel I nach einem der Ansprüche 1 bis 18, die nur einen Phosphorylsubstituenten tragen, und zwar im Rest $R^{11}$, und ihre Salze.

22. Die in den Beispielen 15 bis 24 genannten Verbindungen der Formel I gemäss Anspruch 1.

23. Pharmazeutisch verwendbare Salze von Verbindungen der Formel I nach einem der Ansprüche 1 bis 22.

24. Natriumsalze von Verbindungen der Formel I nach Anspruch 23.

25. Hexapyranoseverbindungen der Formel I und pharmazeutisch verwendbare Salze davon nach einem der Ansprüche 1 bis 24 zur Behandlung von Warmblütern.

26. Hexapyranoseverbindungen der Formel I und pharmazeutisch verwendbare Salze davon nach einem der Ansprüche 1 bis 24 als Immunstimulantien.

27. Verwendung von Hexapyranoseverbindungen der Formel I und von pharmazeutisch verwendbaren Salzen nach einem der Ansprüche 1 bis 24 zur Herstellung von pharmazeutischen Präparaten.

28. Pharmazeutische Präparate, die mindestens eine Verbindung der Formel I und/oder mindestens ein pharmazeutisch verwendbares Salz einer solchen Verbindung gemäss einem der Ansprüche 1-24 zusammen mit einem pharmazeutisch verwendbaren Trägermaterial enthalten.

29. Verwendung einer Verbindung der Formel I oder von einem pharmazeutisch verwendbaren Salz dieser Verbindung gemäss einem der Ansprüche 1-24 als Immunomodulatoren bei Warmblütern einschliesslich des Menschen.

30. Pharmazeutische Präparate, die mindestens eine Verbindung der Formel I und/oder mindestens ein pharmazeutisch verwendbares Salz einer solchen Verbindung gemäss einem der Ansprüche 1-24 und mindestens ein Antibiotikum zusammen mit einem pharmazeutisch verwendbaren Trägermaterial enthalten.

31. Verwendung einer Verbindung der Formel I oder von einem pharmazeutisch verwendbaren Salz dieser Verbindung gemäss einem der Ansprüche 1-24 zusammen mit einem Antibiotikum zur Steigerung der antibiotischen Aktivität des Antibiotikums.

32. Verfahren zur Herstellung von Verbindungen der Formel I und von ihren Salzen gemäss einem der Ansprüche 1-24, dadurch gekennzeichnet, dass man

a) eine Verbindung der Formel II,

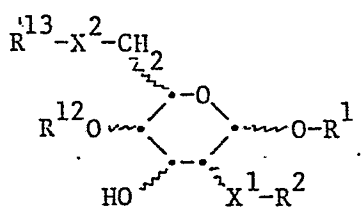

(II)

worin gegebenenfalls vorhandene funktionelle Gruppen, mit Ausnahme der freien Hydroxygruppe in 4-Stellung des Pyranoseteils, in geschützer Form vorliegen können, oder eine Metallverbindung davon mit einer Verbindung der Formel III,

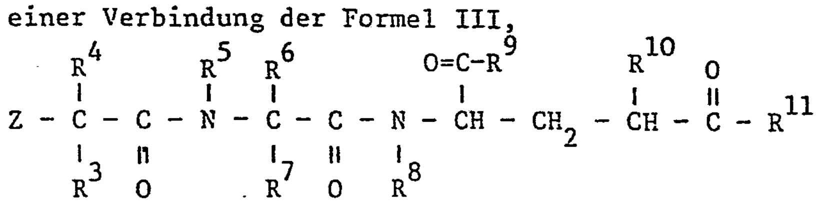

(III)

worin Z eine reaktionsfähige veresterte Hydroxygruppe darstellt und worin gegebenenfalls vorhandene funktionelle Gruppen durch Schutzgruppen geschützt sein können, umsetzt und gegebenenfalls vorhandene Schutzgruppen abspaltet, oder

b) eine Verbindung der Formel IV,

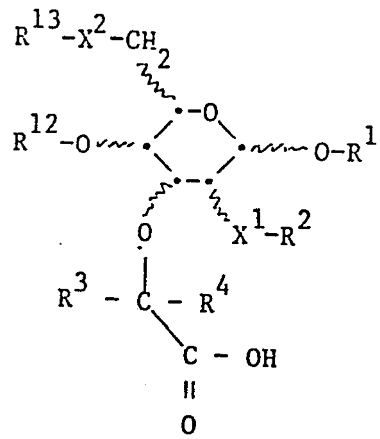

(IV)

worin gegebenenfalls vorhandene funktionelle Gruppen mit Ausnahme der freien Carboxylgruppe, in geschützter Form vorliegen können, oder ein reaktionsfähiges Carboxylderivat davon mit einer Verbindung der Formel V,

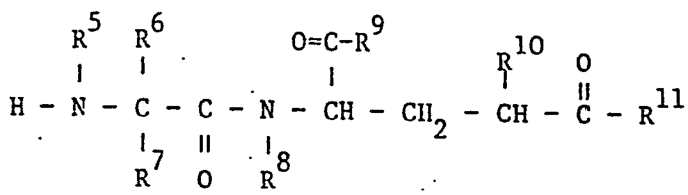

(V)

worin gegebenenfalls vorhandene freie funktionelle Gruppen, mit Ausnahme der Aminogruppe der Formel $HN(R^5)-$, in geschützter Form vorliegen können, oder einem reaktionsfähigen Derivat davon umsetzt und gegebenenfalls vorhandene Schutzgruppen abspaltet, oder

c) eine Verbindung der Formel VI,

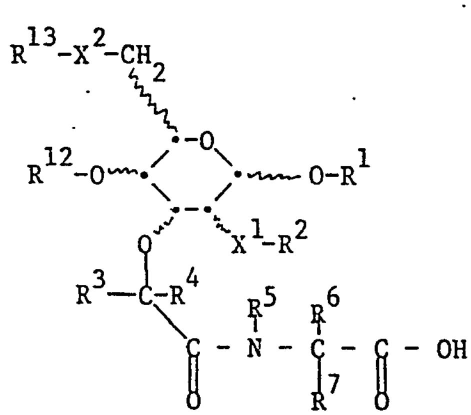

(VI)

worin gegebenenfalls vorhandene freie funktionelle Gruppen, mit Ausnahme der freien Carboxylgruppe, in geschützter Form vorliegen können, oder ein reaktionsfähiges Carboxylderivat davon mit einer Verbindung der Formel VII,

$$H-\underset{R^{8}}{N}-\overset{O=C-R^{9}}{\overset{|}{CH}}-CH_2-\overset{R^{10}}{\overset{|}{CH}}-\overset{O}{\overset{\|}{C}}-R^{11} \qquad (VII)$$

worin gegebenenfalls vorhandene freie funktionelle Gruppen, mit Ausnahme der Aminogruppe der Formel $HN(R^8)-$, in geschützter Form vorliegen, oder einem reaktionsfähigen Derivat davon umsetzt, und gegebenenfalls vorhandene Schutzgruppen abspaltet, oder

d) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht und $Z^1$, $Y^1$, $X^3$ und $A^1$ die oben angegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder von ihren Salzen eine Verbindung der Formel I, worin mindestens eine der Gruppen $R^1$, $R^2$, $R^{12}$ und $R^{13}$ für Wasserstoff steht und die anderen unabhängig voneinander für Wasserstoff, vom Rest der Formel Ia verschiedenes Acyl, eine unter physiologischen Bedingungen abspaltbare Gruppe oder einen Rest der Formel Ia stehen, und worin die übrigen Substituenten, $R^3$, $R^4$, $R^5$, $R^6$, $R^7$, $R^8$, $R^9$, $R^{10}$, $R^{11}$, $X^1$ und $X^2$, die oben gegebenen Bedeutungen haben, wobei gegebenenfalls vorhandene freie funktionelle Gruppen, mit Ausnahme der an der Reaktion teilnehmenden Gruppe(n), in geschützter Form vorliegen können, oder ein reaktionsfähiges Derivat davon, mit einer Säure der Formel $HO - Z^1 - Y^1 - X^3 - A^1$ (X), worin $Z^1$, $Y^1$, $X^3$ und $A^1$ die oben gegebenen Bedeutungen haben, wobei gegebenenfalls vorhandene freie funktionelle Gruppen, mit Ausnahme der Säuregruppe der Formel $HO-Z^1-$, in geschützter Form vorliegen können, oder einem reaktionsfähigen Säurederivat davon umsetzt und in einer erhaltenen Verbindung der Formel I gegebenenfalls vorhandene Schutzgruppen abspaltet, oder

e) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, $Y^1$ für unsubstituiertes oder substituiertes Alkylen steht, das durch Iminocarbonyl oder Oxycarbonyl unterbrochen ist, und $Z^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder von ihren Salzen eine Verbindung der Formel I, worin mindestens eine der Gruppen $R^1$, $R^2$, $R^{12}$ und $R^{13}$ für einen Rest der Formel $-Z^1-Y^1_a-R_x$ (Iaa) steht, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel $R_y-Y^1_b-X^3-A^1$ (XI) umsetzt, wobei $Z^1$, $X^3$ und $A^1$ die oben gegebenen Bedeutungen haben, und $Y^1_a$ und $Y^1_b$ unabhängig voneinander unsubstituiertes oder substituiertes Alkylen bedeuten, und worin eine der Gruppen $R_x$ und $R_y$ für freies oder in reaktionsfähiger, derivatisierter Form vorliegendes Hydroxy oder Amino der Formel $-NH(R^{14})$ steht, und die andere für freies oder in reaktionsfähiger, derivatisierter Form vorliegendes Carboxy steht, während die übrigen Substituenten $R^1$, $R^2$, $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion teilnehmenden funktionellen Gruppen, in geschützter Form vorliegen können, und in einer erhaltenen Verbindung der Formel I vorhandene Schutzgruppen abspaltet, oder

f) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben angegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der

Formel Ia bedeuten, oder von ihren Salzen eine Verbindung der Formel I, worin mindestens eine der Gruppen $R^1$, $R^2$, $R^{12}$ und $R^{13}$ für einen Rest der Formel $-Z^1-Y^1-X^3-H$ (Iab) steht, worin $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, oder ein reaktionsfähiges Derivat davon mit einer Verbindung der Formel XII oder XIII,

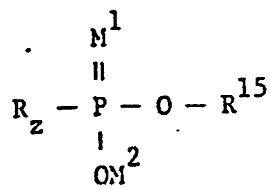

(XII)

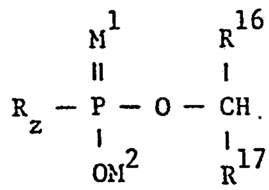

(XIII)

worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig, in Gegenwart eines oxidierenden Mittels, umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

g) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, und ihren Salzen eine Verbindung der Formel I, worin mindestens eine der Gruppen $R^1$, $R^2$, $R^{12}$ und $R^{13}$ für einen Rest der Formel

$$-Z^1-Y^1-X^3-\underset{\underset{OM^2}{|}}{\overset{\overset{M^1}{\|}}{P}}-R_z \qquad \text{(Iac)}$$

steht, worin $R_z$ gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel XIV oder XV,

$$HO-R^{15} \qquad\qquad HO-\underset{\underset{R^{17}}{|}}{\overset{\overset{R^{16}}{|}}{CH}}$$

(XIV) (XV)

worin $R^{15}$, $R^{16}$ und $R^{17}$ die oben gegebenen Bedeutungen haben, oder einem Derivat davon, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

h) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 0 steht, und $A^1$ die obengenannte Bedeutung hat, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten,

und ihren Salzen eine Verbindung der Formel I, worin mindestens eine der Gruppen $R^1$, $R^2$, $R^{12}$ und $R^{13}$ für Wasserstoff steht, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder ein Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, $M^2$ Wasserstoff oder eine abspaltbare Gruppe bedeutet und $R^{15}$, $R^{16}$ und $R^{17}$ die oben gegebenen Bedeutungen haben, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

i) zur Herstellung von solchen Verbindungen der Formel I, worin $R^6$ Niederalkyl bedeutet, das durch den Rest der Formel Id, worin m für 1 steht, und die Gruppen E, $Z^2$, $Y^2$, $X^4$ und $A^2$ die oben gegebenen Bedeutungen haben, substituiert ist, und ihren Salzen eine Verbindung der Formel I, worin $R^6$ für Niederalkyl steht, das durch die Gruppe der Formel -E-H (Idb), worin E die oben gegebene Bedeutung hat, substituiert ist, oder ein reaktionsfähiges Derivat davon mit einer Säure der Formel $HO-Z^2-Y^2-X^4-A^2$ (XVI), worin $Z^2$, $Y^2$, $X^4$ und $A^2$ die oben gegebenen Bedeutungen haben, oder einem reaktionsfähigen Derivat davon umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

j) zur Herstellung von solchen Verbindungen der Formel I, worin $R^6$ für Niederalkyl steht, das durch den Rest der Formel Id, wobei m für 1 steht, $Y^2$ unsubstituiertes oder substituiertes Alkylen bedeutet, das durch Iminocarbonyl oder Oxycarbonyl unterbrochen ist, und die Gruppen E, $Z^2$, $X^4$ und $A^2$ die oben gegebenen Bedeutungen haben, substituiert ist, und ihren Salzen eine Verbindung der Formel I, worin $R^6$ für Niederalkyl steht, das durch die Gruppe $-E-Z^2-Y^2_a-R_x$ (Idc) substituiert ist, mit einer Verbindung der Formel $R_y-Y^2_b-X^4-A^2$ (XVII) umsetzt, worin $Y^2_a$ und $Y^2_b$ unabhängig voneinander unsubstituiertes oder substituiertes Alkylen bedeuten, und worin eine der Gruppen $R_x$ und $R_y$ für freies oder in reaktionsfähiger derivatisierter Form vorliegendes Hydroxy oder Amino der Formel $-NH(R^{14})$ steht, und die andere für freies oder in reaktionsfähiger, derivatisierter Form vorliegendes Carboxy steht, und E, $Z^2$, $X^4$ und $A^2$ die obigen Bedeutungen haben, während die übrigen Substituenten der Ausgangsstoffe $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

k) zur Herstellung von solchen Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die oben gegebenen Bedeutungen haben, und ihren Salzen eine Verbindung der Formel I, worin $R^6$ Niederalkyl bedeutet, das durch eine Gruppe der Formel $- E - Z^2 - Y^2 - X^4 - H$ (Idd) substituiert ist, worin E, $Z^2$, $Y^2$ und $X^4$ die oben gegebenen Bedeutungen haben, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmende Gruppen, in geschützter Form vorliegen können, und in einer erhaltenen Verbindung der Formel I vorhandene Schutzgruppen abspaltet, oder

l) zur Herstellung von solchen Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die obengegebenen Bedeutungen haben und ihren Salzen eine Verbindung der Formel I, worin $R^6$ Niederalkyl bedeutet, das durch eine Gruppe der Formel

$$-E-Z^2-Y^2-X^4-\underset{\underset{OM^2}{|}}{\overset{\overset{M^1}{\|}}{P}}-R_z \qquad \text{(Ide)}$$

substituiert ist, worin $R_z$ für gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und E, $Z^2$, $Y^2$ und $X^4$ die obengegebenen Bedeutungen haben, mit einer Verbindung der Formel XIV oder XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

m) zur Herstellung von solchen Verbindungen der Formel I, worin $R^6$ Niederalkyl bedeutet, das durch einen Rest der Formel Id substituiert ist, worin m für 0 steht, und $A^2$ die obengenannte Bedeutung hat, und ihren Salzen eine Verbindung der Formel I, worin $R^6$ Niederalkyl bedeutet, das durch eine Gruppe der Formel Idb, worin E die oben gegebene Bedeutung hat, substituiert ist, oder ein reaktionsfähiges Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels,umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

n) zur Herstellung von solchen Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes, substituiertes Amino oder die Gruppe der Formel Ie bedeutet, und ihren Salzen eine Verbindung der Formel I, worin eine der Gruppen $R^9$ und $R^{11}$ Hydroxy oder Mercapto darstellt, und die andere für freies Hydroxy oder Mercapto, von einem Rest der Formel Ie verschiedenes veräthertes Hydroxy oder Mercapto, freies oder von einem Rest der Formel Ie verschiedenes substituiertes Amino oder einen Rest der Formel Ie steht, oder ein reaktionsfähiges Säurederivat davon mit einer Verbindung der Formel XVIII,

$$H - X^5 - Y^3 - X^6 - A^3 \qquad \text{(XVIII)}$$

worin $X^5$, $Y^3$, $X^6$ und $A^3$ die oben gegebenen Bedeutungen haben, oder einem reaktionsfähigen Derivat davon, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

o) zur Herstellung von solchen Verbindungen der Formel I, worin eine der Gruppen $R^9$ und $R^{11}$ für den Rest der Formel Ie steht, worin $Y^3$ unsubstituiertes oder substituiertes Alkylen darstellt, das durch Iminocarbonyl oder Oxycarbonyl unterbrochen ist, und worin $X^5$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino, oder die Gruppe der Formel Ie bedeutet, und ihren Salzen eine Verbindung der Formel I, worin eine der Gruppen $R^9$ und $R^{11}$ für den Rest der Formel $-X^5-Y^3_a-R_x$ (Iea) steht, mit einer Verbindung der Formel $R_y-Y^3_b-X^6-A^3$ (XIX), worin eine der Gruppen $R_x$ und $R_y$ für ein freies oder in reaktionsfähiger derivatisierter Form vorliegendes Hydroxy oder Amino der Formel $-NH(R^{14})$ steht, und die andere für freies oder in reaktionsfähiger derivatisierter Form vorliegendes Carboxy steht, $Y^3_a$ und $Y^3_b$ unabhängig voneinander unsubstituiertes oder substituiertes Alkylen bedeuten, und $X^5$, $X^6$ und $A^3$ die oben gegebenen Bedeutungen haben, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, umsetzt und in einer erhaltenen Verbindung der Formel I vorhandene Schutzgruppen abspaltet, oder

p) zur Herstellung von solchen Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino oder die Gruppe der Formel Ie bedeutet, und ihren Salzen eine Verbindung der Formel I, worin eine der Gruppen $R^9$ und $R^{11}$ für den Rest der Formel $-X^5-Y^3-X^6-H$ (Ieb) steht, worin $X^5$, $Y^3$ und $X^6$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes, substituiertes Amino oder die Gruppe der Formel Ie oder Ieb bedeutet, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

q) zur Herstellung von solchen Verbindungen der Formel I, worin mindestens eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino oder die Gruppe der Formel Ie bedeutet, und ihren Salzen eine Verbindung der Formel I, worin mindestens eine der Gruppen $R^9$ und $R^{11}$ den Rest der Formel

$$-X^{5}-Y^{3}-X^{6}-\underset{\underset{OM^{2}}{|}}{\overset{\overset{M^{1}}{\|}}{P}}-R_{z} \qquad \text{(Iec)}$$

bedeutet,worin $R_z$ für gegebenenfalls in reaktionsfähiger,derivatisierter Form vorliegendes Hydroxy bedeutet, $=M^1$ für ein Elektronenpaar oder für Oxo steht,und $M^2$ Wasserstoff oder eine abspaltbare Gruppe darstellt, und $X^5$, $Y^3$ und $X^6$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes, substituiertes Amino oder die Gruppe der Formel Ie oder Iec bedeutet, mit einer Verbindung der Formel XIV oder XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

r) zur Herstellung von solchen Verbindungen der Formel I, worin $R^1$ für Wasserstoff, $X^1$ für -NH-, $R^2$ für den Rest einer organischen Carbonsäure, der Rest der Formel $-X^2-R^{13}$ für Hydroxy und $R^{12}$ für Wasserstoff stehen, und in denen der Pyranosering die Konfiguration der Glucopyranose hat, und ihren Salzen in einer Glucofuranose-Verbindung der Formel XX,

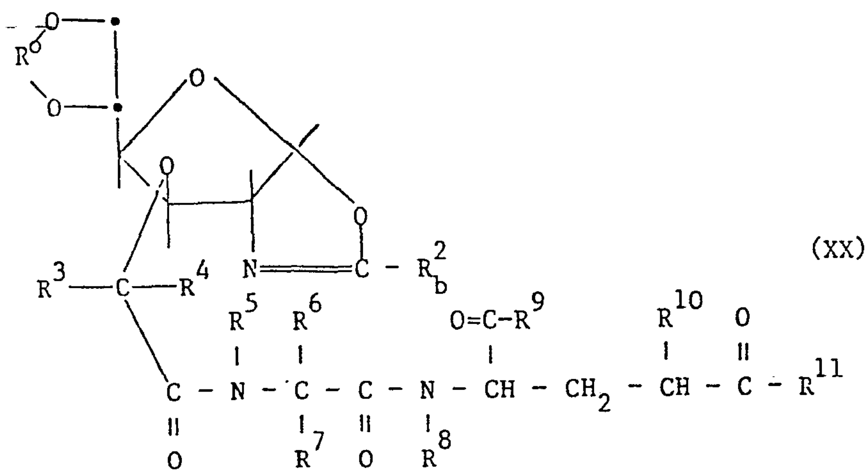

(XX)

worin $R_b^2$ für den Descarbonyl-Rest eines Acylrestes $R^2$ einer organischen Carbonsäure steht, und $R^o$ unsubstituiertes oder substituiertes Methylen bedeutet, und worin $R^3$, $R^4$, $R^5$, $R^6$, $R^7$, $R^8$, $R^9$, $R^{10}$ und $R^{11}$ die oben gegebenen Bedeutungen haben, und gegebenenfalls vorhandene freie funktionelle Gruppen in geschützter Form vorliegen können, den Oxazolin- und den Dioxolanring sauer aufspaltet, und in einer erhaltenen Verbindung der Formel I vorhandene Schutzgruppen abspaltet, und, wenn erwünscht, jeweils nach Durchführung von einem der Verfahren a) bis q) eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder, wenn erwünscht, ein erhaltenes Isomerengemisch in die einzelnen Isomeren auftrennt.

33. Die nach den Verfahren des Anspruchs 32 erhältlichen Verbindungen.

Patentansprüche: (für Oesterreich)

1. Verfahren zur Herstellung von Hexapyranoseverbindungen der Formel I, worin

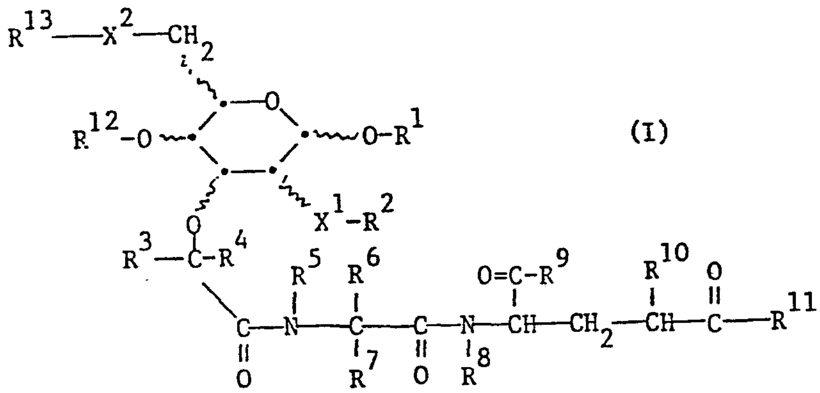

(I)

$X^1$ und $X^2$ unabhängig voneinander eine Gruppe der Formel -O- oder $-N(R^{14})-$, wobei $R^{14}$ für Wasserstoff oder Niederalkyl steht, $R^1$, $R^2$, $R^{12}$ und $R^{13}$ unabhängig voneinander einen Rest der Formel Ia,

$$-(Z^1-Y^1-X^3)_n-A^1 \quad \text{(Ia)}$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der Formel -O- oder $-N(R^{14})-$, worin $R^{14}$ die oben gegebene Bedeutung hat, und $A^1$ für einen Rest der Formel Ib,

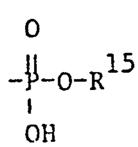

(Ib)

worin $R^{15}$ einen mindestens 7 Kohlenstoffatome aufweisenden aliphatischen oder cycloaliphatischen Rest darstellt, oder für eine Gruppe der Formel Ic,

```
 O       R16
 ||      |
-P - O - CH          (Ic)
 |       |
 OH      R17
```

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder einen unter physiologischen Bedingungen abspaltbaren Rest,

$R^3$, $R^4$, $R^5$, $R^7$ und $R^8$ unabhängig voneinander Wasserstoff oder Niederalkyl,

$R^6$ Wasserstoff oder Niederalkyl, das unsubstituiert oder durch eine Gruppe der Formel Id,

$$-E-(Z^2-Y^2-X^4)_m-A^2 \qquad \text{(Id)}$$

worin m für 0 oder 1, E für eine Gruppe der Formel -O-, -S- oder $-N(R^{14})-$, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel -O- oder $-N(R^{14})-$, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder

$R^5$ und $R^6$ zusammen unsubstituiertes oder substituiertes 1,3- oder

1,4-Niederalkylen,
$R^9$ und $R^{11}$ unabhängig voneinander einen Rest der Formel Ie,

$$-X^5-Y^3-X^6-A^3 \qquad (Ie)$$

worin $X^5$ für eine Gruppe der Formel -O-, -S- oder $-N(R^{14})-$ und $X^6$ für eine Gruppe der Formel -O- oder $-N(R^{14})-$, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und
$R^{10}$ Wasserstoff oder freies, verestertes oder amidiertes Carboxy bedeuten, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ von Acyl, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies oder veräthertes Hydroxy oder Mercapto, durch von der Gruppe der Formel Id verschiedenes verestertes Hydroxy oder Mercapto, durch freies oder von der Gruppe der Formel Id verschiedenes substituiertes Amino, oder durch Cycloalkyl, carbocyclisches Aryl oder stickstoffhaltiges Heteroaryl mit 5 bis 6 Ringgliedern im heterocyclischen Ring substituiert ist, oder $R^7$ von Wasserstoff verschieden ist, und von Salzen von solchen Verbindungen, dadurch gekennzeichnet, dass man

a) eine Verbindung der Formel II,

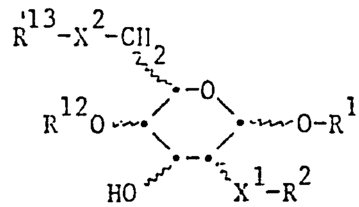

(II)

worin gegebenenfalls vorhandene funktionelle Gruppen, mit Ausnahme der freien Hydroxygruppe in 4-Stellung des Pyranoseteils, in geschützer Form vorliegen können, oder eine Metallverbindung davon mit einer Verbindung der Formel III,

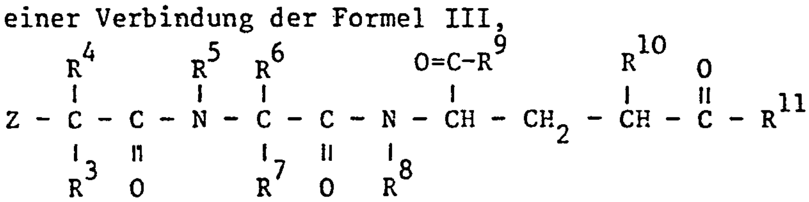

(III)

worin Z eine reaktionsfähige veresterte Hydroxygruppe darstellt und worin gegebenenfalls vorhandene funktionelle Gruppen durch Schutzgruppen geschützt sein können, umsetzt und gegebenenfalls vorhandene Schutzgruppen abspaltet, oder

b) eine Verbindung der Formel IV,

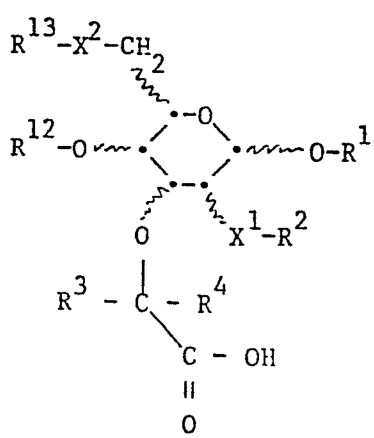

(IV)

worin gegebenenfalls vorhandene funktionelle Gruppen mit Ausnahme der freien Carboxylgruppe, in geschützter Form vorliegen können, oder ein reaktionsfähiges Carboxylderivat davon mit einer Verbindung der Formel V,

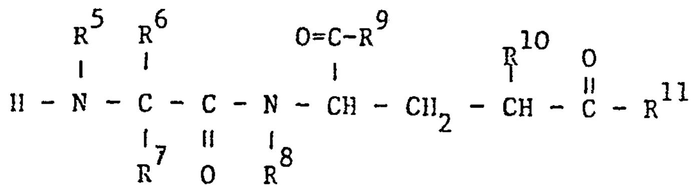

(V)

worin gegebenenfalls vorhandene freie funktionelle Gruppen, mit Ausnahme der Aminogruppe der Formel $HN(R^5)-$, in geschützter Form vorliegen können, oder einem reaktionsfähigen Derivat davon umsetzt und gegebenenfalls vorhandene Schutzgruppen abspaltet, oder

c) eine Verbindung der Formel VI,

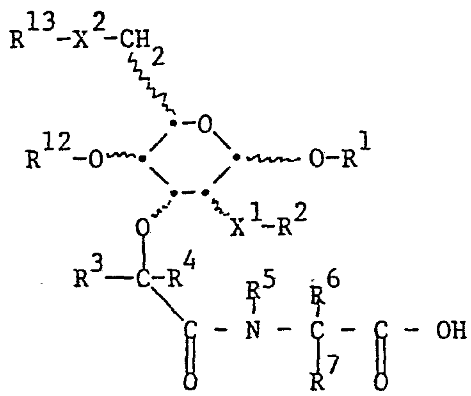

(VI)

worin gegebenenfalls vorhandene freie funktionelle Gruppen, mit Ausnahme der freien Carboxylgruppe, in geschützter Form vorliegen können, oder ein reaktionsfähiges Carboxylderivat davon mit einer Verbindung der Formel VII,

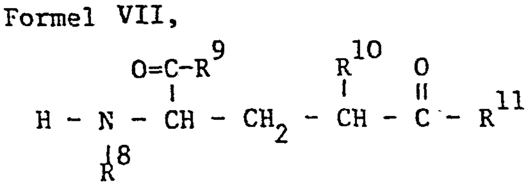

(VII)

worin gegebenenfalls vorhandene freie funktionelle Gruppen, mit Ausnahme der Aminogruppe der Formel $HN(R^8)-$, in geschützter Form vorliegen, oder einem reaktionsfähigen Derivat davon umsetzt, und gegebenenfalls vorhandene Schutzgruppen abspaltet, oder

d) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht und $Z^1$, $Y^1$, $X^3$ und $A^1$ die oben angegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder von ihren Salzen eine Verbindung der Formel I, worin mindestens eine der Gruppen $R^1$, $R^2$, $R^{12}$ und $R^{13}$ für Wasserstoff steht und die anderen unabhängig voneinander für Wasserstoff, vom Rest der Formel Ia verschiedenes Acyl, eine unter physiologischen Bedingungen abspaltbare Gruppe oder einen Rest der Formel Ia stehen, und worin die übrigen Substituenten, $R^3$, $R^4$, $R^5$, $R^6$, $R^7$, $R^8$, $R^9$, $R^{10}$, $R^{11}$, $X^1$ und $X^2$, die oben gegebenen Bedeutungen haben, wobei gegebenenfalls vorhandene freie funktionelle Gruppen, mit Ausnahme der an der Reaktion teilnehmenden Gruppe(n), in geschützter Form vorliegen können, oder ein reaktionsfähiges Derivat davon, mit einer Säure der Formel $HO - Z^1 - Y^1 - X^3 - A^1$ (X), worin $Z^1$, $Y^1$, $X^3$ und $A^1$ die oben gegebenen Bedeutungen haben, wobei gegebenenfalls vorhandene freie funktionelle Gruppen, mit Ausnahme der Säuregruppe der Formel $HO-Z^1-$, in geschützter Form vorliegen können, oder einem reaktionsfähigen Säurederivat davon umsetzt und in einer erhaltenen Verbindung der Formel I gegebenenfalls vorhandene Schutzgruppen abspaltet, oder

e) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, $Y^1$ für unsubstituiertes oder substituiertes Alkylen steht, das durch Iminocarbonyl oder Oxycarbonyl unterbrochen ist, und $Z^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder von ihren Salzen eine Verbindung der Formel I, worin mindestens eine der

Gruppen $R^1$, $R^2$, $R^{12}$ und $R^{13}$ für einen Rest der Formel $-Z^1-Y^1_a-R_x$ (Iaa) steht, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel $R_y-Y^1_b-X^3-A^1$ (XI) umsetzt, wobei $Z^1$, $X^3$ und $A^1$ die oben gegebenen Bedeutungen haben, und $Y^1_a$ und $Y^1_b$ unabhängig voneinander unsubstituiertes oder substituiertes Alkylen bedeuten, und worin eine der Gruppen $R_x$ und $R_y$ für freies oder in reaktionsfähiger, derivatisierter Form vorliegendes Hydroxy oder Amino der Formel $-NH(R^{14})$ steht, und die andere für freies oder in reaktionsfähiger, derivatisierter Form vorliegendes Carboxy steht, während die übrigen Substituenten $R^1$, $R^2$, $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion teilnehmenden funktionellen Gruppen, in geschützter Form vorliegen können, und in einer erhaltenen Verbindung der Formel I vorhandene Schutzgruppen abspaltet, oder

f) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben angegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder von ihren Salzen eine Verbindung der Formel I, worin mindestens eine der Gruppen $R^1$, $R^2$, $R^{12}$ und $R^{13}$ für einen Rest der Formel $-Z^1-Y^1-X^3-H$ (Iab) steht, worin $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, oder ein reaktionsfähiges Derivat davon mit einer Verbindung der Formel XII oder XIII,

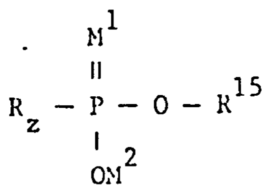

(XII)

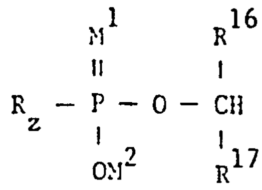

(XIII)

worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig, in Gegenwart eines oxidierenden Mittels, umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

g) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 1 steht, und $Z^1$, $Y^1$, $X^3$ und $A^1$ die oben gegebenen Bedeutungen haben, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, und ihren Salzen eine Verbindung der Formel I, worin mindestens eine der Gruppen $R^1$, $R^2$, $R^{12}$ und $R^{13}$ für einen Rest der Formel

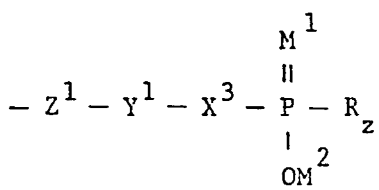

(Iac)

steht, worin $R_z$ gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine

abspaltbare Gruppe darstellt, und $Z^1$, $Y^1$ und $X^3$ die oben gegebenen Bedeutungen haben, und die anderen unabhängig voneinander Wasserstoff, vom Rest der Formel Ia verschiedenes Acyl, eine unter physiologischen Bedingungen abspaltbare Gruppe oder einen Rest der Formel Ia bedeuten, mit einer Verbindung der Formel XIV oder XV,

$$HO-R^{15}$$

(XIV)

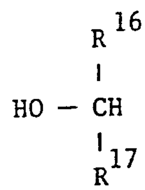

(XV)

worin $R^{15}$, $R^{16}$ und $R^{17}$ die oben gegebenen Bedeutungen haben, oder einem Derivat davon, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

h) zur Herstellung von solchen Verbindungen der Formel I, worin mindestens einer der Reste $R^1$, $R^2$, $R^{12}$ und $R^{13}$ eine Gruppe der Formel Ia bedeutet, worin n für 0 steht, und $A^1$ die obengenannte Bedeutung hat, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, und ihren Salzen eine Verbindung der Formel I, worin mindestens eine der Gruppen $R^1$, $R^2$, $R^{12}$ und $R^{13}$ für Wasserstoff steht, und die anderen Reste unabhängig voneinander Wasserstoff, vom Rest der Formel Ia verschiedenes Acyl, eine unter physiologischen Bedingungen abspaltbare Gruppe oder einen Rest der Formel Ia bedeuten, oder ein Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, $M^2$ Wasserstoff oder eine

abspaltbare Gruppe bedeutet und $R^{15}$, $R^{16}$ und $R^{17}$ die oben gegebenen Bedeutungen haben, wenn notwendig, in Gegenwart eines oxidierenden Mittels umsetzt, wobei in den Ausgangsstoffen die übrigen Substituenten $R^3$, $R^4$, $R^5$, $R^6$, $R^7$, $R^8$, $R^9$, $R^{10}$, $R^{11}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

i) zur Herstellung von solchen Verbindungen der Formel I, worin $R^6$ Niederalkyl bedeutet, das durch den Rest der Formel Id, worin m für 1 steht, und die Gruppen E, $Z^2$, $Y^2$, $X^4$ und $A^2$ die oben gegebenen Bedeutungen haben, substituiert ist, und ihren Salzen eine Verbindung der Formel I, worin $R^6$ für Niederalkyl steht, das durch die Gruppe der Formel -E-H (Idb), worin E die oben gegebene Bedeutung hat, substituiert ist, oder ein reaktionsfähiges Derivat davon mit einer Säure der Formel $HO-Z^2-Y^2-X^4-A^2$ (XVI), worin $Z^2$, $Y^2$, $X^4$ und $A^2$ die oben gegebenen Bedeutungen haben, oder einem reaktionsfähigen Derivat davon umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

j) zur Herstellung von solchen Verbindungen der Formel I, worin $R^6$ für Niederalkyl steht, das durch den Rest der Formel Id, wobei m für 1 steht, $Y^2$ unsubstituiertes oder substituiertes Alkylen bedeutet, das durch Iminocarbonyl oder Oxycarbonyl unterbrochen ist, und die Gruppen E, $Z^2$, $X^4$ und $A^2$ die oben gegebenen Bedeutungen haben, substituiert ist, und ihren Salzen eine Verbindung der Formel I, worin $R^6$ für Niederalkyl steht, das durch die Gruppe $-E-Z^2-Y_a^2-R_x$ (Idc) substi-

tuiert ist, mit einer Verbindung der Formel $R_y-Y_b^2-X^4-A^2$ (XVII) umsetzt, worin $Y_a^2$ und $Y_b^2$ unabhängig voneinander unsubstituiertes oder substituiertes Alkylen bedeuten, und worin eine der Gruppen $R_x$ und $R_y$ für freies oder in reaktionsfähiger derivatisierter Form vorliegendes Hydroxy oder Amino der Formel $-NH(R^{14})$ steht, und die andere für freies oder in reaktionsfähiger, derivatisierter Form vorliegendes Carboxy steht, und E, $Z^2$, $X^4$ und $A^2$ die obigen Bedeutungen haben, während die übrigen Substituenten der Ausgangsstoffe $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

k) zur Herstellung von solchen Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die oben gegebenen Bedeutungen haben, und ihren Salzen eine Verbindung der Formel I, worin $R^6$ Niederalkyl bedeutet, das durch eine Gruppe der Formel $-E-Z^2-Y^2-X^4-H$ (Idd) substituiert ist, worin E, $Z^2$, $Y^2$ und $X^4$ die oben gegebenen Bedeutungen haben, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, und $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmende Gruppen, in geschützter Form vorliegen können, und in einer erhaltenen Verbindung der Formel I vorhandene Schutzgruppen abspaltet, oder

l) zur Herstellung von solchen Verbindungen der Formel I, worin $R^6$ Niederalkyl bedeutet, das durch einen Rest der Formel Id substituiert ist, worin m für 1 steht, und die Gruppen E, $Z^2$, $Y^2$, $X^4$ und $A^2$ die obengegebenen Bedeutungen haben und ihren Salzen eine Verbindung der Formel I, worin $R^6$ Niederalkyl bedeutet, das durch eine Gruppe der Formel

$$-E-Z^2-Y^2-X^4-\overset{\overset{\displaystyle M^1}{\|}}{\underset{\underset{\displaystyle OM^2}{|}}{P}}-R_z \qquad \text{(Ide)}$$

substituiert ist, worin $R_z$ für gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und E, $Z^2$, $Y^2$ und $X^4$ die obengegebenen Bedeutungen haben, mit einer Verbindung der Formel XIV oder XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

m) zur Herstellung von solchen Verbindungen der Formel I, worin $R^6$ Niederalkyl bedeutet, das durch einen Rest der Formel Id substituiert ist, worin m für 0 steht, und $A^2$ die obengenannte Bedeutung hat, und ihren Salzen eine Verbindung der Formel I, worin $R^6$ Niederalkyl bedeutet, das durch eine Gruppe der Formel Idb, worin E die oben gegebene Bedeutung hat, substituiert ist, oder ein reaktionsfähiges Derivat davon mit einer Verbindung der Formel XII oder XIII, worin $R_z$ gegebenenfalls in reaktionsfähiger Form vorliegendes Hydroxy bedeutet, $=M^1$ ein Elektronenpaar oder Oxo darstellt, und $M^2$

Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^7$, $R^8$, $R^9$, $R^{10}$, $R^{11}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

n) zur Herstellung von solchen Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes, substituiertes Amino oder die Gruppe der Formel Ie bedeutet, und ihren Salzen eine Verbindung der Formel I, worin eine der Gruppen $R^9$ und $R^{11}$ Hydroxy oder Mercapto darstellt, und die andere für freies Hydroxy oder Mercapto, von einem Rest der Formel Ie verschiedenes veräthertes Hydroxy oder Mercapto, freies oder von einem Rest der Formel Ie verschiedenes substituiertes Amino oder einen Rest der Formel Ie steht, oder ein reaktionsfähiges Säurederivat davon mit einer Verbindung der Formel XVIII,

$$H - X^5 - Y^3 - X^6 - A^3 \qquad \text{(XVIII)}$$

worin $X^5$, $Y^3$, $X^6$ und $A^3$ die oben gegebenen Bedeutungen haben, oder einem reaktionsfähigen Derivat davon, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

o) zur Herstellung von solchen Verbindungen der Formel I, worin eine der Gruppen $R^9$ und $R^{11}$ für den Rest der Formel Ie steht, worin $Y^3$ unsubstituiertes oder substituiertes Alkylen darstellt, das durch Iminocarbonyl oder Oxycarbonyl unterbrochen ist, und worin $X^5$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino, oder die Gruppe der Formel Ie bedeutet, und ihren Salzen eine Verbindung der Formel I, worin eine der Gruppen $R^9$ und $R^{11}$ für den Rest der Formel $-X^5-Y^3_a-R_x$ (Iea) steht, mit einer Verbindung der Formel $R_y-Y^3_b-X^6-A^3$ (XIX), worin eine der Gruppen $R_x$ und $R_y$ für ein freies oder in reaktionsfähiger derivatisierter Form vorliegendes Hydroxy oder Amino der Formel $-NH(R^{14})$ steht, und die andere für freies oder in reaktionsfähiger derivatisierter Form vorliegendes Carboxy steht, $Y^3_a$ und $Y^3_b$ unabhängig voneinander unsubstituiertes oder substituiertes Alkylen bedeuten, und $X^5$, $X^6$ und $A^3$ die oben gegebenen Bedeutungen haben, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, umsetzt und in einer erhaltenen Verbindung der Formel I vorhandene Schutzgruppen abspaltet, oder

p) zur Herstellung von solchen Verbindungen der Formel I, worin eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino oder die Gruppe der Formel Ie bedeutet, und ihren Salzen eine Verbindung der Formel I, worin eine der Gruppen $R^9$ und $R^{11}$ für den Rest der Formel $-X^5-Y^3-X^6-H$ (Ieb) steht, worin $X^5$, $Y^3$ und $X^6$ die oben gegebenen Bedeutungen haben, und die andere freies Hydroxy oder Mercapto, von einem Rest der Formel Ie

verschiedenes veräthertes Hydroxy oder Mercapto, freies oder von einem Rest der Formel Ie verschiedenes, substituiertes Amino oder die Gruppe der Formel Ie oder Ieb bedeutet, oder ein reaktionsfähiges Derivat davon mit einer Verbindung der Formel XII oder XIII, worin $R_z$ gegebenenfalls in reaktionsfähiger, derivatisierter Form vorliegendes Hydroxy bedeutet, $=M^1$ ein Elektronenpaar oder Oxo darstellt, $M^2$ Wasserstoff oder eine abspaltbare Gruppe bedeutet, und $R^{15}$, $R^{16}$ und $R^{17}$ die oben gegebenen Bedeutungen haben, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, und gegebenenfalls vorhandene funktionelle Gruppen, mit Ausnahme der an der Reaktion teilnehmenden Gruppen, in geschützter Form vorliegen können, und in einer erhaltenen Verbindung der Formel I vorhandene Schutzgruppen abspaltet, oder

q) zur Herstellung von solchen Verbindungen der Formel I, worin mindestens eine der Gruppen $R^9$ und $R^{11}$ für den Rest der Formel Ie steht, worin $X^5$, $Y^3$, $X^6$ und $A^3$ die oben gegebenen Bedeutungen haben, und die andere freies Hydroxy oder Mercapto, von einem Rest der Formel Ie verschiedenes veräthertes Hydroxy oder Mercapto, freies oder von einem Rest der Formel Ie verschiedenes substituiertes Amino oder die Gruppe der Formel Ie bedeutet, und ihren Salzen eine Verbindung der Formel I, worin mindestens eine der Gruppen $R^9$ und $R^{11}$ den Rest der Formel

$$-X^5-Y^3-X^6-\overset{\overset{\displaystyle M^1}{\|}}{\underset{\underset{\displaystyle OM^2}{|}}{P}}-R_z \qquad \text{(Iec)}$$

bedeutet, worin $R_z$ für gegebenenfalls in reaktionsfähiger, derivatisierter Form vorliegendes Hydroxy bedeutet, $=M^1$ für ein Elektronenpaar oder für Oxo steht, und $M^2$ Wasserstoff oder eine abspaltbare Gruppe darstellt, und $X^5$, $Y^3$ und $X^6$ die oben gegebenen Bedeutungen haben, und die andere freies Hydroxy oder Mercapto, von einem Rest der Formel Ie verschiedenes veräthertes Hydroxy oder Mercapto, freies oder von einem Rest

der Formel Ie verschiedenes, substituiertes Amino oder die Gruppe der Formel Ie oder Iec bedeutet, mit einer Verbindung der Formel XIV oder XV, worin $R^{15}$, $R^{16}$ und $R^{17}$ die oben gegebenen Bedeutungen haben, oder einem Derivat davon, wenn notwendig in Gegenwart eines oxidierenden Mittels, umsetzt, wobei in den Ausgangsstoffen die übrigen Substituenten $R^1$, $R^2$, $R^3$, $R^4$, $R^5$, $R^6$, $R^7$, $R^8$, $R^{10}$, $R^{12}$, $R^{13}$, $X^1$ und $X^2$ die oben gegebenen Bedeutungen haben, wobei freie funktionelle Gruppen, mit Ausnahme der an der Reaktion beteiligten Gruppen, in geschützter Form vorliegen können, und in einer erhaltenen Verbindung der Formel I vorhandene Schutzgruppen abspaltet, oder

r) zur Herstellung von solchen Verbindungen der Formel I, worin $R^1$ für Wasserstoff, $X^1$ für -NH-, $R^2$ für den Rest einer organischen Carbonsäure, der Rest der Formel $-X^2-R^{13}$ für Hydroxy und $R^{12}$ für Wasserstoff stehen, und in denen der Pyranosering die Konfiguration der Glucopyranose hat, und ihren Salzen in einer Glucofuranose-Verbindung der Formel XX,

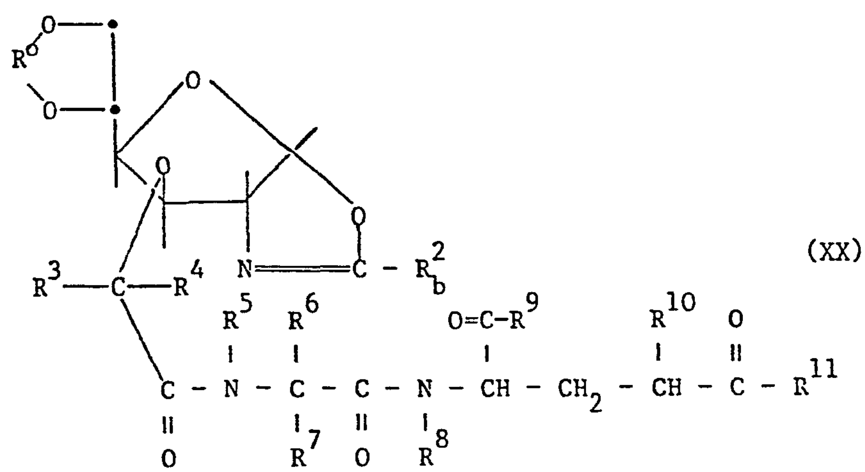

(XX)

worin $R_b^2$ für den Descarbonyl-Rest eines Acylrestes $R^2$ einer organischen Carbonsäure steht, und $R^o$ unsubstituiertes oder substituiertes Methylen bedeutet, und worin $R^3$, $R^4$, $R^5$, $R^6$, $R^7$, $R^8$, $R^9$, $R^{10}$ und $R^{11}$ die oben gegebenen Bedeutungen haben, und gegebenenfalls vorhandene freie funktionelle Gruppen in geschützter Form vorliegen können, den Oxazolin- und den Dioxolanring sauer aufspaltet, und in

einer erhaltenen Verbindung der Formel I vorhandene Schutzgruppen abspaltet, und, wenn erwünscht, jeweils nach Durchführung von einem der Verfahren a) bis q) eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder, wenn erwünscht, ein erhaltenes Isomerengemisch in die einzelnen Isomeren auftrennt.

2. Verfahren nach Anspruch 1, dadurch gekennzeichnet, dass man Verbindungen der Formel I nach Anspruch 1, worin $R^1$ für den Rest der Formel Ia gemäss Anspruch 1 steht, $R^2$, $R^{12}$ und $R^{13}$ unabhängig voneinander Wasserstoff, vom Rest der Formel Ia, worin n für 1 steht, verschiedenes Acyl oder eine unter physiologischen Bedingungen abspaltbare Gruppe bedeuten, $R^5$ Wasserstoff oder Niederalkyl bedeutet, $R^6$ für Wasserstoff, unsubstituiertes oder durch freies oder veräthertes Hydroxy oder Mercapto, von einer Gruppe der Formel Id gemäss Anspruch 1 verschiedenes, verestertes Hydroxy oder Mercapto, freies oder von einer entsprechenden Gruppe der Formel Id verschiedenes, substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie gemäss Anspruch 1 verschiedenes, veräthertes Hydroxy oder Mercapto oder freies oder von einem Rest der Formel Ie verschiedenes, substituiertes Amino bedeuten, wobei freie funktionelle Gruppen in geschützter Form vorliegen können, und Salze von solchen Verbindungen herstellt.

3. Verfahren nach Anspruch 1, dadurch gekennzeichnet, dass man Verbindungen der Formel I nach Anspruch 1, worin $R^2$ für den Rest

der Formel Ia gemäss Anspruch 1 steht, $R^1$, $R^{12}$ und $R^{13}$ unabhängig voneinander Wasserstoff, vom Rest der Formel Ia, worin n für 1 steht, verschiedenes Acyl oder eine unter physiologischen Bedingungen abspaltbare Gruppe darstellen, $R^5$ Wasserstoff oder Niederalkyl bedeutet, $R^6$ für Wasserstoff, unsubstituiertes oder durch freies oder veräthertes Hydroxy oder Mercapto, von einer entsprechenden Gruppe der Formel Id gemäss Anspruch 1 verschiedenes, verestertes Hydroxy oder Mercapto, freies oder von einer Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie gemäss Anspruch 1 verschiedenes, veräthertes Hydroxy oder Mercapto oder freies oder von einem Rest der Formel Ie verschiedenes substituiertes Amino bedeuten, wobei freie funktionelle Gruppen in geschützter Form vorliegen können, und Salze von solchen Verbindungen herstellt.

4. Verfahren nach Anspruch 1, dadurch gekennzeichnet, dass man Verbindungen der Formel I nach Anspruch 1, worin $R^{13}$ für den Rest der Formel Ia gemäss Anspruch 1 steht, $R^1$, $R^2$ und $R^{12}$ unabhängig voneinander Wasserstoff, vom Rest der Formel Ia, worin n für 1 steht, verschiedenes Acyl oder eine unter physiologischen Bedingungen abspaltbare Gruppe darstellen, $R^5$ Wasserstoff oder Niederalkyl bedeutet, $R^6$ für Wasserstoff, unsubstituiertes oder durch freies oder veräthertes Hydroxy oder Mercapto, von einer Gruppe der Formel Id gemäss Anspruch 1 verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen

unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie gemäss Anspruch 1 verschiedenes veräthertes Hydroxy oder Mercapto oder freies oder von einem Rest der Formel Ie verschiedenes substituiertes Amino bedeuten, wobei freie funktionelle Gruppen in geschützter Form vorliegen können, und Salze von solchen Verbindungen herstellt.

5. Verfahren nach Anspruch 1, dadurch gekennzeichnet, dass man Verbindungen der Formel I nach Anspruch 1, worin $R^1$, $R^2$, $R^{12}$ und $R^{13}$ unabhängig voneinander Wasserstoff, vom Rest der Formel Ia gemäss Anspruch 1, worin n für 1 steht, verschiedenes Acyl oder eine unter physiologischen Bedingungen abspaltbare Gruppe darstellen, $R^6$ für durch einen Rest der Formel Id oder Ida substituiertes Niederalkyl steht,

$$-\overset{\overset{\displaystyle O}{\|}}{C}-E-(Y^2-X^4)_m-A^2 \qquad \text{(Ida)}$$

worin m, E, $Z^2$, $Y^2$, $X^4$ und $A^2$ die in Anspruch 1 gegebenen Bedeutungen haben, und $R^9$ und $R^{11}$ unabhängig voneinander freies Hydroxy oder Mercapto, von einem Rest der Formel Ie gemäss Anspruch 1 verschiedenes veräthertes Hydroxy oder Mercapto, freies oder von einem Rest der Formel Ie verschiedenes substituiertes Amino bedeuten, wobei freie funktionelle Gruppen in geschützter Form vorliegen können, und Salze von solchen Verbindungen herstellt.

6. Verfahren nach Anspruch 1, dadurch gekennzeichnet, dass man Verbindungen der Formel I nach Anspruch 1, worin $R^1$, $R^2$, $R^{12}$ und $R^{13}$ unabhängig voneinander für Wasserstoff, den Acylrest einer aliphatischen, cycloaliphatischen, aromatischen oder araliphatischen Carbonsäure mit bis zu 90 Kohlenstoffatomen, Tri-niederalkyl-silyl oder einen Rest der Formel Ia stehen, worin n und $X^3$ die in Anspruch 1 gegebenen Bedeutungen haben, $Z^1$ für Carbonyl steht, $Y^1$

Niederalkylen bedeutet, das durch Iminocarbonyl oder Oxycarbonyl unterbrochen sein kann, und $A^1$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder mit einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, $R^6$ Wasserstoff oder Niederalkyl bedeutet, das unsubstituiert oder durch einen Rest der Formel Id substituiert ist, worin m, E und $X^4$ die oben gegebenen Bedeutungen haben, $Z^2$ für Carbonyl steht, $Y^2$ Niederalkylen bedeutet, das durch Iminocarbonyl oder Oxycarbonyl unterbrochen sein kann, und $A^2$ für einen Rest der Formel Ib oder Ic, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Acylrest verestertes Hydroxymethyl bedeuten, oder $R^6$ Niederalkyl bedeutet, das durch Hydroxy oder Mercapto, durch mit einem bis zu 90 Kohlenstoffatome enthaltenden aliphatischen Rest veräthertes Hydroxy oder Mercapto, durch mit einem bis zu 90 Kohlenstoffatome aufweisenden aliphatischen Acylrest verestertes Hydroxy oder Mercapto, der von der Gruppe der Formel Id verschieden ist, durch Amino, durch mit einem bis zu 90 Kohlenstoffatome enthaltenden Acylrest substituiertes Amino, der von einem Rest der Formel Id verschieden ist, durch freies Carboxy, Niederalkoxycarbonyl, Carbamoyl, Niederalkylaminocarbonyl, Carboxyniederalkylaminocarbonyl oder amidiertes Carboxyl der Formel Ida gemäss Anspruch 5, unsubstituiertes oder durch Hydroxy, Niederalkoxy oder Halogen substituiertes Phenyl oder durch Imidazolyl oder Indolyl substituiert ist, oder $R^5$ und $R^6$ zusammen 1,3- oder 1,4-Niederalkylen bedeuten, $R^9$ und $R^{11}$ unabhängig voneinander Hydroxy, Niederalkoxy, Amino, Niederalkylamino, Carboxyniederalkylamino, oder einen Rest der Formel Ie bedeuten, worin $X^5$ und $X^6$ die oben gegebenen Bedeutungen haben, $Y^3$ Niederalkylen bedeutet, das durch Iminocarbonyl oder Oxycarbonyl unterbrochen sein kann, und $A^3$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, und $R^{10}$ für Wasserstoff, Carboxy, Niederalkoxycarbonyl, Carbamoyl, Niederalkylaminocarbonyl oder Carboxyniederalkylaminocarbonyl steht, wobei freie funktionelle Gruppen in geschützter Form vorliegen können, mit der Massgabe, dass die Verbindungen der Formel I mindestens einen Rest $A^1$, $A^2$ oder $A^3$ aufweisen, und mit der weiteren Massgabe, dass in Verbindungen der Formel I, worin mindestens einer der Reste $R^9$ oder $R^{11}$ für die Gruppe der Formel Ie steht, der Pyranosering vom D-Glucopyranosering, oder $R^1$ von Wasserstoff, oder $X^1$ vom Rest der Formel $-N(R^{14})-$ und $R^2$ vom Acylrest einer aliphatischen, cycloaliphatischen, aromatischen oder araliphatischen Carbonsäure mit bis zu 90 Kohlenstoffatomen, oder $R^{12}$ von Wasserstoff, oder der Rest der Formel $-X^2-R^{13}$ von Hydroxy, oder $R^4$ von Wasserstoff, oder $R^6$ von Wasserstoff oder von Niederalkyl, das unsubstituiert oder durch freies Hydroxy oder Mercapto, durch mit einem bis zu 90 Kohlenstoffatome enthaltenden aliphatischen Rest veräthertes Hydroxy oder Mercapto, durch von der Gruppe der Formel Id verschiedenes, verestertes Hydroxy oder Mercapto, durch freies oder mit einem bis zu 90 Kohlenstoffatome enthaltenden Acylrest substituiertes und von der Gruppe der Formel Id verschiedenes Amino, durch freies Carboxy, Niederalkylaminocarbonyl, unsubstituiertes oder durch Hydroxy, Niederalkoxy oder Halogen substituiertes Phenyl, oder durch Imidazolyl oder Indolyl substituiert ist, oder $R^7$ von Wasserstoff verschieden ist, und Salze von solchen Verbindungen herstellt.

7. Verfahren nach Anspruch 1, dadurch gekennzeichnet, dass man Verbindungen der Formel I nach Anspruch 6, worin $R^1$, $R^2$, $R^{12}$ und $R^{13}$ unabhängig voneinander für Wasserstoff, den Acylrest einer unsubstituierten oder durch Hydroxy, Amino und/oder Alkanoylamino substituierten Alkancarbonsäure mit bis zu 90 Kohlenstoffatomen, Tri-niederalkyl-silyl oder einen Rest der Formel Ia gemäss Anspruch 6 stehen, herstellt.

8. Verfahren nach Anspruch 1, dadurch gekennzeichnet, dass man Verbindungen der Formel I, nach Anspruch 6, worin sich der Zuckerteil von der D-Glucose ableitet,
$X^1$ für die Gruppe NH und
$X^2$ für Sauerstoff stehen,
$R^1$ Wasserstoff, Niederalkanoyl oder die Gruppe der Formel Ia bedeutet, worin n für 1 steht, $Z^1$ Carbonyl bedeutet, $Y^1$ Niederalkylen darstellt, das durch Iminocarbonyl unterbrochen sein kann, $X^3$ für eine Gruppe der Formel -O- oder -NH- steht, und $A^1$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden Alkylrest, der unsubstituiert oder durch Hydroxy, Amino und/oder Alkanoylamino mit bis zu 30 Kohlenstoffatomen substituiert ist, veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander

Hydroxymethyl darstellen, worin die Hydroxygruppe mit einem 7 bis 30 Kohlenstoffatome aufweisenden unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R^2$ Niederalkanoyl, Hydroxyniederalkanoyl, Benzoyl oder die Gruppe der Formel Ia bedeutet, worin n, $Z^1$, $Y^1$, $X^3$ und $A^1$ die oben in diesem Anspruch gegebenen Bedeutungen haben, $R^{12}$ Wasserstoff oder Niederalkanoyl darstellt, $R^{13}$ Wasserstoff, Alkanoyl oder Hydroxyalkanoyl mit bis zu 90 Kohlenstoffatomen, Alkanoylaminoalkanoyl mit bis zu 30 Kohlenstoffatomen oder die Gruppe der Formel Ia bedeutet, worin n, $Z^1$, $Y^1$, $X^3$ und $A^1$ die oben in diesem Anspruch gegebenen Bedeutungen haben, $R^3$ und $R^7$ für Wasserstoff oder Methyl stehen, $R^4$, $R^5$, $R^8$ und $R^{10}$ für Wasserstoff stehen, $R^6$ Wasserstoff, unsubstituiertes oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl, Indolyl oder durch eine Gruppe der Formel Id substituiertes Niederalkyl bedeutet, worin m für 1 steht, E für eine Gruppe der Formel -O- oder -S- steht, $Z^2$ Carbonyl bedeutet, $Y^2$ Niederalkylen darstellt, das durch Iminocarbonyl unterbrochen sein kann, $X^4$ für eine Gruppe der Formel -O- steht, und $A^2$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem, 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxy-

methyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, und jeder der Reste $R^9$ und $R^{11}$ unabhängig voneinander für Hydroxy, Niederalkoxy, Amino, Niederalkylamino, Carboxyniederalkylamino oder einen Rest der Formel Ie stehen, worin $X^5$ für eine Gruppe der Formel -O- oder -NH- steht, $Y^3$ Niederalkylen darstellt, das durch Iminocarbonyl unterbrochen sein kann, $X^6$ für eine Gruppe der Formel -O- steht und $A^3$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R^9$ für Amino, Niederalkylamino oder Carboxyniederalkylamino und $R^{11}$ für Hydroxy stehen, mit der Massgabe, dass die Verbindungen mindestens einen Rest aus der Gruppe $A^1$, $A^2$ und $A^3$ aufweisen, und mit der weiteren Massgabe, dass in

Verbindungen der Formel I, worin mindestens einer der Reste $R^9$ oder $R^{11}$ für die Gruppe der Formel Ie steht, $R^1$ von Wasserstoff, oder $R^2$ von Niederalkanoyl, Hydroxyniederalkanoyl oder Benzoyl, oder $R^{12}$ von Wasserstoff, oder $R^{13}$ von Wasserstoff, oder $R^6$ von Wasserstoff, unsubstituiertem oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl oder Indolyl substituiertem Niederalkyl, oder $R^7$ von Wasserstoff verschieden ist, und Salze dieser Verbindungen herstellt.

9. Verfahren nach Anspruch 1, dadurch gekennzeichnet, dass man Verbindungen der Formel I nach Anspruch 6, worin
$X^1$ eine Gruppe der Formel $-N(R^{14})-$, wobei
$R^{14}$ für Wasserstoff oder $C_{1-4}$-Alkyl steht,
$X_2$ Sauerstoff,
$R^1$ Wasserstoff, Niederalkanoyl oder die Gruppe der Formel Ia, worin n für 0 oder 1, $Z^1$ für Carbonyl, $Y^1$ für Niederalkylen, das durch Iminocarbonyl unterbrochen sein kann, $X^3$ für Sauerstoff und $A^1$ für einen Rest der Formel Ib oder Ic stehen, worin $R^{15}$ für einen 7 - 30 Kohlenstoffatome aufweisenden unsubstituierten oder durch Hydroxy, Amino und/oder Alkanoylamino mit bis zu 22 Kohlenstoffatomen substituierten Alkyl- oder Alkenylrest, $R^{16}$ für Wasserstoff und $R^{17}$ für 2-Hydroxy- oder 1,2-Dihydroxyäthyl, wobei in einem Rest $R^{17}$ mindestens eine Hydroxygruppe mit einem 7 - 30 Kohlenstoffatome aufweisenden Alkyl- oder Alkenylrest veräthert oder mit einem 7 - 30 Kohlenstoffatome aufweisenden Alkanoyl- oder Alkenoylrest verestert ist, oder $R^{16}$ und $R^{17}$ unabhängig voneinander für Hydroxymethyl, wobei die Hydroxygruppe mit einem 7 - 30 Kohlenstoffatome aufweisenden Alkyl- oder Alkenylrest veräthert oder mit einem 7-30 Kohlenstoffatome aufweisenden Alkanoyl- oder Alkenoylrest verestert ist, stehen,
$R^2$ Niederalkanoyl, Hydroxyniederalkanoyl, Benzoyl oder unabhängig von $R^1$,$R^{12}$ und $R^{13}$ eine wie vorstehend in diesem Anspruch definierte Gruppe der Formel Ia,

$R^3$, $R^4$, $R^5$, $R^7$ und $R^8$ unabhängig voneinander Wasserstoff, Methyl oder Aethyl,
$R^6$ Wasserstoff oder unsubstituiertes oder durch freies Hydroxy, freies Mercapto, Niederalkoxy, Niederalkylthio, Alkanoyloxy mit 2 - 30 Kohlenstoffatomen, Alkenoyloxy mit 6 - 30 Kohlenstoffatomen, Phenyl, 4-Hydroxy-phenyl oder durch eine Gruppe der Formel Id substituiertes Niederalkyl, worin m für 0 oder 1, E für Sauerstoff oder Schwefel, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der wie vorstehend in diesem Anspruch definierten Formeln Ib oder Ic stehen,
$R^9$ und $R^{11}$ unabhängig voneinander Hydroxy, Niederalkoxy, Amino, Niederalkylamino, Carboxyniederalkylamino, Niederalkoxycarbonylniederalkylamino, Carbamoylniederalkylamino oder einen Rest der Formel Ie, worin $X^5$ für Sauerstoff oder NH, $Y^3$ für Niederalkylen, das durch Iminocarbonyl unterbrochen sein kann, $X^6$ für Sauerstoff und $A^3$ unabhängig von $A^1$ und $A^2$ für einen Rest der wie vorstehend in diesem Anspruch definierten Formeln Ib oder Ic stehen, $R^{10}$ Wasserstoff, $R^{12}$ Wasserstoff, Niederalkanoyl oder den gleichen Rest wie $R^{13}$, und
$R^{13}$ Wasserstoff, Alkanoyl oder Alkenoyl mit jeweils bis zu 30 Kohlenstoffatomen oder unabhängig von $R^1$ und $R^2$ einen wie vorstehend in diesem Anspruch definierten Rest der Formel Ia bedeuten, mit der Massgabe, dass die Verbindungen mindestens einen und höchstens zwei Reste aus der Gruppe $A^1$, $A^2$ und $A^3$ aufweisen und mit der weiteren Massgabe, dass in denjenigen Verbindungen, in denen mindestens ein Rest aus der Gruppe $R^9$ und $R^{11}$ für die Gruppe der Formel Ie steht, $R^1$ von Wasserstoff, oder $R^2$ von Niederalkanoyl, Hydroxyniederalkanoyl oder Benzoyl, oder $R^4$ von Wasserstoff, oder $R^6$ von Wasserstoff oder von unsubstituiertem oder durch freies Hydroxy, freies Mercapto, Niederalkoxy, Niederalkylthio, Alkanoyloxy, Alkenoyloxy, Phenyl oder 4-Hydroxy-phenyl substituiertem Niederalkyl, oder $R^7$, $R^{12}$ oder $R^{13}$

von Wasserstoff verschieden ist, und Salze dieser Verbindungen herstellt.

10. Verfahren nach Anspruch 1, dadurch gekennzeichnet, dass man Verbindungen der Formel I nach Anspruch 9, worin

$X^1$ die Gruppe NH,
$X^2$ Sauerstoff,
$R^1$ Wasserstoff oder Niederalkanoyl,
$R^2$ Niederalkanoyl, Benzoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen Alkylrest mit 7 bis 22 C-Atomen, $R^{16}$ für Wasserstoff und $R^{17}$ für 1,2-Dihydroxyäthyl stehen, wobei beide Hydroxygruppen unabhängig voneinander jeweils mit einem 10 - 22 Kohlenstoffatome aufweisenden Alkanoyl- oder Alkenoylrest verestert sind,
$R^3$ Wasserstoff oder Methyl,
$R^4$, $R^5$, $R^7$ und $R^8$ Wasserstoff,
$R^6$ Wasserstoff oder unsubstituiertes oder durch freies Hydroxy, Alkanoyloxy mit 2 bis 22 Kohlenstoffatomen, Alkenoyloxy mit 6 - 22 Kohlenstoffatomen, Phenyl oder durch eine Gruppe der Formel Id substituiertes Niederalkyl, worin m für 0 oder 1, E für Sauerstoff, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der vorstehend definierten Formeln Ib und Ic stehen, $R^9$ und $R^{11}$ unabhängig voneinander Hydroxy, Niederalkoxy, Amino, Niederalkylamino, α-Carboxy-niederalkylamino, α-Niederalkoxycarbonyl-niederalkylamino, α-Carbamoyl-niederalkylamino oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhänig von $A^1$ und $A^2$ für einen Rest der vorstehend definierten Formeln Ib oder Ic stehen,
$R^{10}$ Wasserstoff,

$R^{12}$ Wasserstoff, Niederalkanoyl oder den gleichen Rest wie $R^{13}$ und $R^{13}$ Wasserstoff, Alkanoyl mit 2 bis 22 Kohlenstoffatomen, Alkenoyl mit 6 bis 22 Kohlenstoffatomen oder unabhängig von $R^2$ einen wie vorstehend definierten Rest der Formel Ia bedeuten, und Salze dieser Verbindungen herstellt.

11. Verfahren nach Anspruch 1, dadurch gekennzeichnet, dass man Verbindungen der Formel I nach Anspruch 10, worin
$X^1$ die Gruppe NH,
$X^2$ Sauerstoff,
$R^1$ Wasserstoff oder $C_{2-4}$-Alkanoyl,
$R^2$ $C_{2-4}$-Alkanoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen unverzweigten Alkylrest mit 12 bis 18 C-Atomen, $R^{16}$ für Wasserstoff und $R^{17}$ für 1,2-Dipalmitoyloxy-äthyl oder 2-Oleoyloxy-1-palmitoyloxy-äthyl stehen,
$R^3$ Wasserstoff oder Methyl,
$R^4$, $R^5$, $R^7$, $R^8$ und $R^{10}$ Wasserstoff,
$R^6$ unsubstituiertes oder durch einen Rest der Formel Id substituiertes Methyl, Aethyl oder Isopropyl, worin m für 0 oder 1, E für Sauerstoff, $Z^2$ für Carbonyl, $Y^2$ für unsubstituiertes oder durch Phenyl substituiertes Niederalkylen, das durch ein oder zwei Iminocarbonylgruppen unterbrochen sein kann, $X^4$ für Sauerstoff und $A^2$ unabhängig von $A^1$ und $A^3$ für einen Rest der vorstehend definierten Formeln Ib oder Ic stehen,
$R^9$ Amino,
$R^{11}$ Hydroxy oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhängig von $A^1$ und $A^2$ für einen Rest der vorstehend definierten Formeln Ib oder Ic stehen,

$R^{12}$ Wasserstoff, Acetyl oder den gleichen Rest wie $R^{13}$ und $R^{13}$ Wasserstoff, Acetyl oder, unabhängig von $R^2$, einen wie vorstehend definierten Rest der Formel Ia bedeuten, und Salze dieser Verbindungen herstellt.

12. Verfahren nach Anspruch 1, dadurch gekennzeichnet, dass man Verbindungen der Formel I nach einem der Ansprüche 1 bis 11, worin die Reste $A^1$, $A^2$ und $A^3$ für einen Rest der Formel Ic stehen, und ihre Salze herstellt.

13. Verfahren nach Anspruch 1, dadurch gekennzeichnet, dass man Verbindungen der Formel I nach einem der Ansprüche 1 bis 7 und 9 bis 12, worin sich der Zuckerteil von (D)-Galactose oder (D)-Mannose ableitet, und ihre Salze herstellt.

14. Verfahren nach Anspruch 1, dadurch gekennzeichnet, dass man Verbindungen der Formel I nach einem der Ansprüche 1 bis 7 und 9 bis 12, worin sich der Zuckerteil von (D)-Glucose ableitet, und ihre Salze herstellt.

15. Verfahren nach Anspruch 1, dadurch gekennzeichnet, dass man Verbindungen der Formel I, nach einem der Ansprüche 1 bis 14, die an einem asymmetrisch substituierten C(-$R^3$)-Atom die (D)-Konfiguration, an einem asymmetrisch substituierten C(-$R^6$)-Atom die (L)-Konfiguration und am C(-N-$R^8$)-Atom die (D)-Konfiguration aufweisen, und ihre Salze herstellt.

16. Verfahren nach Anspruch 1, dadurch gekennzeichnet, dass man Verbindungen der Formel I nach einem der Ansprüche 1 bis 14, die an einem asymmetrisch substituierten C(-$R^3$)-Atom die (L)-Konfiguration, an einem asymmetrisch substituierten C(-$R^6$)-Atom die (L)-Konfiguration und am C(-N-$R^8$)-Atom die (D)-Konfiguration aufweisen, und ihre Salze herstellt.

17. Verfahren nach Anspruch 1, dadurch gekennzeichnet, dass man

Verbindungen der Formel I nach einem der Ansprüche 1 bis 16, die einen Rest der Formel Ia, worin n für 1 steht, oder einen Rest der Formel Id, worin m für 1 steht, enthalten, und ihre Salze herstellt.

18. Verfahren nach Anspruch 1, dadurch gekennzeichnet, dass man Verbindungen der Formel I nach einem der Ansprüche 1 bis 16, die einen Rest der Formel Ia, worin n für 0 steht, oder einen Rest der Formel Id, worin m für 0 steht, enthalten, und ihre Salze herstellt.

19. Verfahren nach Asnspruch 1, dadurch gekennzeichnet, dass man Verbindungen der Formel I nach einem der Ansprüche 1 bis 18, die nur einen Phosphorylsubstituenten tragen, und zwar im Rest $R^{13}$, und ihre Salze herstellt.

20. Verfahren nach Anspruch 1, dadurch gekennzeichnet, dass man Verbindungen der Formel I nach einem der Ansprüche 1 bis 18, die nur einen Phosphorylsubstituenten tragen, und zwar im Rest $R^6$, und ihre Salze herstellt.

21. Verfahren nach Anspruch 1, dadurch gekennzeichnet, dass man Verbindungen der Formel I nach einem der Ansprüche 1 bis 18, die nur einen Phosphorylsubstituenten tragen, und zwar im Rest $R^{11}$, und ihre Salze herstellt.

22. Verfahren nach Anspruch 1, dadurch gekennzeichnet, dass man die in den Beispielen 15 bis 24 genannten Verbindungen der Formel I herstellt.

23. Verfahren nach Anspruch 1, dadurch gekennzeichnet, dass man pharmazeutisch verwendbare Salze von Verbindungen der Formel I nach einem der Ansprüche 1 bis 22 herstellt.

24. Verfahren nach Anspruch 1, dadurch gekennzeichnet, dass man Natriumsalze von Verbindungen der Formel I nach Anspruch 23 herstellt.

25. Verfahren nach einem der Ansprüche 1 bis 24, dadurch gekennzeichnet, dass man zur Abspaltung von Schutzgruppen mit einer Säure oder mit Wasserstoff in Gegenwart eines Katalysators behandelt.

26. Verfahren nach einem der Ansprüche 1 bis 24, dadurch gekennzeichnet, dass man als Verbindungen mit einer oder mehreren reaktionsfähigen veresterten Hydroxygruppen Verbindungen verwendet, worin die Hydroxygruppe(n) mit einer starken anorganischen Säure oder einer Sulfonsäure verestert ist (sind).

27. Verfahren nach Anspruch 26, dadurch gekennzeichnet, dass man als Verbindungen mit einer oder mehreren reaktionsfähigen veresterten Hydroxygruppen Verbindungen verwendet, worin die Hydroxygruppe(n) mit Chlor-, Brom- oder Jodwasserstoffsäure oder mit einer niederaliphatischen Sulfonsäure oder einer unsubstituierten oder substituierten Benzolsulfonsäure verestert ist (sind).

28. Verfahren nach einem der Ansprüche 1 bis 24, dadurch gekennzeichnet, dass man als Verbindungen mit einer oder mehreren Hydroxyschutzgruppen Verbindungen verwendet, worin die Hydroxygruppe(n) mit niederaliphatischen Acyl-, Aroyl- oder von Kohlensäurederivaten sich ableitenden Resten oder mit in α-Stellung verzweigten Alkylresten oder mit α-Mono-, -Di- oder -Triarylniederalkylresten oder mit acetalbildenden Resten oder mit Triniederalkylsilylgruppen geschützt ist (sind).

29. Verfahren nach Anspruch 28, dadurch gekennzeichnet, dass man als Verbindungen mit einer oder mehreren Hydroxyschutzgruppen Verbindungen verwendet, worin die Hydroxygruppe(n) mit Niederalkanoyl-, Benzoyl-, Trimethylsilyl-, Dimethyl-tert.-butylsilyl-, Benzyloxycarbonyl-, Niederalkoxycarbonyl-, tert.-Butyl- oder mit gegebenenfalls substituierten Benzyl-, Triphenylmethyl- oder Tetrahydropyranylresten geschützt ist (sind).

30. Verfahren nach einem der Ansprüche 1 bis 24, dadurch gekennzeichnet, dass man als Verbindungen mit einer oder mehreren Carboxyschutzgruppen Verbindungen verwendet, worin die Carboxygruppe als tert.-Niederalkoxycarbonyl, unsubstituiertes oder durch Niederalkyl, Niederalkoxy, Hydroxy-, Halogen und/oder Nitro substituiertes Mono-, Di- oder Triphenylmethoxycarbonyl, unsubstituiertes oder durch Halogen substituiertes Benzoylmethoxycarbonyl, 2-Halogen-niederalkoxy-carbonyl, 2-(Triniederalkyl-silyl)-äthoxycarbonyl, 2-(Triphenyl-silyl)-äthoxycarbonyl oder Triniederalkyl-silyloxycarbonyl vorliegt.

31. Verfahren nach Anspruch 30, dadurch gekennzeichnet, dass man als Verbindungen mit einer oder mehreren Carboxyschutzgruppen Verbindungen verwendet, worin die Carboxygruppe(n) mit tert. Butyl-, Benzyl- oder mit gegebenenfalls durch Halogen oder Niederalkoxy substituierten Triphenylmethyl- oder Benzhydrylresten geschützt ist (sind).

32. Verfahren nach einem der Ansprüche 1 bis 24, dadurch gekennzeichnet, dass man als Verbindungen mit einer oder mehreren aktivierten Carbonsäuregruppen Verbindungen verwendet, worin die Carbonsäuregruppe(n) als Anhydrid, aktiviertes Amid oder als aktivierter Ester vorliegt (vorliegen).

33. Verfahren nach Anspruch 32, dadurch gekennzeichnet, dass man als Säureanhydride solche mit Kohlensäurehalbestern, mit dihalogenierter Phosphorsäure, mit einer organischen Carbonsäure, mit einer organischen Sulfonsäure oder Säurechloride, -bromide oder -azide, als aktivierte Säureamide Imidazolide, Pyrazolide oder Isooxazolide und als aktivierte Ester Vinylester, N',N'-disubstituierte Amidinoester, durch Halogen, Nitro, Sulfonyl oder Phenyldiazo substituierte Phenylester, Cyanmethylester, Phenylthioester oder Ester mit einer N-Hydroxy-amino- oder N-Hydroxy-amido-Verbindung verwendet.

34. Verfahren nach Anspruch 32, dadurch gekennzeichnet, dass man als Säureanhydride solche mit Kohlensäureniederalkylester, als Säureamide Imidazolide oder Isooxazolide und als aktivierte Ester Cyan- oder

Carboxymethylester, Acetylaminoäthylthioester, p-Nitro- oder 2,4,5-Trichlor-phenylester, N-Hydroxy-succinimid-, N-Hydroxy-phthalimid- oder N-Hydroxy-piperidinester, 8-Hydroxy-chinolinester, Methoxyäthylthioester oder durch Umsetzung mit Carbodiimid unter Zusatz von N-Hydroxysuccinimid oder einem 1-Hydroxybenzotriazol oder 3-Hydroxy-4-oxo-3,4-dihydro-benzo[d]-1,2,3-triazin erhaltene Ester verwendet.

35. Die nach dem Verfahren gemäss einem der Ansprüche 1-34 erhältlichen Verbindungen.

36. Verfahren zur Herstellung von pharmazeutischen Präparaten, dadurch gekennzeichnet, dass man eine nach einem Verfahren gemäss einem der Ansprüche 1-34 erhaltene Verbindung der Formel I mit einem pharmazeutisch verwendbaren Trägermaterial mischt.

0056992

Europäisches
Patentamt

## GEBÜHRENPFLICHTIGE PATENTANSPRÜCHE

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt für die Anspruchsgebühren entrichtet wurden,

nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen,
nämlich:

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche

0056992

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung
EP 82 10 0445

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y | FR - A - 2 361 902 (CIBA-GEIGY) | |
| | * Seiten 9 und 10 * | 1,23 |
| | & DE - A - 2 655 500 | |
| | --- | |
| P/X | EP - A - 0 025 495 (CIBA GEIGY) | |
| | * Seiten 75-92 * | 1,23 |
| | --- | |
| P/X | EP - A - 0 027 258 (CIBA-GEIGY) | |
| | * Seiten 76-93 * | 1,23 |
| | ---------- | |

KLASSIFIKATION DER ANMELDUNG (Int Cl.³)

C 07 H 13/04
C 07 H 15/04
C 07 H 15/18
A 61 K 31/70//
C 07 C 103/52

RECHERCHIERTE SACHGEBIETE (Int Cl.³)

C 07 H 15/00
C 07 H 13/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-28,30,32,33
Unvollständig recherchierte Patentansprüche:
Nicht recherchierte Patentansprüche: 29,31
Grund für die Beschränkung der Recherche:

Verfahren zur chirurgischen oder therapeutischen Behandlung des menschlichen oder tierischen Körpers (siehe Art. 52(4) des Europäischen Patentübereinkommens).

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 22.02.1982 | VERHULST |